# EUROPEAN PATENT APPLICATION

(11) **EP 4 520 833 A1**
(43) Date of publication of application: **12.03.2025**
(21) Application number: 23799508.9
(22) Date of filing: 02.05.2023
(51) Int. Cl.: C12N 15/86, C12N 1/15, C12N 1/19, C12N 1/21, C12N 5/10, C12N 7/01, C12N 15/11, C12N 15/35, C12N 15/38, C12N 15/48, C12N 15/864, C12N 15/867

(54) **PLASMID FOR VIRUS-DERIVED STRUCTURE**

(30) Priority: 02.05.2022 JP 2022076060
(71) Applicant: Synplogen Co., Ltd., Kobe-shi, Hyogo 650-0047 (JP)
(72) Inventor: SAITO, Shunsuke, Kobe-shi, Hyogo 650-0047 (JP); TSUGE, Kenji, Kobe-shi, Hyogo 650-0047 (JP); NISHIMURA, Yuya, Kobe-shi, Hyogo 650-0047 (JP)
(74) Representative: Müller-Boré & Partner Patentanwälte PartG mbB
(86) International application number: PCT/JP2023/017131
(87) International publication number: WO 2023/214579

(57) **Abstract**

The present disclosure provides production of a virus-derived construct plasmid. In one aspect, the present disclosure provides a plasmid encoding at least some elements of a virus-derived construct. In one embodiment, the present disclosure provides a plasmid containing at least some of nucleic acid sequences required to construct a virus-derived construct in an operably linked form. In one embodiment, the nucleic acid sequence required to construct a virus-derived construct includes (A) a nucleic acid sequence including a terminal repeat, (B) a nucleic acid sequence encoding a packaging factor, (C) a nucleic acid sequence encoding a structural protein, and (D) a nucleic acid sequence encoding a functional cofactor.

## Description

### Technical Field

The present disclosure provides a plasmid for producing a virus-derived construct. The present disclosure also provides a virus-derived construct produced from a plasmid and a composition containing the same.

### Background Art

Various virus-derived constructs such as adenoviral vectors (Patent Literature 1) have been developed for gene therapy, vaccine therapy, and the like.

For the preparation of virus-derived constructs, it is usually necessary to introduce virus-derived construct plasmids into producer cells. Therefore, synthesis and construction of virus-derived construct plasmids are required. The designed virus-derived construct plasmid is often synthesized and constructed in Escherichia coli or the like, and has never been synthesized and constructed in cells of other organisms.

### Citation List

### Patent Literature

Patent Literature 1: WO 2001/090392 A

### Summary of Invention

### Solution to Problem

The present inventors have found that a virus-derived construct plasmid can be produced in a combination of a first microorganism and a second microorganism. Based on this finding, the present disclosure provides a virus-derived construct plasmid capable of replicating in a first microorganism and a second microorganism. Further, the present inventors have found a novel structural plasmid that produces a virus-derived construct, and the present disclosure provides the same. In addition, the present disclosure also provides a microorganism containing a virus-derived construct plasmid and a virus-derived construct produced from the virus-derived construct plasmid.

Accordingly, the present disclosure provides the following.

### (Item 1)

A method for producing a virus-derived construct plasmid, the method including:
i) a step of obtaining a virus-derived construct plasmid containing at least one of nucleic acid sequences required to construct a virus-derived construct from a first microbial host cell; and
ii) a step of introducing the virus-derived construct plasmid into a second microbial host cell and placing the second microbial host cell under conditions in which the virus-derived construct plasmid is amplified.

### (Item 2)

A method for producing a virus-derived construct, the method including:
i) a step of obtaining a virus-derived construct plasmid containing at least one of nucleic acid sequences required to construct a virus-derived construct from a first microbial host cell;
ii) a step of introducing the virus-derived construct plasmid into a second microbial host cell and placing the second microbial host cell under conditions in which the virus-derived construct plasmid is amplified; and
iii) a step of introducing an introduced plasmid based on the virus-derived construct plasmid into a producer cell to form a virus-derived construct.

### (Item 3)

The method according to any one of the above items, further including, between the step ii) and the step iii), a step of cloning a nucleic acid containing at least one of the nucleic acid sequences required to construct a virus-derived construct in the virus-derived construct plasmid to produce the introduced plasmid.

### (Item 4)

The method according to any one of the above items, in which the producer cell is an animal cell.

### (Item 5)

The method according to any one of the above items, in which the producer cell is a human embryonic kidney cell and provides a viral vector genome copy number of 4 x 10³ or more per producer cell.

### (Item 6)

The method according to any one of the above items, in which the nucleic acid sequence required to construct a virus-derived construct includes:
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

### (Item 7)

The method according to any one of the above items, in which the introduced plasmid contains the nucleic acid sequences (A) to (D).

### (Item 8)

The method according to any one of the above items, in which the first microbial host cell is Bacillus subtilis.

### (Item 9)

The method according to any one of the above items, in which the second microbial host cell is Escherichia coli.

### (Item 10)

The method according to any one of the above items, in which the step i) includes forming the virus-derived construct plasmid from a nucleic acid fragment.

### (Item 11)

The method according to any one of the above items, in which the nucleic acid fragment is an acyclic nucleic acid fragment containing a tandem repeat nucleic acid sequence.

### (Item 12)

The method according to any one of the above items, in which the step i) includes bringing the first microbial host cell that is competent into contact with a nucleic acid containing at least one of the nucleic acid sequences required to construct a virus-derived construct.

### (Item 13)

A plasmid containing at least some of nucleic acid sequences required to construct a virus-derived construct in an operably linked form, in which the nucleic acid sequences required to construct a virus-derived construct include at least two of nucleic acid sequences (A) to (D):
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

### (Item 14)

The plasmid according to any one of the above items, in which the plasmid contains the nucleic acid sequences (A) to (D) in an operably linked form.

### (Item 15)

The plasmid according to any one of the above items, in which the nucleic acid sequence including a terminal repeat of (A) contains a desired gene.

### (Item 16)

The plasmid according to any one of the above items, in which the virus-derived construct is a virus-derived construct originating from an adeno-associated virus or a retrovirus.

### (Item 17)

The plasmid according to any one of the above items, in which the virus-derived construct is a viral vector originating from an adeno-associated virus.

### (Item 18)

The plasmid according to any one of the above items, in which the nucleic acid sequence including a terminal repeat of (A) includes two inverted terminal repeats (ITRs),
the nucleic acid sequence encoding a packaging factor of (B) includes rep,
the nucleic acid sequence encoding a structural protein of (C) includes cap, and
the nucleic acid sequence encoding a functional cofactor of (D) includes at least one of E1A, E1B, E2A, E2B, E3, E4, and VA.

### (Item 19)

The plasmid according to any one of the above items, in which at least one of the two ITRs is derived from any of adeno-associated virus serotypes 1 to 12 and variants thereof.

### (Item 20)

The plasmid according to any one of the above items, in which the nucleic acid sequence (B) includes rep derived from any of adeno-associated virus serotypes 1 to 12 and variants thereof.

### (Item 21)

The plasmid according to any one of the above items, in which the nucleic acid sequence (B) includes a promoter different from an adeno-associated virus promoter and at least one of rep78, rep76, rep52, and rep40 located downstream thereof.

### (Item 22)

The plasmid according to any one of the above items, in which the promoter is a drug-responsive promoter.

### (Item 23)

The plasmid according to any one of the above items, in which the terminal repeat of (A) and the nucleic acid sequence (B) include nucleic acid sequences derived from different adeno-associated virus serotypes.

### (Item 24)

The plasmid according to any one of the above items, in which the nucleic acid sequence (C) includes cap derived from any of adeno-associated virus serotypes 1 to 12 and variants thereof.

### (Item 25)

The plasmid according to any one of the above items, in which the nucleic acid sequence (C) includes a promoter different from an adeno-associated virus promoter and at least one of VP1, VP2, and VP3 located downstream thereof.

### (Item 26)

The plasmid according to any one of the above items, in which the nucleic acid sequence (D) includes a nucleic acid sequence encoding a functional cofactor derived from any of an adenovirus, a herpes virus, a bocavirus, and variants thereof.

### (Item 27)

The plasmid according to any one of the above items, in which the nucleic acid sequence (D) includes a nucleic acid sequence derived from any of adeno virus serotypes 1 to 52 and variants thereof.

### (Item 28)

The plasmid according to any one of the above items, in which the nucleic acid sequence (D) includes a promoter different from an adeno-associated virus promoter and at least one of E1A, E1B, E2A, E2B, E3, and E4 located downstream thereof.

### (Item 29)

The plasmid according to any one of the above items, in which the promoter is a drug-responsive promoter.

### (Item 30)

The plasmid according to any one of the above items, in which the nucleic acid sequence including a terminal repeat of (A) includes a 5' ITR, a promoter, a desired gene, a terminator, and a 3' ITR from upstream.

### (Item 31)

The plasmid according to any one of the above items, in which the virus-derived construct is a viral vector originating from a retrovirus.

### (Item 32)

The plasmid according to any one of the above items, in which the nucleic acid sequence including a terminal repeat of (A) includes two long terminal repeats (LTRs),
the nucleic acid sequence encoding a packaging factor of (B) includes ψ,
the nucleic acid sequence encoding a structural protein of (C) includes at least one of gag, pol, VSV-G, and env, and
the nucleic acid sequence encoding a functional cofactor of (D) includes Rev.

### (Item 33)

The plasmid according to any one of the above items, in which the plasmid contains a drug-responsive promoter upstream of at least one of the nucleic acid sequences (C) and (D).

### (Item 34)

The plasmid according to any one of the above items, in which the nucleic acid sequence including a terminal repeat of (A) includes a 5' LTR, a promoter, a desired gene, a terminator, and a 3' LTR from upstream.

### (Item 35)

The plasmid according to any one of the above items, in which the nucleic acid sequence (B) is present within the nucleic acid sequence (A), and the nucleic acid sequences (C) and (D) are present outside the nucleic acid sequence (A).

### (Item 36)

The plasmid according to any one of the above items, in which the plasmid contains 8 or more genes encoded by the nucleic acid sequences (A) to (D) in an operably linked form.

### (Item 37)

The plasmid according to any one of the above items, in which a drug-selectable marker nucleic acid sequence is contained between any two genes encoded by the nucleic acid sequences (A) to (D).

### (Item 38)

The plasmid according to any one of the above items, in which the nucleic acid sequence required to construct a virus-derived construct is about 10 kb or more.

### (Item 39)

The plasmid according to any one of the above items, in which the desired gene is 2 to 100 genes.

### (Item 40)

The plasmid according to any one of the above items, in which the plasmid contains, in an operably linked form:
a nucleic acid sequence that promotes plasmid replication in a first microbial host cell; and
a nucleic acid sequence that promotes plasmid replication in a second microbial host cell.

### (Item 41)

The plasmid according to any one of the above items, in which the first microbial host cell is Bacillus subtilis.

### (Item 42)

The plasmid according to any one of the above items, in which the second microbial host cell is Escherichia coli.

### (Item 43)

The plasmid according to any one of the above items, in which the plasmid provides a viral vector genome copy number of 4 x 10³ or more per producer cell when introduced into a human embryonic kidney cell that is a producer cell.

### (Item 44)

The plasmid according to any one of the above items, in which the plasmid provides a viral vector genome copy number of 1 x 10⁴ or more per producer cell when introduced into a human embryonic kidney cell that is a producer cell.

### (Item 45)

The plasmid according to any one of the above items, in which the plasmid provides a viral vector having a viral vector particle number with respect to a viral vector genome copy number (VP/VG) of 15 or less when introduced into a human embryonic kidney cell that is a producer cell.

### (Item 46)

The plasmid according to any one of the above items, in which the plasmid provides a viral vector having a viral vector particle number with respect to a viral vector genome copy number (VP/VG) of 10 or less when introduced into a human embryonic kidney cell that is a producer cell.

### (Item 47)

The plasmid according to any one of the above items, in which the plasmid and the producer cell together contain the nucleic acid sequence required to construct a virus-derived construct.

### (Item 48)

The plasmid according to any one of the above items, in which a covalently closed circular (CCC) purity is 80% or more.

### (Item 49)

A microbial host cell containing the plasmid according to any one of the above items.

### (Item 50)

A composition for preparing a virus-derived construct, the composition containing the plasmid according to any one of the above items.

### (Item 51)

A method for producing a viral vector, the method including a step of introducing the plasmid according to any one of the above items into a producer cell to construct a virus-derived construct.

### (Item 52)

The method according to any one of the above items, in which at least some of nucleic acids contained in the plasmid are incorporated into a chromosome of the producer cell.

### (Item 53)

A virus-derived construct produced by the method according to any one of the above items.

### (Item 54)

A virus-derived construct-containing composition produced by the method according to any one of the above items.

### (Item 55)

The virus-derived construct-containing composition according to any one of the above items, in which a viral vector particle number with respect to a viral vector genome copy number (VP/VG) is 15 or less.

### (Item 56)

A producer cell into which the plasmid according to any one of the above items is introduced, in which at least some of nucleic acids contained in the plasmid are incorporated into a chromosome of the producer cell.

### (Item A1)

A method for producing a virus-derived construct plasmid, the method including:
i) a step of obtaining a virus-derived construct plasmid containing at least one of nucleic acid sequences required to construct a virus-derived construct from a first microbial host cell; and
ii) a step of introducing the virus-derived construct plasmid into a second microbial host cell and placing the second microbial host cell under conditions in which the virus-derived construct plasmid is amplified.

### (Item A2)

A method for producing a virus-derived construct, the method including:
i) a step of obtaining a virus-derived construct plasmid containing at least one of nucleic acid sequences required to construct a virus-derived construct from a first microbial host cell;
ii) a step of introducing the virus-derived construct plasmid into a second microbial host cell and placing the second microbial host cell under conditions in which the virus-derived construct plasmid is amplified; and
iii) a step of introducing an introduced plasmid based on the virus-derived construct plasmid into a producer cell to form a virus-derived construct.

### (Item A3)

The method according to any one of the above items, further including, between the step ii) and the step iii), a step of cloning a nucleic acid containing at least one of the nucleic acid sequences required to construct a virus-derived construct in the virus-derived construct plasmid to produce the introduced plasmid.

### (Item A4)

The method according to any one of the above items, in which the producer cell is an animal cell.

### (Item A5)

The method according to any one of the above items, in which the producer cell is a human embryonic kidney cell and provides a viral vector genome copy number of 4 x 10³ or more per producer cell.

### (Item A6)

The method according to any one of the above items, in which the step i) includes obtaining the virus-derived construct plasmid from a plurality of nucleic acid fragments including at least two nucleic acid fragments containing a vector partial nucleic acid sequence other than the nucleic acid sequence required to construct a virus-derived construct.

### (Item A7)

The method according to any one of the above items, in which the vector partial nucleic acid sequence includes a nucleic acid sequence or a multiple cloning site having at least one function of nucleic acid replication, drug resistance, copy number adjustment, and stability improvement.

### (Item A8)

The method according to any one of the above items, further including, before the step i), a step of producing at least two of the plurality of nucleic acid fragments by chemical synthesis or an enzymatic reaction.

### (Item A9)

The method according to any one of the above items, further including, before the step i), a step of producing all of the plurality of nucleic acid fragments by chemical synthesis or an enzymatic reaction.

### (Item A10)

The method according to any one of the above items, in which the nucleic acid sequence required to construct a virus-derived construct includes:
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

### (Item A11)

The method according to any one of the above items, in which the introduced plasmid contains the nucleic acid sequences (A) to (D).

### (Item A12)

The method according to any one of the above items, in which the first microbial host cell is Bacillus subtilis.

### (Item A13)

The method according to any one of the above items, in which the second microbial host cell is Escherichia coli.

### (Item A14)

The method according to any one of the above items, in which the step i) includes forming the virus-derived construct plasmid from a nucleic acid fragment.

### (Item A15)

The method according to any one of the above items, in which the nucleic acid fragment is an acyclic nucleic acid fragment containing a tandem repeat nucleic acid sequence.

### (Item A16)

The method according to any one of the above items, in which the step i) includes bringing the first microbial host cell that is competent into contact with a nucleic acid containing at least one of the nucleic acid sequences required to construct a virus-derived construct.

### (Item A17)

A virus-derived construct plasmid prepared by the method according to any one of the above items.

### (Item A18)

A virus-derived construct prepared by the method according to any one of the above items.

### (Item A19)

A virus-derived construct-containing composition prepared by the method according to any one of the above items.

### (Item A20)

A plasmid containing at least some of nucleic acid sequences required to construct a virus-derived construct in an operably linked form,
in which the nucleic acid sequences required to construct a virus-derived construct include at least two of nucleic acid sequences (A) to (D):
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

### (Item A21)

The plasmid according to any one of the above items, in which the plasmid contains the nucleic acid sequences (A) to (D) in an operably linked form.

### (Item A22)

The plasmid according to any one of the above items, in which the nucleic acid sequence including a terminal repeat of (A) contains a desired gene.

### (Item A23)

The plasmid according to any one of the above items, in which the virus-derived construct is a virus-derived construct originating from an adeno-associated virus or a retrovirus.

### (Item A24)

The plasmid according to any one of the above items, in which the virus-derived construct is a viral vector originating from an adeno-associated virus.

### (Item A25)

The plasmid according to any one of the above items, in which the nucleic acid sequence including a terminal repeat of (A) includes two inverted terminal repeats (ITRs),
the nucleic acid sequence encoding a packaging factor of (B) includes rep,
the nucleic acid sequence encoding a structural protein of (C) includes cap, and
the nucleic acid sequence encoding a functional cofactor of (D) includes at least one of E1A, E1B, E2A, E2B, E3, E4, and VA.

### (Item A26)

The plasmid according to any one of the above items, in which at least one of the two ITRs is derived from any of adeno-associated virus serotypes 1 to 12 and variants thereof.

### (Item A27)

The plasmid according to any one of the above items, in which the nucleic acid sequence (B) includes rep derived from any of adeno-associated virus serotypes 1 to 12 and variants thereof.

### (Item A28)

The plasmid according to any one of the above items, in which the nucleic acid sequence (B) includes a promoter different from an adeno-associated virus promoter and at least one of rep78, rep76, rep52, and rep40 located downstream thereof.

### (Item A29)

The plasmid according to any one of the above items, in which the promoter is a drug-responsive promoter.

### (Item A30)

The plasmid according to any one of the above items, in which the terminal repeat of (A) and the nucleic acid sequence (B) include nucleic acid sequences derived from different adeno-associated virus serotypes.

### (Item A31)

The plasmid according to any one of the above items, in which the nucleic acid sequence (C) includes cap derived from any of adeno-associated virus serotypes 1 to 12 and variants thereof.

### (Item A32)

The plasmid according to any one of the above items, in which the nucleic acid sequence (C) includes a promoter different from an adeno-associated virus promoter and at least one of VP1, VP2, and VP3 located downstream thereof.

### (Item A33)

The plasmid according to any one of the above items, in which the nucleic acid sequence (D) includes a nucleic acid sequence encoding a functional cofactor derived from any of an adenovirus, a herpes virus, a bocavirus, and variants thereof.

### (Item A34)

The plasmid according to any one of the above items, in which the nucleic acid sequence (D) includes a nucleic acid sequence derived from any of adeno virus serotypes 1 to 52 and variants thereof.

### (Item A35)

The plasmid according to any one of the above items, in which the nucleic acid sequence (D) includes a promoter different from an adeno-associated virus promoter and at least one of E1A, E1B, E2A, E2B, E3, and E4 located downstream thereof.

### (Item A36)

The plasmid according to any one of the above items, in which the promoter is a drug-responsive promoter.

### (Item A37)

The plasmid according to any one of the above items, in which the nucleic acid sequence including a terminal repeat of (A) includes a 5' ITR, a promoter, a desired gene, a terminator, and a 3' ITR from upstream.

### (Item A38)

The plasmid according to any one of the above items, in which the virus-derived construct is a viral vector originating from a retrovirus.

### (Item A39)

The plasmid according to any one of the above items, in which the nucleic acid sequence including a terminal repeat of (A) includes two long terminal repeats (LTRs),
the nucleic acid sequence encoding a packaging factor of (B) includes ψ,
the nucleic acid sequence encoding a structural protein of (C) includes at least one of gag, pol, VSV-G, and env, and
the nucleic acid sequence encoding a functional cofactor of (D) includes Rev.

### (Item A40)

The plasmid according to any one of the above items, in which the plasmid contains a drug-responsive promoter upstream of at least one of the nucleic acid sequences (C) and (D).

### (Item A41)

The plasmid according to any one of the above items, in which the nucleic acid sequence including a terminal repeat of (A) includes a 5' LTR, a promoter, a desired gene, a terminator, and a 3' LTR from upstream.

### (Item A42)

The plasmid according to any one of the above items, in which the nucleic acid sequence (B) is present within the nucleic acid sequence (A), and the nucleic acid sequences (C) and (D) are present outside the nucleic acid sequence (A).

### (Item A43)

The plasmid according to any one of the above items, in which the plasmid contains 8 or more genes encoded by the nucleic acid sequences (A) to (D) in an operably linked form.

### (Item A44)

The plasmid according to any one of the above items, in which a drug-selectable marker nucleic acid sequence is contained between any two genes encoded by the nucleic acid sequences (A) to (D).

### (Item A45)

The plasmid according to any one of the above items, in which the nucleic acid sequence required to construct a virus-derived construct is about 10 kb or more.

### (Item A46)

The plasmid according to any one of the above items, in which the desired gene is 2 to 100 genes.

### (Item A47)

The plasmid according to any one of the above items, in which the plasmid contains, in an operably linked form:
a nucleic acid sequence that promotes plasmid replication in a first microbial host cell; and
a nucleic acid sequence that promotes plasmid replication in a second microbial host cell.

### (Item A48)

The plasmid according to any one of the above items, in which the first microbial host cell is Bacillus subtilis.

### (Item A49)

The plasmid according to any one of the above items, in which the second microbial host cell is Escherichia coli.

### (Item A50)

The plasmid according to any one of the above items, in which the plasmid provides a viral vector genome copy number of 4 x 10³ or more per producer cell when introduced into a human embryonic kidney cell that is a producer cell.

### (Item A51)

The plasmid according to any one of the above items, in which the plasmid provides a viral vector genome copy number of 1 x 10⁴ or more per producer cell when introduced into a human embryonic kidney cell that is a producer cell.

### (Item A52)

The plasmid according to any one of the above items, in which the plasmid provides a viral vector having a viral vector particle number with respect to a viral vector genome copy number (VP/VG) of 15 or less when introduced into a human embryonic kidney cell that is a producer cell.

### (Item A53)

The plasmid according to any one of the above items, in which the plasmid provides a viral vector having a viral vector particle number with respect to a viral vector genome copy number (VP/VG) of 10 or less when introduced into a human embryonic kidney cell that is a producer cell.

### (Item A54)

The plasmid according to any one of the above items, in which the plasmid and the producer cell together contain the nucleic acid sequence required to construct a virus-derived construct.

### (Item A55)

The plasmid according to any one of the above items, in which a covalently closed circular (CCC) purity is 80% or more.

### (Item A56)

The plasmid according to any one of the above items, in which nucleic acid fragments produced by chemical synthesis or an enzymatic reaction include at least two nucleic acid fragments containing a vector partial nucleic acid sequence.

### (Item A57)

The plasmid according to any one of the above items, in which only a plurality of nucleic acid fragments produced by chemical synthesis or an enzymatic reaction are circularized.

### (Item A58)

The plasmid according to any one of the above items, in which the nucleic acid fragments include three or more nucleic acid fragments.

### (Item A59)

The plasmid according to any one of the above items, in which each of the nucleic acid fragments has a length of 2 kb or less.

### (Item A60)

The plasmid according to any one of the above items, in which the vector partial nucleic acid sequence includes 40% or less of a total sequence of the virus-derived construct plasmid.

### (Item A61)

The plasmid according to any one of the above items, in which the vector partial nucleic acid sequence includes a nucleic acid sequence having at least one function of nucleic acid replication, drug resistance, copy number adjustment, and stability improvement, the virus-derived construct plasmid contains a restriction enzyme recognition sequence upstream and downstream of the vector partial nucleic acid sequence, and the same sequence as the restriction enzyme recognition sequence is not included in the nucleic acid sequence required to construct a virus-derived construct.

### (Item A62)

A microbial host cell containing the plasmid according to any one of the above items.

### (Item A63)

A composition for preparing a virus-derived construct, the composition containing the plasmid according to any one of the above items.

### (Item A64)

A method for producing a viral vector, the method including a step of introducing the plasmid according to any one of the above items into a producer cell to construct a virus-derived construct.

### (Item A65)

The method according to any one of the above items, in which at least some of nucleic acids contained in the plasmid are incorporated into a chromosome of the producer cell.

### (Item A66)

A virus-derived construct produced by the method according to any one of the above items.

### (Item A67)

A virus-derived construct-containing composition produced by the method according to any one of the above items.

### (Item A68)

The virus-derived construct-containing composition according to any one of the above items, in which a viral vector particle number with respect to a viral vector genome copy number (VP/VG) is 15 or less.

### (Item A69)

A producer cell into which the plasmid according to any one of the above items is introduced, in which at least some of nucleic acids contained in the plasmid are incorporated into a chromosome of the producer cell.

In the present disclosure, it is intended that one or a plurality of features can be provided in further combination in addition to the specified combination. Still further embodiments and advantages of the present disclosure will be appreciated by those skilled in the art upon reading and understanding the following detailed description, as necessary. Advantageous Effects of Invention

According to the present disclosure, it is possible to produce a virus-derived construct in an unconventional and improved manner based on a virus-derived construct plasmid.

### Brief Description of Drawings

FIG. 1 illustrates a structure of pGETS103-AAV-Helper-RC2 plasmid (All-in-One structure).
FIG. 2 illustrates an outline of construction of pGETS103-AAV-Helper-RC2 plasmid from 22 fragments by the OGAB method. The dotted box indicates the portion removed by cleavage with AarI (uppercase letters indicate linker-derived sequences, and lowercase letters indicate vector-derived sequences). The first, third, and sixteenth fragments were obtained by chemical synthesis and MAP method. Other fragments were obtained by PCR.
FIG. 3 illustrates the results of electrophoresis of pGETS103-AAV-Helper-RC2 plasmid constructed by the OGAB method.
FIG. 4 illustrates a structure and nucleic acid introduction site of plasmid pGETS118-AarI. The dotted box portion indicates a portion removed by cleavage with AarI.
FIG. 5 illustrates additional 7 types of All-in-One structures for producing AAV vectors introduced into pGETS118-AarI. The white outlined square shown under each All-in-One structure indicates the number of unit DNAs used for construction and the approximate corresponding regions.
FIG. 6 illustrates a structure of pAAV-CMV plasmid.
FIG. 7 illustrates a structure of pRC2-mi342 plasmid.
FIG. 8 illustrates a structure of pHelper plasmid.
FIG. 9 illustrates a structure of pGETS103-AAV plasmid.
FIG. 10 illustrates a structure of pGETS103-Helper plasmid.
FIG. 11 illustrates a structure of pGETS103-RC2 plasmid.
FIG. 12 illustrates the results of viral vector production from plasmid rAAVs #1 to #5. The vertical axis represents the amounts of viral genomes (VG: left) or viral particles (VP: right) produced from each plasmid.
FIG. 13 illustrates the results of viral vector production from plasmid rAAVs #6 and #7. The vertical axis represents the amounts of viral genomes (VG: left) or viral particles (VP: right) produced from each plasmid.
FIG. 14 illustrates a structure of pGETS151-AAV-Helper-RC2 plasmid (All-in-One structure).
FIG. 15 illustrates the results of viral vector production from plasmid rAAVs #8 and #9. The vertical axis represents the amount of viral genomes (VG) produced from each plasmid.
FIG. 16 illustrates the results of viral vector production from plasmid rAAVs #10 and #11. The vertical axis represents the amount of viral genomes (VG) produced from each plasmid.
FIG. 17 illustrates an All-in-One structure for producing an adenoviral vector. The white outlined squares shown below indicate the number of unit DNAs used for construction and the approximate corresponding regions. GOI indicates a desired gene.
FIG. 18 illustrates a structure and nucleic acid introduction site of plasmid pBET131-AarI. The dotted box portion indicates a portion removed by cleavage with AarI.
FIG. 19 illustrates a structure of an AAV All-in-One vector plasmid using Rep for AAV1 and Cap for AAV6 based on pGETS103-ΔAarI. The white outlined square shown under the All-in-One structure indicates the number of unit DNAs used for construction and the approximate corresponding regions.
FIG. 20 illustrates a structure of an AAV All-in-One vector plasmid using CAG promoter, Rep for AAV5, and Cap for AAV1 based on pGETS103-ΔAarI. The white outlined square shown under the All-in-One structure indicates the number of unit DNAs used for construction and the approximate corresponding regions.
FIG. 21 illustrates a structure of an AAV All-in-One vector plasmid using EF1α promoter, Rep for AAV8, and Cap for AAV9 based on pGETS103-ΔAarI. The white outlined square shown under the All-in-One structure indicates the number of unit DNAs used for construction and the approximate corresponding regions.
FIG. 22 illustrates a structure of an AAV All-in-One vector plasmid in which Rep, Cap, and Helper based on pGETS103-ΔAarI are arranged in a different order. This is also an example using SV40 promoter. The white outlined square shown under the All-in-One structure indicates the number of unit DNAs used for construction and the approximate corresponding regions.
FIG. 23 illustrates a structure of an AAV All-in-One vector plasmid in which Rep, Cap, and Helper based on pBETS131-AarI are arranged in a different order. The white outlined square shown under the All-in-One structure indicates the number of unit DNAs used for construction and the approximate corresponding regions.
FIG. 24 illustrates a structure of an AAV All-in-One vector plasmid in which an element of a Helper gene based on pBETS103-ΔAarI is changed. The white outlined square shown under the All-in-One structure indicates the number of unit DNAs used for construction and the approximate corresponding regions.
FIG. 25 illustrates a structure of an exemplary lentiviral vector plasmid constructed based on pGETS103ΔAarI.
FIG. 26 illustrates a structure of an exemplary retroviral vector plasmid constructed based on pGETS103ΔAarI.
FIG. 27 illustrates a structure of an exemplary adenoviral vector plasmid constructed based on pGETS103ΔAarI.
FIG. 28 illustrates a structure of a viral vector plasmid for which an attempt is made to construct the entire structure from nucleic acid fragments completely chemically synthesized or enzymatically synthesized by the OGAB method.
FIG. 29 illustrates a structure of pGETS151-AAV-EGFP-WRPE-Helper-RC2 plasmid.
FIG. 30 illustrates a structure of pAAV-EGFP-WRPE plasmid.
FIG. 31 illustrates the results of viral vector production from pGETS151-AAV-EGFP-WRPE-Helper-RC2 plasmid (Single-transfection method) and the triple transfection method containing the same viral vector constituent genes. The vertical axis represents the amount of viral genomes (VG) produced from each plasmid.

### Description of Embodiments

Hereinafter, the present disclosure will be described while showing the best mode. Throughout the present specification, the expression of singular forms is to be understood as including the concept of plural forms unless otherwise stated. Therefore, the singular article (for example, "a", "an", "the", or the like in English) should be understood to include the concept of plural forms unless otherwise stated. In addition, the terms used As used herein are to be understood as being used in the sense commonly used in the art unless otherwise stated. Therefore, unless defined otherwise, all technical and scientific terms used As used herein have the same meaning as commonly understood by those skilled in the art to which the present disclosure pertains. In the case of conflict, the present specification (including definitions) will control.

Hereinafter, the definitions of the terms and/or basic technical concepts that are particularly used As used herein will be described as appropriate.

As used herein, the term "plasmid" refers to circular DNA that is present separately from chromosomes in cells or that is present separately from chromosomes when introduced into cells.

As used herein, the term "nucleic acid sequence that promotes plasmid replication" is used in the same meaning as "nucleic acid sequence that promotes plasmid amplification", and refers to any nucleic acid sequence that promotes replication (in this context, it is the same as amplification) of a plasmid present in a host cell (for example, Bacillus subtilis) when introduced into a host cell. Preferably, the nucleic acid sequence that promotes plasmid replication is operably linked with, but not limited to, a nucleic acid sequence encoding a target plasmid. Details of the nucleic acid sequence that promotes plasmid replication, the target plasmid, the relationship therebetween, and the like, are described elsewhere As used herein. Examples of the nucleic acid sequence that promotes plasmid replication include a nucleic acid sequence containing an origin of replication that operates in a target host cell (for example, Bacillus subtilis). For example, a nucleic acid sequence that promotes plasmid replication in Bacillus subtilis has the ability to facilitate plasmid replication in Bacillus subtilis, and may further have the ability to facilitate plasmid replication in microorganisms other than Bacillus subtilis (for example, Escherichia coli). The nucleic acid sequence that promotes plasmid replication in Bacillus subtilis and other organisms (for example, Escherichia coli) may be a nucleic acid sequence in which the same region is utilized to promote plasmid replication in both Bacillus subtilis and other organisms (for example, Escherichia coli), may be a contiguous sequence on a virus-derived construct plasmid of the present disclosure, or may be a sequence located at a remote location. For example, plasmid pSTK1 (Issay Narumt et al., BIOTECHNOLOGY LETTERS, Volume 17, No. 5 (May 1995) pp. 475-480) and plasmid pBS195 (Molekuliarnaia Genetika et al., May 01, 1991, (5): 26-29, PMID: 1896058) have been shown to be replicable in the same replication initiation region in both Bacillus subtilis and Escherichia coli, and such nucleic acid sequences may be used as nucleic acid sequences to promote plasmid replication in both Bacillus subtilis and Escherichia coli. Also, a shuttle plasmid (P Trieu-Cuot et al., EMBO J. Dec 16, 1985; 4 (13A): 3583-3587) produced by linking BR322 (Escherichia coli plasmid) and pC194 (Bacillus subtilis plasmid), and a plasmid of the B. subtilis Secretory Protein Expression System containing pUBori and ColE1 ori provided by Takara Bio Inc (Shiga-ken) are replicable in both Escherichia coli and Bacillus subtilis, and such distantly positioned nucleic acid sequences can also be used as a nucleic acid sequence that promotes plasmid replication in both Bacillus subtilis and Escherichia coli. Examples of the replication mechanism of nucleic acid (plasmid) in Bacillus subtilis include rolling circle type, theta type, oriC, and phage, and any mechanism can be used as a sequence to be replicated in Bacillus subtilis. The rolling circle type replication mechanism is a mechanism in which one strand of double-stranded DNA is replicated and then the other strand is replicated, and the plasmid tends to be destabilized because the time during which the nucleic acid exists as single-stranded DNA is long. The theta type replication mechanism is a mechanism in which the replication of double-stranded DNA (plasmid) is simultaneously initiated in two directions from the origin of replication as in the case of replication of a bacterial chromosome, and can be preferably used in the replication of long DNA (for example, 10 kb or more) as in the present disclosure (Janniere, L., A. Gruss, and S. D. Ehrlich. 1993. Plasmids, p. 625-644. InA. L. Sonenshein, J. A. Hoch, and R. Losick (ed.), Bacillus subtilis and othergram-positive bacteria: biochemistry, physiology and molecular genetics. American Society for Microbiology, Washington, D.C.). The replication mechanism of oriC operates in the same manner as in replication of the host bacteria (for example, Bacillus subtilis) chromosome. Examples of the nucleic acid sequence that promotes plasmid replication in Bacillus subtilis include a plasmid or a portion thereof, or an origin of replication or a variant thereof, which is known to activate a replication mechanism by rolling circle type, theta type, oriC, phage, or the like. As rolling circle plasmids, pUB110, pC194, pE194, pT181, and the like are known, and as theta plasmids, pAMβ1, pTB19, pLS32, pLS20, and the like are known. The nucleic acid sequence that promotes plasmid replication in Bacillus subtilis can include a sequence that is identical or similar to a known replication starting point (for example, 90% or more, 95% or more, 97% or more, 98% or more, 99% or more, or 99.5% or more sequence identity). For example, when a DNA fragment in which a candidate DNA fragment and a selectable marker gene effective in Bacillus subtilis, such as a drug resistance gene, are linked is introduced into Bacillus subtilis and then cultured (with addition of a drug or the like), and then, the culture is replicated extrachromosomally, it can be determined that the candidate DNA fragment has a nucleic acid sequence that promotes plasmid replication in Bacillus subtilis. The nucleic acid sequence that promotes plasmid amplification is a DNA fragment having a replication starting point, and refers to a DNA fragment that can replicate independently of chromosomal DNA. Whether or not these DNA fragments can be replicated can be confirmed by a method in which a selectable marker gene such as a drug resistance gene is linked to these DNA fragments, the resultant is cultured under selective conditions such as a drug, plasmid DNA is then purified, and a band of the DNA is observed by electrophoresis. In addition to the replication starting point, the plasmid may contain a gene of rep protein that induces a DNA replication enzyme of the host at the replication starting point, a distribution mechanism gene for reliably distributing the plasmid to daughter cells, a selectable marker gene, and the like. The replication starting point may be fused within the gene of rep protein.

As used herein, the term "packaging cell" refers to a cell for producing virus-derived construct plasmids.

As used herein, the term "virus-derived construct plasmid" refers to a plasmid for producing a virus-derived construct in a producer cell containing genes loaded on the virus-derived construct. The virus-derived construct plasmid may contain a sequence of a gene (desired gene) to be expressed in a target cell of a virus-derived construct between two terminal repeats, may further contain a promoter, and may further contain other elements (for example, an enhancer, a terminator, or the like).

As used herein, the term "producer cell" means a cell capable of producing a desired virus-derived construct, and may be a cell in which a gene required for producing a virus-derived construct is expressed from a chromosome and an introduced plasmid. A desired virus-derived construct can be produced by introducing the virus-derived construct plasmids of the present disclosure into producer cells. For example, in the case of an AAV vector, E1A and E1B are required for production thereof, but since HEK293 has E1A and E1B, E1A and E1B can be removed from a helper plasmid. Other cells also function as producer cells when modified to have such functional cofactors.

As used herein, the term "transformed organism" refers to an organism that can be used in the OGAB method and can incorporate an acyclic long chain nucleic acid to produce a plasmid. As the transformed organism, a microorganism that spontaneously incorporates a nucleic acid can be used. A representative transformed organism is Bacillus subtilis.

As used herein, the term "Bacillus subtilis" refers to an aerobic, gram-positive, catalase-positive bacterium that is commonly present in soil and plants and is present in the gastrointestinal tract of ruminants and humans, and refers to any bacterium having no pathogenicity and having a natural transformation ability, including the scientific name Bacillus subtilis or Bacillus amyloliquefaciens, Bacillus licheniformis, Bacillus pumilus, or the like, which is a bacterium belonging to the genus Bacillus that is regarded as being a spore-forming bacteria, which has a size of 0.7 to 0.8 × 2.3 µm, is mesophilic, and has an optimal growth temperature of 25 to 40°C. In a preferred embodiment, Bacillus subtilis is Marburg 168 strain or its derivative strain, RM125 strain, which is a strain with a high natural transformation ability in Bacillus subtilis. The 168 strain is the most genetically studied gram-positive bacterium and is available from the American Type Culture Collection (ATCC) and the like together with the RM125 strain, and can be suitably used in the present disclosure because it is harmless to humans and animals itself and the OGAB method is known to be applicable.

As used herein, the term "microorganism" means an organism including small (for example, 1 mm or less, or 0.1 mm or less, or invisible to the naked eye) algae, protists, fungi (mold, mycete, and the like), yeasts, and animals (protozoa, metazoan, and the like) in addition to eubacteria and archaea.

As used herein, the term "animal cell" refers to a cell obtained from an animal (human, mouse, rat, monkey, dog, or the like) or a cell obtained by modifying a cell obtained from an animal.

As used herein, the term "virus-derived construct" refers to a construct containing a nucleic acid construct at least partially having a structure derived from a virus or a protein produced therefrom. Examples of the virus-derived construct include a viral vector, a virus-like particle (VLP), an oncolytic virus (including a virus modified to proliferate only in cancer cells), and a viral replicon (self-replicating viral genome lacking the ability to produce viral particles).

As used herein, the term "viral vector" refers to a construct that at least partially has a structure derived from a virus and is capable of introducing a nucleic acid into a target cell. Typically, the viral vector is in the form of viral particles containing nucleic acids containing viral structural proteins and heterologous genes.

As used herein, the term "nucleic acid sequence required to construct a virus-derived construct" refers to a nucleic acid sequence (for example, a combination of nucleic acid sequences) that enables a producer cell containing the nucleic acid sequence to produce a virus-derived construct. **In** one embodiment, the nucleic acid sequence required to construct a virus-derived construct includes a nucleic acid sequence encoding a structural protein of a virus, a nucleic acid sequence encoding a packaging factor, and a nucleic acid sequence encoding two terminal repeats of a virus and a functional cofactor. The term "nucleic acid sequence encoding a functional cofactor" may include or consist essentially of, for example, a nucleic acid sequence encoding a structural protein of a virus, a nucleic acid sequence encoding a packaging factor, a nucleic acid sequence required to construct any viral-derived construct other than two viral terminal repeats of a virus. Each of these nucleic acid sequences can vary for each virus-derived construct. These details are described elsewhere As used herein. In a preferred embodiment, as the nucleic acid sequence required to construct a virus-derived construct, an advantageous sequence common to AAV and adenovirus can be utilized, and more preferably, an advantageous sequence for AAV can be utilized. The advantageous sequence common to such AAV and adenovirus is characterized by containing a desired gene between inverted terminal repeats (ITRs) and optionally a functional cofactor, rep, and cap, or L1, L2, L3, L4, and L5, and the advantageous sequence for AAV contains a desired gene between ITRs and optionally a functional cofactor, rep, and cap. The arrangement of the functional cofactor, rep, and cap has the characteristic that it may be outside the two ITRs. For example, examples thereof include, but are not limited to, sequences of specific functional cofactors E1A, E1B, E2A, E2B, E3, and E4.

As used herein, the term "packaging factor" includes a protein that encapsulates the genome of a virus in a structural protein, and examples thereof include rep and ψ. Without intending to be limiting, a packaging factor derived from any of adenovirus serotypes 1 to 52 or adeno-associated virus serotypes 1 to 12, or variants thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) can be utilized. The protein may be either naturally occurring or artificially mutated. The "artificial mutated" protein can be produced by, for example, introducing an appropriate mutation into a naturally occurring sequence described in a known literature.

As used herein, the term "structural protein" refers to a protein produced from a gene of a virus, at least a portion of which is present on the surface of the virus or a virus-derived construct (for example, crossing an envelope). A structural protein can be responsible for infectivity into cells.

As used herein, the term "capsid" refers to a protein produced from a gene of a virus (encapsulated in an envelope, as necessary) that is present on the surface of the virus or a virus-derived construct, and is also referred to as "capsid protein".

As used herein, the term "repetitive sequence" or "tandem repeat" (a nucleic acid sequence) is a generic term for a nucleic acid sequence of a biological genome in which the same sequence is repeatedly (particularly several times or more) found. Any repetitive sequence used in the art can be used in the present disclosure. Typically, a promoter sequence appears repeatedly.

As used herein, the term "terminal repeat" is a generic term for those present at the terminal among those in which the same sequence is found repeatedly (particularly several times or more) in the nucleic acid sequence of the biological genome. Examples of the terminal repeat include, but are not limited to, an inverted terminal repeat (ITR) or a long terminal repeat (LTR). A terminal repeat derived from any of adenovirus serotypes 1 to 52 or adeno-associated virus serotypes 1 to 12, or variants thereof (rh10, DJ, DJ/8, PHP.eB, PHP.S, AAV2-retro, AAV2-QuadYF, AAV2.7m8, AAV6.2, rh.74, AAV2.5, AAV-TT, Anc80, and the like) can be utilized. The terminal repeat may be either naturally occurring or artificially mutated. The "artificial mutated" protein can be produced by, for example, introducing an appropriate mutation into a naturally occurring sequence described in a known literature.

As used herein, the term "functional cofactor" refers to a gene that supports amplification of a virus that cannot proliferate alone. In the present disclosure, examples of the functional cofactor include, but are not limited to, a nucleic acid sequence encoding a structural protein of the virus, a nucleic acid sequence encoding a packaging factor of the virus, and a nucleic acid sequence required to construct, proliferate, enhance activity, or reduce toxicity of any virus-derived construct other than the two terminal repeats of the virus. Examples of the functional cofactor that can be used include, but are not limited to, E1A, E1B, E2A, E2B, E4, RPE, WRPE, PPT, oPRE, an enhancer, an insulator, and a silencer sequence.

As used herein, the term "loaded nucleic acid" means a nucleic acid carried by a virus-derived construct (a viral vector or the like). Whether or not it is a loaded nucleic acid can be confirmed by treating a virus-derived construct preparation with DNase to decompose the nucleic acid outside the viral particle, inactivating the DNase, and then confirming the nucleic acid extracted from the viral particle. Whether or not it is a loaded nucleic acid can also be confirmed by examining the sequence of the resulting nucleic acid product (preferably a full-length or a length close to the full-length).

As used herein, the term "host cell" refers to a cell (including the progeny of such a cell) into which an exogenous nucleic acid or protein or viral or virus-derived construct is introduced.

As used herein, the terms "protein", "polypeptide", and "peptide" are used interchangeably, and refer to an amino acid polymer of any length. The polymer may be linear, branched, or cyclic. An amino acid may be a naturally-occurring, non-naturally occurring, or modified amino acid. The term also encompasses naturally or artificially modified polymers. Examples of such a modification include disulfide bond formation, glycosylation, lipidation, acetylation, phosphorylation, and any other manipulation or modification (for example, conjugation with a labeling component).

As used herein, the terms "polynucleotide" and "nucleic acid" are used interchangeably, and refer to a polymer of nucleotides of any length. Examples of the nucleic acid include DNA, RNA, cDNA, mRNA, rRNA, tRNA, microRNA (miRNA), and lncRNA. The term also includes a "polynucleotide derivative". The "polynucleotide derivative" refers to a polynucleotide containing a nucleotide derivative or having an unusual internucleotidic linkage. The "nucleotide derivative" refers to a nucleotide having a structure different from a normal nucleotide used in natural DNA or RNA, and examples thereof include a locked nucleic acid (LNA), an ethylene nucleic acid such as a 2'-O,4'-C-ethylene-bridged nucleic acid (ENA), other bridged nucleic acids (BNAs), a hexitol nucleic acid (HNA), an amido-bridged nucleic acid (AmNA), a morpholino nucleic acid, tricyclo-DNA (tcDNA), a polyether nucleic acid (see, for example, U.S. Patent No. 5,908,845), and a cyclohexene nucleic acid (CENA). Examples of the unusual internucleotidic linkage include an internucleotidic linkage in which a phosphate diester linkage is converted to a phosphorothioate linkage, an internucleotidic linkage in which a phosphate diester linkage is converted to an N3'-P5' phosphoramidate linkage, and an internucleotidic linkage in which ribose and a phosphate diester linkage are converted to peptide nucleic acid linkages.

Unless otherwise indicated, a particular nucleic acid sequence is also intended to encompass a conservatively modified variant (for example, a degenerate codon substituent) and a complementary sequence thereof, as well as the sequence explicitly indicated. Specifically, the degenerate codon substituent can be achieved by creating a sequence in which the third position of one or more selected (or all) codons is substituted with a mixed-base and/or deoxyinosine residue. For example, variants based on specific wild-type sequences, such as adenovirus serotypes 1 to 52 and adeno-associated virus serotypes 1 to 12, contain, in addition to variants (for example, rh10, DJ, DJ/8, PHP.eB, PHP.S, AAV2-retro, AAV2-QuadYF, AAV2.7m8, AAV6.2, rh.74, AAV2.5, AAV-TT, Anc80, and the like), nucleic acids containing a sequence having at least 70%, at least 80%, at least 85%, at least 90%, at least 95%, at least 96%, at least 97%, at least 98%, at least 99%, or at least 99.5% sequence identity to the original sequence.

As used herein, the term "gene" refers to a nucleic acid portion that performs a certain biological function. Examples of the biological function include coding a polypeptide or protein, coding protein-non-coding functional RNA (rRNA, tRNA, microRNA (miRNA), lncRNA, or the like), controlling the production of a polypeptide, protein, or protein-non-coding functional RNA, specifically binding to a specific protein, and controlling the cleavage a nucleic acid or replication. Therefore, As used herein, in addition to the nucleic acid portion encoding a protein or non-protein-coding functional RNA, the gene contains a transcriptional/translational regulatory sequence such as a promoter, a terminator, an enhancer, an insulator, a silencer, an origin of replication, or an internal ribosome entry site, and a nucleic acid portion required for packaging into viral particles. As used herein, the term "gene product" may refer to a polypeptide, protein, or non-protein coding functional RNA encoded by a gene.

As used herein, the fact that two genes are cis refers to that the genes are present on the same nucleic acid molecule or a nucleic acid molecule of a complementary strand (in the case of a double-stranded nucleic acid). In addition, As used herein, the fact that two genes are trans refers to that, in a cell (in an organism), these genes are not present on the same nucleic acid molecule or a nucleic acid molecule of a complementary strand (in the case of a double-stranded nucleic acid). For example, the gene present on the genome and the gene on the nucleic acid introduced with the virus-derived construct can be trans. As necessary, whether or not two genes are trans can be determined in a state at the time when the two genes become simultaneously present in a cell (for example, introduction of a nucleic acid into a cell).

When referring to the number of genes As used herein, one gene refers to a gene that contains a continuous sequence in a form that is normally (most frequent or 50% or more probability) present on the genome of an organism. For example, two exons encoding a protein can be two genes. For example, when the promoter sequence and the sequence encoding the protein form a continuous sequence, the nucleic acid portion containing the promoter sequence and the sequence encoding the protein can be one gene. For example, when a protein that becomes functional when cleaved is encoded by a continuous sequence on the genome, the protein can be encoded by one gene. When referring to a gene in terms of function, the nucleic acid sequence does not need to be a continuous sequence, for example, a plurality of exons encoding a protein are collectively referred to as genes of the protein.

As used herein, the term "deficiency" of a gene refers to that the nucleic acid does not contain the gene or contain a gene that is modified so as not to perform the normal function (for example, the function of producing a functional protein) of the gene.

As used herein, the term "operably linked" refers to the placement of expression (actuation) of a desired sequence under the control of a transcriptional/translational regulatory sequence (for example, a promoter, an enhancer, or the like) or a translational regulatory sequence. In order for a promoter to be operably linked to a gene, the promoter is usually arranged immediately upstream of the gene, but not necessarily adjacent.

As used herein, the term "transcriptional/translational regulatory sequence" collectively refers to a promoter sequence, a polyadenylation signal, a transcription termination sequence, an upstream regulatory domain, an origin of replication, an enhancer, IRES, and the like, which act in combination to enable the replication, transcription, and translation of a coding sequence in a recipient cell. Not all of these transcriptional/translational regulatory sequences need to be present, as long as replication, transcription, and translation of the selected coding sequence are possible in a suitable host cell. Those skilled in the art can easily identify a regulatory nucleic acid sequence from public information. Furthermore, those skilled in the art can identify a transcriptional/translational regulatory sequence that can be applied for use purposes, for example, in vivo, ex vivo, or in vitro.

As used herein, the term "promoter" refers to a segment of a nucleic acid sequence that controls transcription of an operably linked nucleic acid sequence. The promoter contains a specific sequence that is sufficient for recognition, binding, and transcription initiation by RNA polymerase. The promoter may contain a sequence that regulates recognition, binding, or transcription initiation by RNA polymerase.

As used herein, the term "enhancer" refers to a segment of a nucleic acid sequence that has the function of increasing the expression efficiency of a gene of interest.

As used herein, the term "silencer" refers to a segment of a nucleic acid sequence that has the function of reducing the expression efficiency of a gene of interest, contrary to an enhancer.

As used herein, the term "insulator" refers to a segment of a nucleic acid sequence that has the function of cis-regulation, which regulates the expression of genes at distant locations on the sequence of DNA.

As used herein, the term "terminator" refers to a segment of a nucleic acid sequence that is located downstream of a region coding a protein and is involved in the termination of transcription when the nucleic acid is transcribed into mRNA.

As used herein, the term "origin of replication" refers to a segment of a nucleic acid sequence at which replication begins by partial unwinding of the DNA double helix when a protein (for example, initiator DnaA protein or the like) that recognizes the nucleic acid sequence binds or when RNA is synthesized.

As used herein, an internal ribosome entry site ("IRES") refers to a nucleic acid segment that promotes entry or retention of ribosomes during translation of a nucleic acid sequence downstream thereof.

As used herein, the term "homology" of nucleic acids refers to a degree of identity of two or more nucleic acid sequences with respect to one another, and having "homology" generally refers to having a high degree of identity or similarity. Therefore, the identity or similarity of sequences is higher as homology of two nucleic acids is high. The "similarity" is a numerical value calculated for a similar base in addition to identity, and here, the similar base refers to a case where some of bases in a mixed base (for example, R = A + G, M = A + C, W = A + T, S = C + G, Y = C + T, K = G + T, H = A + T + C, B = G + T + C, D = G + A+ T, V = A + C + G, N = A + C + G + T) are identical. Whether two types of nucleic acids have homology can be investigated by a direct comparison of sequences or by a hybridization method under stringent conditions. When two nucleic acid sequences are directly compared, the genes have homology when the nucleic acid sequences are typically at least 50% homology, preferably at least 70% homology, and more preferably at least 80%, 90%, 95%, 96%, 97%, 98%, or 99% homology.

Amino acids can be mentioned As used herein by either their commonly known three letter symbols or their one character symbols recommended by the IUPAC-IUB Biochemical Nomenclature Commission. Similarly, nucleotides can be mentioned by their commonly recognized one character codes. As used herein, a comparison of similarity, identity, and homology of an amino acid sequence and a base sequence is calculated by using a sequence analysis tool BLAST and default parameters. For example, identity can be searched using BLAST 2.10.1+ (published on June 18, 2020) of the NCBI. As used herein, a value for identity generally refers to a value obtained when aligned under the default conditions using BLAST described above. However, when a higher value is obtained by changing a parameter, the highest value is set as the value of identity. In a case where identity is evaluated in a plurality of regions, the highest value in the plurality of regions is set as the value of identity. Similarity is a numerical value calculated by taking into consideration a similar amino acid in addition to identity.

As used herein, unless otherwise specified, it is understood that reference to a biological material (for example, a protein, a nucleic acid, or a gene) is also a reference to a variant of the biological material (for example, a variant having modification in an amino acid sequence) that performs a function similar to (but not to the same extent as) the biological function of the biological material. Such a variant can contain a fragment of the original molecule, and a molecule that is at least 30%, 40%, 50%, 60%, 70%, 80%, 90%, 95%, 98%, or 99% homology as compared to the sequence of the original molecule that is aligned over the amino acid sequence or nucleic acid sequence of the original biological material of the same size, or aligned by computer homology program known in the art. A variant can contain a molecule containing a modified amino acid (modification by, for example, disulfide bond formation, glycosylation, lipidation, acetylation, or phosphorylation) or a modified nucleotide (for example, modification by methylation).

As used herein, the term "stringent conditions" refers to well-known conditions commonly used in the art. As the stringent conditions, for example, the following conditions can be adopted. Incubation is performed at 37°C overnight (1) using low ionic strength and high temperature for washing (for example, at 50°C, 0.015 M sodium chloride/0.0015 M sodium citrate/0.1% sodium dodecyl sulfate), (2) using a denaturant such as formamide during hybridization (for example, at 42°C, 50% (v/v) formamide and 0.1% bovine serum albumin/0.1% ficol/0.1% polyvinylpyrrolidone/50 mM sodium phosphate buffer with pH 6.5, and 750 mM sodium chloride, 75 mM sodium citrate), or (3) in a solution containing 20% formamide, 5 x SSC, 50 mM sodium phosphate (pH 7.6), 5 x Denhardt's solution, 10% dextran sulfate, and 20 mg/ml of denatured sheared salmon sperm DNA, and then the filter is washed at 1 x SSC at about 37 to 50°C. Note that a formamide concentration may be 50% or more. A washing time may be 5, 15, 30, 60, or 120 minutes or longer. A plurality of factors such as a temperature and a salt concentration are considered as factors affecting the stringency of the hybridization reaction, and for details, reference can be made to Ausubel et al., Current Protocols in Molecular Biology, Wiley Interscience Publishers, (1995). Examples of "highly stringent conditions" are 0.0015 M sodium chloride, 0.0015 M sodium citrate, 65 to 68°C, or 0.015 M sodium chloride, 0.0015 M sodium citrate, and 50% formamide, 42°C. Hybridization can be performed according to the methods described in experimental textbooks such as Molecular Cloning 2nd ed., Current Protocols in Molecular Biology, Supplement 1-38, DNA Cloning 1: Core Techniques, A Practical Approach, Second Edition, Oxford University Press (1995). Here, a sequence including only the A sequence or only the T sequence is preferably excluded from a sequence that hybridizes under stringent conditions. Moderately stringent conditions can be readily determined by those skilled in the art, for example, based on DNA length, and are shown in Sambrook et al., Molecular Cloning: A Laboratory Manual, # 3, Vol. 1, 7.42-7.45 Cold Spring Harbor Laboratory Press, 2001; and include, for nitrocellulose filters, the use of a pre-wash solution of 5 × SSC, 0.5% SDS, and 1.0 mM EDTA (pH 8.0), hybridization conditions of about 50% formamide, 2 x SSC-6 x SSC (or other similar hybridization solution such as Stark's solution in about 50% formamide at about 42°C) at about 40 to 50°C, and washing conditions of 0.5 x SSC, 0.1% SDS at about 60°C. Accordingly, the polypeptide used in the present disclosure also includes a polypeptide encoded by a nucleic acid molecule that hybridizes under highly or moderately stringent conditions to the nucleic acid molecule encoding the polypeptide specifically described in the present disclosure.

As used herein, the term "corresponding" amino acid or nucleic acid refers to an amino acid or a nucleotide which has or is expected to have, in a certain polypeptide molecule or polynucleotide molecule, a similar action as a predetermined amino acid or nucleotide in a reference polypeptide or a polynucleotide for comparison, and, particularly in the case of enzyme molecules, refers to an amino acid which is present at a similar position in an active site and makes a similar contribution to catalytic activity. For example, for an antisense molecule, it can be a similar portion in an ortholog corresponding to a specific portion of the antisense molecule. A corresponding amino acid can be a specific amino acid subjected to, for example, cysteination, glutathionylation, S-S bond formation, oxidation (for example, oxidation of a methionine side chain), formylation, acetylation, phosphorylation, glycosylation, myristylation, or the like. Alternatively, a corresponding amino acid can be an amino acid responsible for dimerization. Such a "corresponding" amino acid or nucleic acid may be a region or a domain over a certain range. Accordingly, it is referred As used herein as a "corresponding" region or domain in such a case.

As used herein, the term "corresponding" gene (for example, a polynucleotide sequence or molecule) refers to a gene (for example, a polynucleotide sequence or molecule) in a certain species which has or is expected to have a similar action as a predetermined gene in a reference species for comparison. When a plurality of genes having such an action are present, the corresponding gene refers to a gene having the same evolutionary origin. Accordingly, a gene corresponding to a certain gene can be an ortholog of such a gene. For example, the cap of serotype 1 AAV can correspond to cap of serotype 2 AAV. For example, a corresponding gene in a certain virus can be found by search on a sequence database for the virus using a gene sequence of the virus serving as a reference of the corresponding gene as a query sequence.

In accordance with the present disclosure, the term "activity" refers to a function of a molecule in the broadest sense As used herein. Activity, although not intended to be limiting, generally includes a biological function, biochemical function, physical function, and chemical function of a molecule. Examples of the activity include enzymatic activity, an ability to interact with another molecule, an ability to activate, promote, stabilize, inhibit, suppress, or destabilize a function of another molecule, stability, and an ability to localize at a specific position in a cell. When applicable, the term also relates to a function of a protein complex in the broadest sense.

As used herein, when referring to a certain gene or a nucleic acid molecule or a polypeptide related thereto, the term "biological function" refers to a specific function that the gene, the nucleic acid molecule, or the polypeptide can have in a living body. Examples of the biological function include, but are not limited to, ability to recognize a specific cell surface structure, enzymatic activity, and ability to bind to a specific protein. In the present disclosure, examples of the biological function include, but are not limited to, a function in which a certain promoter is recognized in a specific host cell. As used herein, the biological function can be exerted by "biological activity". As used herein, the term "biological activity" refers to an activity that a certain agent (for example, a polynucleotide, a protein, or the like) can have in a living body. Biological activity encompasses an activity of exerting a variety of functions (for example, transcription promoting activity), and also encompasses, for example, an activity of activating or inactivating another molecule by an interaction with a certain molecule. For example, when a certain factor is an enzyme, the biological activity thereof encompasses enzyme activity thereof. **In** another example, in a case where a certain factor is a ligand, binding to a receptor corresponding to the ligand is encompassed. Such biological activity can be measured by a technique that is well known in the art. Accordingly, the term "activity" refers to a variety of measurable indicators indicating or revealing binding (either direct or indirect); and affecting a response (that is, having a measurable effect in response to some exposure or stimulus), including, for example, the amount of upstream or downstream protein in a host cell or a measure of other similar functions.

As used in the present disclosure, the term "infectivity" of a virus or virus-derived construct refers to the ability to introduce a nucleic acid within the virus or virus-derived construct into a cell by adhesion or membrane fusion of the virus or virus-derived construct to the cell. The term "replicative ability" of a virus or virus-derived construct refers to the ability to produce infectious viral particles or virus-derived construct particles within an infected cell.

As used herein, the terms "transformation", "transduction", and "transfection" are used interchangeably, unless otherwise stated, and refer to the introduction of a nucleic acid into a host cell (as necessary, via a virus or virus-derived construct). As the transformation method, any method can be used as long as it is a method of introducing a nucleic acid into a host cell, and examples thereof include various well-known techniques such as the use of competent cells, an electroporation method, a method using a particle gun (gene gun), and a calcium phosphate method.

As used herein, the term "purified" substance or biological factor (for example, a nucleic acid, a protein, or the like) refers to a substance or a biological factor in which at least a portion of a factor naturally accompanying the biological factor is removed. Thus, the purity of the biological factor in the purified biological factor is generally higher than the purity in the normal state of the biological factor (that is, concentrated). The term "purified" As used herein refers to the presence of preferably at least 75% by weight, more preferably at least 85% by weight, still more preferably at least 95% by weight, and most preferably at least 98% by weight of a biological factor of the same type. The substance used in the present disclosure is preferably a "purified" substance.

As used herein, the term "pharmaceutical ingredient" means an optional ingredient that can constitute a medicine, and examples thereof include an active ingredient (which itself exhibits drug efficacy) and an additional ingredient (ingredient which itself is not expected to exhibit drug efficacy, but is expected to play a certain role (for example, an excipient, a lubricant, a surfactant, or the like) when contained as a medicine). The pharmaceutical ingredient may be a single substance or a combination of a plurality of substances and agents. Any combination such as a combination of an active ingredient and an additive ingredient or a combination of an adjuvant and an active ingredient can also be included.

As used herein, the term "active ingredient" refers to an ingredient that exerts an intended medicinal effect, and can correspond to one or a plurality of ingredients.

As used herein, the term "additional ingredient" refers to an optional ingredient that is not expected to have a medicinal effect but plays a certain role when contained as a medicine, and examples of the additional ingredient include a pharmaceutically acceptable carrier, a stabilizer, an adjuvant, a solubility improving agent, a solubilizing agent, a diluent, an excipient, a buffer, a binder, a diluent, a flavoring agent, and a lubricant.

As used herein, an "agent" is used in a broad sense, and may be any substance or other elements (for example, energy such as light, radioactivity, heat, and electricity) as long as the intended object can be achieved. Examples of such a substance include, but are not limited to, a protein, a polypeptide, an oligopeptide, a peptide, a polynucleotide, an oligonucleotide, a nucleotide, a nucleic acid (including, for example, DNA such as cDNA or genomic DNA, RNA such as mRNA), a polysaccharide, an oligosaccharide, a lipid, an organic small molecule (for example, a hormone, a ligand, an information transmitting substance, an organic small molecule, a molecule synthesized by combinatorial chemistry, a small molecule that can be used as a medicine (for example, a small molecule ligand or the like), or the like), a composite molecule thereof, and a mixture thereof.

As used herein, the term "complex" or "complex molecule" means any construct having two or more portions. For example, when one portion is a polypeptide, the other portion may be a polypeptide or a substance other than the polypeptide (for example, a substrate, a sugar, a lipid, a nucleic acid, other hydrocarbons, or the like). As used herein, two or more portions constituting the complex may be bonded by a covalent bond or may be bonded by other bonds (for example, a hydrogen bond, an ionic bond, a hydrophobic interaction, van der Waals force, and the like).

As used herein, the term "label" refers to the presence (for example, a substance, energy, an electromagnetic wave, and the like) for identifying a molecule or substance to be targeted from others. Examples of such a labeling method include a radioisotope (RI) method, a fluorescence method, a biotin method, and a chemiluminescence method. When a plurality of target proteins or factors or means for capturing the target proteins are labeled by a fluorescence method, the target proteins are labeled with fluorescent substances having different maximum fluorescence emission wavelengths. A difference in maximum fluorescence emission wavelength is preferably 10 nm or more. Any label that does not affect the function can be used, and examples of the fluorescent substance include Alexa^{™} Fluor. Alexa^{™} Fluor is a water-soluble fluorescent dye obtained by modifying coumarin, rhodamine, fluorescein, cyanine, or the like, is a series corresponding to a wide range of fluorescence wavelengths, and is significantly stable, bright, and less pH sensitive as compared with other fluorescent dyes having corresponding wavelengths. Examples of a combination of fluorescent dyes having a maximum fluorescence wavelength of 10 nm or more include a combination of Alexa^{™} 555 and Alexa^{™} 633 and a combination of Alexa^{™} 488 and Alexa^{™} 555. Examples of other fluorescent labels include a cyanine dye (for example, Cy3 and Cy5 of CyDye^{™} series and the like), a rhodamine 6G reagent, N-acetoxy-N2-acetylaminofluorene (AAF), and AAIF (iodine derivative of AAF). In the present disclosure, such a label can be utilized to modify the object to be targeted so that the object can be detected by detection means used. Such modifications are known in the art and those skilled in the art can carry out such methods as appropriate for the label and depending on the object to be targeted.

As used herein, the term "kit" refers to a unit generally providing portions to be provided (for example, a virus-derived construct, a manual, and the like) that are usually divided into two or more sections. The form of the kit is preferred when it is intended to provide a composition that should not be provided in a mixed state for stability or the like, but is preferably mixed immediately before use. Such a kit preferably includes an instruction or manual describing how to use the provided portions or how a reagent should be processed. When the kit is used As used herein as a reagent kit, the kit generally includes an instruction or the like describing how to use a virus-derived construct and the like.

As used herein, the term "instruction" is a document with an explanation of the method of use of the present disclosure for a physician or other users. The instruction has a description of administration of the medicine of the present disclosure and the like. In addition, the instruction may include instruction of the administration form. The instruction is prepared in accordance with a format defined by the regulatory agency of the country in which the present disclosure is practiced (for example, the Ministry of Health, Labor and Welfare in Japan, Food and Drug Administration (FDA) in the United States or the like), with an explicit description showing approval by the regulatory agency. The instruction is a so-called package insert and is usually provided in, but is not limited to, a paper medium. For example, the instruction can also be provided in a form such as an electronic medium (for example, a web site or an e-mail provided on the Internet).

The term "about" refers to the indicated value plus or minus 10%. "About" when used for temperature refers to the indicated temperature plus or minus 5°C, and "about" when used for pH refers to the indicated pH plus or minus 0.5.

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the description As used herein. It is also understood that the following embodiments may be used alone or in combination.

### (Virus-derived construct plasmid)

In one aspect, the present disclosure provides a plasmid encoding at least some elements of a virus-derived construct. In one embodiment, the present disclosure provides a virus-derived construct plasmid capable of replicating in a combination of a first microorganism and a second microorganism. It is intended that any means for achieving this are within the scope of the present disclosure. For example, unless explicitly stated, a description of a method of using a component is intended to simultaneously encompass embodiments that reflect other means, such as a composition containing the same component, the use of the component, and the component for use in the method.

In one aspect, the present disclosure provides a plasmid encoding at least some elements of a virus-derived construct. In one embodiment, the present disclosure provides a plasmid containing at least some of nucleic acid sequences required to construct a virus-derived construct in an operably linked form. In one embodiment, the nucleic acid sequence required to construct a virus-derived construct includes (A) a nucleic acid sequence including a terminal repeat (for example, derived from any of serotypes 1 to 12 or variants thereof), (B) a nucleic acid sequence encoding a packaging factor (for example, rep, which can be derived from any of adeno-associated virus serotypes 1 to 12 or variants thereof), (C) a nucleic acid sequence encoding a structural protein (for example, cap, which can be derived from any of adeno-associated virus serotypes 1 to 12 or variants thereof), and (D) a nucleic acid sequence encoding a functional cofactor (for example, at least one of E1A, E1B, E2A, E4, and VA, which can be derived from any of adenovirus serotypes 1 to 52 or variants thereof). In one embodiment, the virus-derived construct plasmid of the present disclosure contains 2, 3, or 4 of the nucleic acid sequences of (A) to (D).

In one embodiment, the nucleic acid sequence required to constitute a virus is located in a continuous region on the virus-derived construct plasmid. A virus-derived construct plasmid having such a structure can be formed by incorporating nucleic acid fragments containing a nucleic acid sequence required to constitute a virus into an original plasmid. In one embodiment, the nucleic acid sequence required to constitute a virus is present in a continuous region of about 90% or less, about 80% or less, about 70% or less, about 60% or less, about 50% or less, about 40% or less, about 30% or less, about 25% or less, about 20% or less, about 15% or less, about 10% or less, or about 5% or less, and about 80% or more, about 70% or more, about 60% or more, about 50% or more, about 40% or more, about 30% or more, about 25% or more, about 20% or more, about 15% or more, about 10% or more, about 5% or more, or about 1% or more (consistent combination of upper and lower limits) of the total base length of the virus-derived construct plasmid. In one embodiment, elements other than the terminal repeat of the nucleic acid sequence required to constitute a virus (for example, one or a plurality of a nucleic acid sequence encoding a structural protein of a virus, a nucleic acid sequence encoding a packaging factor, and a functional cofactor) may each be located between or outside two terminal repeats. In the virus-derived construct plasmids of the present disclosure, the elements of the nucleic acid sequence required to constitute a virus may be arranged in any order and position, and it is understood that various arrangements other than the arrangements specifically shown in the drawings and the like can be used. In a case where the nucleic acid sequence described in a specific function such as a functional cofactor contains a plurality of elements, the order and position of each element in the virus-derived construct plasmid can be arbitrary, unless otherwise specified, and arbitrary elements (for example, VA, E2A, and E4) can be arranged in succession (without sandwiching other genes therebetween).

In one embodiment, the nucleic acid sequence required to constitute a virus contains two terminal repeats of the virus, and a base length of a region sandwiched between the two terminal repeats (excluding the terminal repeats themselves) can be about 1 kb or more, about 5 kb or more, about 10 kb or more, about 20 kb or more, about 30 kb or more, about 40 kb or more, about 50 kb or more, about 70 kb or more, or about 100 kb or more. In one embodiment, the nucleic acid sequence required to constitute a virus contains two terminal repeats of the virus and another portion, and the another portion is located outside the region sandwiched between the two terminal repeats. In one embodiment, the virus-derived construct plasmid of the present disclosure contains a promoter, a desired gene, and a terminator from upstream between a 5' ITR and a 3' ITR. In particular, it is intended that the nucleic acid sequence encoding a structural protein of a virus may be a variant of a wild-type sequence (for example, adeno-associated virus serotypes 1 to 12).

In one embodiment, the virus-derived construct plasmid of the present disclosure contains a promoter upstream of the element, as necessary. For example, the virus-derived construct plasmids of the present disclosure may contain a promoter upstream of one or a plurality of the nucleic acid sequences of (B), (C), and (D) or the genes contained therein of the nucleic acid sequences required to construct a virus-derived construct. For example, the virus-derived construct plasmids of the present disclosure may contain a promoter upstream of one or a plurality of desired genes. In one embodiment, the virus-derived construct plasmid of the present disclosure contains a drug-selectable marker nucleic acid sequence between any two of the nucleic acid sequences of (A) to (D). In one embodiment, the virus-derived construct plasmid of the present disclosure contains a drug-selectable marker nucleic acid sequence between any two genes encoded by the nucleic acid sequences of (A) to (D).

In one embodiment, the virus-derived construct plasmids of the present disclosure are used to produce a viral vector, a virus-like particle (VLP), an oncolytic virus (including a virus modified to proliferate only in cancer cells), or a viral replicon (self-replicating viral genome lacking the ability to produce viral particles).

In one embodiment, a virus-derived construct plasmid of the present disclosure can be produced by a method including: i) a step of obtaining a virus-derived construct plasmid containing at least one of nucleic acid sequences required to construct a virus-derived construct from a first microbial host cell; and ii) a step of introducing the virus-derived construct plasmid into a second microbial host cell and placing the second microbial host cell under conditions in which the virus-derived construct plasmid is amplified. In one embodiment, the first microbial host cell is Bacillus subtilis. In one embodiment, the second microbial host cell is Escherichia coli. Compared to virus-derived construct plasmids synthesized in with Bacillus subtilis and replicated with Bacillus subtilis, which were obtained only from Bacillus subtilis, virus-derived construct plasmids synthesized with Bacillus subtilis and replicated in Escherichia coli unexpectedly provided more virus-derived constructs per plasmid when introduced into producer cells in the same number of moles. Therefore, virus-derived construct plasmids produced via different microbial host cells may have excellent performance. In addition, for example, due to a cause such as introduction of an unintended mutation, a specific gene (GC-rich gene or the like) cannot be amplified in Escherichia coli, and may be amplified in Bacillus subtilis. Thus, it can be advantageous to use a plurality of types of microorganisms in the amplification of virus-derived construct plasmids. In one embodiment, the step i) includes forming the virus-derived construct plasmid from nucleic acid fragments. In one embodiment, after a virus-derived construct plasmid is constructed by the method for producing a virus-derived construct plasmid of the present disclosure, a portion of the virus-derived construct plasmid (for example, a region including the nucleic acid sequences (A) to (D)) may be introduced into another plasmid to produce a new virus-derived construct plasmid.

In one embodiment, the virus-derived construct plasmid of the present disclosure contains, in an operably linked form: a nucleic acid sequence that promotes plasmid replication in a first microbial host cell; and a nucleic acid sequence that promotes plasmid replication in a second microbial host cell. In one embodiment, the first microbial host cell is a transformed organism (Bacillus subtilis or the like). In one embodiment, the second microbial host cell is Escherichia coli. In one embodiment, a nucleic acid sequence that promotes plasmid replication in Bacillus subtilis can contain an origin of replication that operates in Bacillus subtilis, and an origin of replication contained in plasmids such as oriC, pTB19 (Imanaka, T., et al. J. Gen. Microbioi. 130, 1399-1408 (1984)) or pLS32 (Tanaka, T and Ogra, M. FEBS Lett. 422, 243-246 (1998)), and pAMβ1 (Swinfield, T. J., et al. Gene 87, 79-90 (1990)). Examples of the nucleic acid sequence that promotes plasmid replication in Bacillus subtilis include a plasmid or a portion thereof, or an origin of replication or a variant thereof, which is known to activate a replication mechanism by rolling circle type, theta type, oriC, phage, or the like. In one embodiment, the nucleic acid sequence that promotes plasmid replication in Bacillus subtilis can include a sequence that is identical or similar to a known replication starting point (for example, 90% or more, 95% or more, 97% or more, 98% or more, 99% or more, or 99.5% or more sequence identity). In one embodiment, the nucleic acid sequence that promotes plasmid replication in a microbial host cell may be arranged within or outside a continuous region where the nucleic acid sequence required to constitute a virus is present.

In one embodiment, the virus-derived construct plasmid of the present disclosure may contain a promoter and/or enhancer that operates in Bacillus subtilis. Examples of the promoter in Bacillus subtilis include Pspac (Yansura, D. and Henner, D. J. Pro. Natl. Acad. Sci, USA 81, 439-443 (1984.)) and Pr promoter (Itaya, M. Biosci. Biotechnol. Biochem. 63, 602-604 (1999)), which can be regulated in expression by isopropyl s-D-thiogalactopyranoside (IPTG). The nucleic acid element that operates in Bacillus subtilis does not need to be derived from Bacillus subtilis, and a nucleic acid element that operates with high efficiency or the like can be selected. **In** one embodiment, the origin of replication, promoter, and/or enhancer that operates in Bacillus subtilis in the virus-derived construct plasmid is located outside the region encoding the genome (which may contain a modification) of the origin virus of the virus-derived construct or a portion thereof.

**In** one embodiment, the virus-derived construct plasmids of the present disclosure can be produced or used in cells of organisms other than Bacillus subtilis, and thus can contain a transcriptional/translational regulatory sequence that operates in these organisms, such as an origin of replication, a promoter, and a transcription termination sequence. The transcriptional/translational regulatory sequence for each organism is known and can be appropriately selected by those skilled in the art. **In** one embodiment, the virus-derived construct plasmids of the present disclosure can be produced or replicated in Escherichia coli, a yeast, and the like, and thus can contain a transcriptional/translational regulatory sequence that operates in these microorganisms. **In** one embodiment, the virus-derived construct plasmids of the present disclosure can be introduced into producer cells and thus can contain a transcriptional/translational regulatory sequence that operates in producer cells. **In** particular, since the producer cell can be an animal cell, the virus-derived construct plasmids of the present disclosure can contain a transcriptional/translational regulatory sequence that operates in an animal cell. **In** one embodiment, the virus-derived construct plasmids of the present disclosure do not contain the sequence of at least some genes of the entire genome of the virus.

In one embodiment, the virus-derived construct plasmid of the present disclosure contains a desired gene. In one embodiment, the desired gene can be located between two terminal repeats. In one embodiment, the desired gene in the virus-derived construct plasmids of the present disclosure can ultimately be contained in the loaded nucleic acid of the virus-derived construct. Such a desired gene may be encoded by a therapeutic protein, may be encoded by a gene for gene therapy, may be encoded by a gene for gene cell therapy such as CART therapy, may be encoded by a non-protein coding functional RNA (rRNA, tRNA, microRNA (miRNA), lncRNA, or the like), or may contain a transcriptional/translational regulatory sequence such as a promoter, a terminator, an insulator, an enhancer, a silencer, and an origin of replication in combination or independently thereof. In one embodiment, the desired gene contains a viral promoter, such as a cytomegalovirus promoter, a CAG promoter, an SV40 promoter, or an RSV promoter (as necessary, upstream of the protein-coding gene). In one embodiment, the desired gene contains an IRES (as necessary, upstream of the protein-coding gene). In one embodiment, the desired gene can be integrated into chromosomes of a subject to whom the virus-derived construct is administered. In the embodiment, the desired gene may have a function of controlling expression of gene originally contained in the subject, or may result in long-term protein expression. For example, a virus-derived construct based on adeno-associated virus, a retrovirus, and the like can be incorporated into chromosomes of a subject. In one embodiment, the desired gene can be incorporated into a therapeutic cell (for example, chromosome) (for example, via a treatment of the cells with a virus-derived construct ex vivo). In one embodiment, the virus-derived construct plasmids of the present disclosure do not contain all the genes required for the origin virus of the virus-derived construct to proliferate, and accordingly, the virus-derived construct produced may be configured not to have proliferative ability.

In one embodiment, the virus-derived construct plasmid of the present disclosure, when introduced into a producer cell, can provide the number of genomic copies of the virus-derived construct of 1 x 10² or more, 4 x 10² or more, 1 x 10³ or more, 4 x 10³ or more, 1 x 10⁴ or more, 4 x 10⁴ or more, or 1 x 10⁵ or more per producer cell. In one embodiment, the virus-derived construct plasmid of the present disclosure can provide a virus-derived construct in which a viral vector particle number with respect to a viral vector genome copy number (VP/VG) of 35, 30, 25, 20, 15, 10, 5, 1 or less.

In one embodiment, the above performance of the virus-derived construct plasmid is determined when evaluated under the following conditions: 1.5 × 10⁷ 293T cells are seeded on the day before transfection in a 15 cm-dish. After 22 hours, the medium is replaced with 19 mL of DMEM medium (2% FBS, 1% penicillin/streptomycin), and the cells are cultured at 37°C and 5% CO₂ for 2 hours. To 293T cells in a confluent state (the number of cells: 3 × 10⁷), 500 µL of DMEM medium containing 2.1 pmol of the virus-derived construct plasmid and 500 µL of DMEM medium containing PEIpro in an amount of 1-fold of DNA are added. The cells are cultured at 37°C and 5% CO₂ for 72 hours, 1/80 volume of 0.5 M EDTA (pH 8.0) is added, and then, the cells are allowed to stand at room temperature for 10 minutes. In one embodiment, the above performance of the virus-derived construct plasmid is determined when evaluated under the following conditions:9.0 x 10⁷ VPC 2.0 cells were seeded in a 125 mL Erlenmeyer flask on the day of transfection. After 1 hour, 3 mL of AAV-MAX Enhancer was added, and culture was performed at 37°C and 8% CO₂ for 2 hours. A transfection solution obtained by mixing 3 mL of Viral-Plex^{™} Complexation Buffer containing 3.0 pmol of the virus-derived construct plasmids, 90 µL of AAV-MAX Transfection Booster, and 180 µL of AAV-MAX Transfection Reagent is added to the VPC 2.0 cells, and the mixture is cultured at 37°C and 8% CO₂ for 72 hours. Then, the cell suspension is recovered, and centrifugation is performed at 180 × g for 5 minutes to completely remove the supernatant. In one embodiment, the above performance of the virus-derived construct plasmid is determined when the genome titer (VG) is quantified by real-time PCR (SYBR Green method) and the particle titer (VP) is quantified and evaluated by ELISA.

In one embodiment, the desired gene in the virus-derived construct plasmid of the present disclosure can include a plurality of genes. In one embodiment, the desired gene can include 2 to 100 genes, for example, 2 or more, 3 or more, 4 or more, 5 or more, 7 or more, 10 or more, 12 or more, 15 or more, 20 or more, 25 or more, 30 or more, 40 or more, or 50 or more and 100 or less, 90 or less, 80 or less, 70 or less, 60 or less, 50 or less, 40 or less, 35 or less, 30 or more, 25 or less, 20 or less, 17 or less, 15 or less, 12 or less, or 10 or less genes. In one embodiment, the desired gene in the virus-derived construct plasmid of the present disclosure can have a base length of about 0.1 to 1,000 kbp, for example, about 0.1 kbp or more, about 0.3 kbp or more, about 1 kbp or more, about 2 kbp or more, about 5 kbp or more, about 7 kbp or more, about 10 kbp or more, about 20 kbp or more, about 50 kbp or more, or about 100 kbp or more, and about 1,000 kbp or less, about 700 kbp or less, about 500 kbp or less, about 200 kbp or less, about 100 kbp or less, about 70 kbp or less, about 50 kbp or less, about 20 kbp or less, or about 10 kbp or less. Since the virus-derived construct plasmids of the present disclosure can be constructed into a complex structure containing a plurality of genes by the OGAB method, a desired gene can also be constructed into a complex structure. Since the size of the loaded nucleic acid can be limited by the type of virus-derived construct, the type of virus-derived construct can be selected depending on the size of the desired gene. In one embodiment, the desired gene includes a plurality of genes, such that a nucleic acid having high functionality can be delivered to a subject, such as a combination of a tissue (for example, cancer tissue)-specific or time-specific promoter of a subject and a therapeutic gene operably linked thereto (therapeutic protein coding sequence, gene for gene therapy, gene for gene cell therapy, or the like), or a sequence encoding a series of enzymes that enables coordinated expression of a series of enzymes that control a metabolic cascade.

In one embodiment, the virus-derived construct plasmid of the present disclosure contains at least two, at least three, at least four, at least five, or more nucleic acid fragments containing a vector partial nucleic acid sequence in a nucleic acid fragment produced by chemical synthesis or an enzymatic reaction (for example, a reaction using a restriction enzyme). In one embodiment, the virus-derived construct plasmid of the present disclosure is produced by circularizing only a plurality of nucleic acid fragments produced by chemical synthesis or an enzymatic reaction. In one embodiment, the virus-derived construct plasmid of the present disclosure is produced from 3 or more, 4 or more, 5 or more, 7 or more, 10 or more, 15 or more, 20 or more nucleic acid fragments. In one embodiment, the nucleic acid fragments used to produce the virus-derived construct plasmid of the present disclosure can independently have a length of 20 kb or less, 15 kb or less, 10 kb or less, 7 kb or less, 5 kb or less, 4 kb or less, 3 kb or less, 2 kb or less, 1.5 kb or less, 1 kb or less, 0.7 kb or less, or 0.5 kb or less.

In one embodiment, the vector partial nucleic acid sequence contain about 60% or less, about 50% or less, about 40% or less, about 30% or less, about 20% or less, or about 10% or less of the total sequence of the virus-derived construct plasmid of the present disclosure. A vector partial nucleic acid sequence designed for a specific virus-derived construct plasmid can be shortened by omitting unnecessary elements, and can be designed to have a low proportion in the entire plasmid. In one embodiment, the vector partial nucleic acid sequence includes a nucleic acid sequence having at least one function of nucleic acid replication, drug resistance, copy number adjustment, and stability improvement. A recognition sequence by a specific restriction enzyme (for example, as described As used herein) may be contained upstream and/or downstream of the entire element or vector partial nucleic acid sequence. Such a restriction enzyme recognition sequence may include a sequence that is not found in other portions of the virus-derived construct plasmid (for example, the nucleic acid sequence required to construct a virus-derived construct), and therefore, the vector portion may be independently operable.

In one embodiment, examples of the protein encoded by the desired gene in the virus-derived construct plasmid of the present disclosure include a therapeutic polypeptide (for example, replacement therapy), and an immunogenic polypeptide (for example, pathogen polypeptide, cancer antigen polypeptide, or the like). Examples of the therapeutic polypeptide include cystic fibrosis transmembrane regulator protein (CFTR), dystrophin (mini-dystrophin and micro-dystrophin), myostatin propeptide, follistatin, soluble activin type II receptor, IGF-1, anti-inflammatory polypeptide, sarcospan, utrophin, mini-utrophin, a coagulation factor (for example, Factor VIII, Factor IX, Factor X, or the like), erythropoietin, angiostatin, endostatin, catalase, tyrosine hydroxylase, superoxide dismutase, leptin, LDL receptor, lipoprotein lipase, ornithine transcarbamylase, β-globin, α-globin, spectrin, α1-antitrypsin, adenosine deaminase, hypoxanthine-guanine phosphoribosyltransferase, β-glucocerebrosidase, sphingomyelinase, lysosomal hexosaminidase A, branched chain ketoacid dehydrogenase, RP65 protein, cytokine (for example, α-interferon, β-interferon, interferon-γ, interleukin-2, interleukin-4, granulocyte-macrophage colony-stimulating factor, lymphotoxin, or the like), peptide growth factor, neurotrophic factor and hormone (for example, somatotropin, insulin, insulin-like growth factors 1 and 2, platelet-derived growth factor, epidermal growth factor, fibroblast growth factor, nerve growth factor, neurotrophic factor-3 and -4, brain-derived neurotrophic factor, bone morphogenetic protein, glial-derived growth factor, transforming growth factor-α and -β, or the like), lysosomal acid α-glucosidase, α-galactosidase A, soluble tumor necrosis growth factor α receptor, S100A1, parvalbumin, adenylyl cyclase type 6, anti-inflammatory factor, anti-myostatin protein, aspartoacylase, suicide gene product (for example, thymidine kinase, cytosine deaminase, diphtheria toxin, or tumor necrosis factor), tumor suppressor gene product (for example, p53, Rb, or Wt-1), TRAIL, and FAS-ligand.

Examples of the pathogen polypeptide include a cell surface protein of a pathogenic organism such as bacteria, fungi, or parasites, and a protein (for example, spike protein, envelope protein, capsid protein, or the like) expressed on the surface of a virus. Specific examples of the pathogen polypeptide include orthomyxovirus immunogen (for example, influenza virus hemagglutinin (HA) or nucleoprotein), lentivirus immunogen (for example, HIV or SIV envelope GP160 protein, matrix/capsid protein, gag, pol, or env gene product), arenavirus immunogen (for example, Lassa fever virus nucleocapsid protein or envelope glycoprotein), poxvirus immunogen (for example, vaccinia L1 or L8 gene product), flavivirus immunogen (for example, yellow fever virus or Japanese encephalitis virus immunogen), filovirus immunogen (for example, Ebola virus or Marburg virus immunogen such as NP and GP gene products), bunyavirus immunogen (for example, RVFV, CCHF, or SFS virus immunogen), coronavirus immunogen (for example, human coronavirus immunogen such as human coronavirus envelope glycoprotein), polio immunogen, herpes virus immunogen (for example, CMV, EBV, or HSV immunogen), mumps virus immunogen, measles virus immunogen, rubella virus immunogen, diphtheria toxin or other diphtheria immunogens, pertussis antigen, and hepatitis (for example, hepatitis A, hepatitis B, hepatitis C, or the like) immunogen.

Examples of the cancer antigen polypeptide include BRCA1 gene product, BRCA2 gene product, gp100, tyrosinase, GAGE-1/2, BAGE, RAGE, LAGE, NY-ESO-1, CDK-4, β-catenin, MUM-1, caspase-8, KIAA0205, HPVE, SART-1, PRAME, p15, melanoma tumor antigen, MART-1, gp100 MAGE-1, MAGE-2, MAGE-3, CEA, TRP-1, TRP-2, P-15, tyrosinase, HER-2/neu gene product, CA125, LK26, FB5 (endosialin), TAG72, AFP, CA19-9, NSE, DU-PAN-2, CA50, SPan-1, CA72-4, HCG, STN (sialyl In antigen), c-erbB-2 protein, PSA, L-CanAg, estrogen receptor, milk fat globulin, p53 tumor suppressor protein, mucin antigen, telomerase, nuclear matrix protein, prostatic acid phosphatase, and papilloma virus antigen.

In one embodiment, the desired gene can be a gene for treating a particular disease (for example, gene for gene therapy). The gene to be selected for a specific disease can be appropriately selected by those skilled in the art. Examples of such a disease include infections due to various types of pathogens, cystic fibrosis, hemophilia A, hemophilia B, thalassemia, anemia, Alzheimer's disease, multiple sclerosis, Parkinson's disease, Huntington's disease, amyotrophic lateral sclerosis, spinal muscular atrophy, epilepsy, cancer (melanoma, adenocarcinoma, thymoma, lymphoma, sarcoma, lung cancer, liver cancer, colon cancer, leukemia, uterine cancer, breast cancer, prostate cancer, ovarian cancer, cervical cancer, bladder cancer, kidney cancer, pancreatic cancer, brain cancer, or the like), diabetes, muscular dystrophy, Gaucher's disease, Hurler syndrome, adenosine deaminase deficiency, glycogen storage disease, congenital emphysema, Lesch-Nyhan syndrome, Niemann-Pick disease, Tay-Sachs disease, Angelman syndrome, maple syrup urine disease, age-related macular degeneration, amaurosis, diabetic retinopathy, retinal degenerative disease, astrocytomas, glioblastoma, heart failure, peripheral artery disease, arthritis, joint disorder, intimal hyperplasia, AIDS, muscle wasting, kidney deficiency, hepatitis, LDL receptor deficiency, hyperammonemia, Krabbe's disease, Batten disease, spinal cerebral ataxias, phenylketonuria, autoimmune disease, amino acid metabolic disorder, organic acid metabolic disorder, fatty acid metabolic disorder, mitochondrial disease, sugar metabolic disorder, lysosomal disease, peroxisomal disorder, metal metabolic disorder, purine pyrimidine metabolic disorder, vitamin metabolic disorder, neurotransmitter disorder, lipid metabolic disorder, connective tissue disorder, congenital porphyria, α1-antitrypsin deficiency, lysosomal storage disease, mucopolysaccharidosis disorder, Fabry disease, Canavan disease, Leigh disease, Refsum's disease, Tourette's Syndrome, primary lateral sclerosis, progressive muscular atrophy, Pick disease, muscular dystrophy, myasthenia gravis, Binswanger disease, cerebral infarction, mood disorder, depression, bipolar affective disorder, persistent affective disorder, secondary mood disorder, schizophrenia, drug dependence, anxiety, obsessional disorder, somatoform disorder, dissociative disorder, grief, post-partum depression, hallucination, delusion, dementia, paranoia, autism spectrum disorder, attention-deficit hyperactivity disorder, psychosexual disorder, sleeping disorder, pain disorder, eating disorder, weight disorder, obesity, cachexia, anorexia nervosa, and bulimia. In one embodiment, the desired gene can be incorporated into a therapeutic cell (for example, chromosome) which is used for CART therapy or the like (for example, via a treatment of the cells with a virus-derived construct ex vivo).

### (Virus-derived construct)

The virus-derived construct plasmid of the present disclosure is used to produce a virus-derived construct. In one embodiment, the present disclosure provides a method for producing a virus-derived construct using the virus-derived construct plasmid of the present disclosure. In one embodiment, the present disclosure provides a virus-derived construct-containing composition or virus-derived construct produced as described above. Examples of the virus-derived construct include a virus-derived construct based on a retrovirus, lentivirus, adeno-associated virus, or adenovirus. In one embodiment, the present disclosure provides a method for producing a virus-derived construct, the method including: i) a step of obtaining a virus-derived construct plasmid containing at least one of nucleic acid sequences required to construct a virus-derived construct from a first microbial host cell; ii) a step of introducing the virus-derived construct plasmid into a second microbial host cell and placing the second microbial host cell under conditions in which the virus-derived construct plasmid is amplified; and iii) a step of introducing an introduced plasmid based on the virus-derived construct plasmid into a producer cell (for example, an animal cell) to form a virus-derived construct.

In one embodiment, the virus-derived construct plasmid of the present disclosure is introduced into a producer cell to produce a virus-derived construct. Examples of the producer cell include, but are not limited to, human embryonic kidney cells (prepared by transfecting human embryonic kidney cells with DNA obtained by cleaving adenovirus 5), 911 cells, PER.C6 cells, E1-transformed amniotic membrane cells, E1-transformed A549 cells, GH329: HeLa cells, HEK293 cells, IT293SF cells, HEK293 T, HEK293 F, Vero cells, CHO cells, Sf9 cells, Freestyle (trademark) 293-F, Expi293-F (trademark), Expi293 inducible, Expi293 NGT-Viral Production Cells 1.0, Viral Production Cells 2.0 (VPC 2.0 cells), AAVpro (registered trademark) 293T Cell Line, Lenti-X (trademark) 293T Cell Line, FreeStyle (trademark) CHO-S cells, ExpiCHO-S (trademark), VirusExpress (trademark) 293 AAV producing cells, and VirusExpress (trademark) 293T lentivirus-producing cells. Any known suitable producer cell can be selected depending on the type of virus-derived construct to be produced. In one embodiment, the virus-derived construct plasmid of the present disclosure is deficient in a small number (for example, 1, 2, 3, 4, or 5) of genes of the origin virus of the virus-derived construct, and may contain all other genes. In one embodiment, the virus-derived construct plasmid of the present disclosure is deficient in a portion of a set of genes required to produce a virus-derived construct, and the genes of the set of genes deficient in the virus-derived construct plasmid are supplied in the producer cell in trans with the virus-derived construct plasmid. In one embodiment, the virus-derived construct plasmid of the present disclosure can be introduced alone into a producer cell without being combined with another plasmid to allow for the production of a virus-derived construct. In one embodiment, the virus-derived construct plasmid of the present disclosure can be introduced into a producer cell expressing all of the genes in which the virus-derived construct plasmid is deficient in the set of genes required to produce a virus-derived construct. For example, the virus-derived construct plasmids of the present disclosure containing AAV cap and rep, and adenoviruses E2A, E4, and VA can be introduced alone into HEK293 cells expressing adenoviruses E1A and E1B to produce virus-derived constructs. In one embodiment, the virus-derived construct plasmid of the present disclosure can be introduced into a producer cell together with another nucleic acid containing a gene in which the virus-derived construct plasmid is deficient in the set of genes required to produce a virus-derived construct or a product of the gene (for example, viral particles) thereof.

In one embodiment, a virus-derived construct can be produced by introducing a virus-derived construct plasmid into a producer cell infected with the origin virus of the virus-derived construct and causing homologous recombination in the cells. The virus-derived construct plasmid used in the embodiment contains nucleic acid sequences having homology to the desired gene and to any region of the genome of the origin virus (for example, regions between genes). In one embodiment, the virus-derived construct plasmid can have a structure containing a desired gene between any genes in the genome of the origin virus of the virus-derived construct to be produced. In one embodiment, at least some of the nucleic acids contained in the virus-derived construct plasmid are incorporated into chromosomes of the producer cell.

In one embodiment, the virus-derived construct plasmid of the present disclosure contains segments for cutting loaded nucleic acids of the origin virus of the virus-derived construct to be produced (LTR in a retrovirus, ITR in AAV, and the like), and in one embodiment, the desired gene is contained between the segments. In one embodiment, the virus-derived construct plasmid of the present disclosure contains a desired gene between the segments and a promoter and/or terminator operably linked thereto (for example, those that are operable in the subject to which the virus-derived construct is targeted). In one embodiment, the virus-derived construct plasmids of the present disclosure do not contain the genes required for replication of the origin virus of the virus-derived construct between the segments. In one embodiment, the virus-derived construct plasmid of the present disclosure contains a packaging signal of the origin virus of the virus-derived construct to be produced.

The virus-derived construct produced has a structural protein (capsid or the like), which can be involved in binding to tissue or cell. Therefore, the targeting of the virus-derived construct to tissue or cell can be modulated by modifying the structural protein to modify its binding to the tissue or cell (or a surface structure thereof). In one embodiment, the virus-derived construct plasmids of the present disclosure may contain genes encoding a structural protein, and the structural protein may be modified. For example, the modification of the structural protein that modulates targeting of tissue or cell includes substitution or fusion with other proteins (other viral capsids (for example, capsids of other serotypes of viruses, VSV-G), antigen-binding regions of antibodies, ligand proteins of the subject organism (ligands for cancer antigens), and the like). In addition, a virus-derived construct containing an envelope can also contain cell membrane components of producer cells in the envelope. Therefore, the targeting of the virus-derived construct containing an envelope to tissue or cell can be modulated by modifying the cell membrane components of the producer cells. In one embodiment, in the case of a virus-derived construct without a structural protein, a non-virus-derived material such as a lipid particle can be used to modulate targeting to cells. In one embodiment, the virus-derived construct may be designed to target neural cells (cells of peripheral nervous system or central nervous system, brain cells such as neurons and oligodendrocytes, and the like), lung cells, ocular cells (retinal cells, retinal pigment epithelium, corneal cells, and the like), epithelial cells (for example, intestinal or respiratory epithelial cells), muscle cells (for example, skeletal muscle cells, cardiac muscle cells, smooth muscle cells, and diaphragm muscle cells), dendritic cells, pancreatic cells (islet cells and the like), hepatocytes, cardiomyocytes, bone cells (for example, bone marrow stem cells), hematopoietic stem cells, spleen cells, keratinocytes, fibroblasts, endothelial cells, prostate cells, germ cells, cancer cells, and the like. A surface structure (a receptor or the like) specific to each cell is known, and those skilled in the art can appropriately select a protein that strongly or specifically binds to the surface structure.

In one embodiment, the virus-derived construct plasmids of the present disclosure may contain a gene encoding a protein obtained by attenuating the protein of the origin virus of the virus-derived construct to be produced. The virus-derived construct plasmids of the present disclosure can contain a gene encoding any known attenuated viral protein.

In one embodiment, the present disclosure provides a plasmid-containing composition containing a population of virus-derived construct plasmids of the present disclosure. In one embodiment, the plasmid-containing composition can have a covalently closed circular (CCC) purity of the plasmid of about 60% or more, about 70% or more, about 75% or more, about 80% or more, about 85% or more, about 90% or more, or about 95% or more. The virus-derived construct plasmid of the present disclosure and the virus-derived construct plasmid-containing composition of the present disclosure produced by the method of the present disclosure can have structural characteristics that are not fully analyzable or significantly difficult to analyze, such as in the steric conformation or base modification of plasmids. Therefore, the characteristic of being produced by the method of the present disclosure is one of the characteristics of appropriately expressing the virus-derived construct plasmid of the present disclosure and the virus-derived construct plasmid-containing composition of the present disclosure.

### • Adenovirus-derived construct

An adenovirus is a virus belonging to the genus Mastadenovirus in the family Adenoviridae, and the viral particle is composed of a nucleocapsid and a double-stranded linear DNA genome, and has an icosahedral structure of 90 to 100 nm. Infection of an adenovirus is initiated by adsorption of the viral capsids to the coxsackie-adenovirus receptor (CAR) on the cell surface, and then the virus can enter into the cell via the integrins on the cell surface. Thereafter, the viral genome that escapes from the lysosome can reach the nucleus, resulting in replication of the viral genome. Viral replication is initiated by first expressing E1A protein and activating expression of other early proteins E1B, E2, E3, and E4. In the viral genome, a complex, in which a terminal protein (TP) expressed from E2 and deoxycytidine are covalently bound to each other and a polymerase is further bound thereto, is formed, and replication is initiated. The genome also contains five late transcription units (L1, L2, L3, L4, and L5) encoding structural proteins, including penton (L2), hexon (L3), scaffold protein (L4), and fiber protein (L5), under the control of a single promoter. Both ends of the genome contain inverted terminal repeats (ITRs) required for virus replication. The structural proteins of the virus are translated in the cytoplasm and then migrate into the nucleus to constitute viral particles, such that the structural proteins recognize the packaging signal (ψ) of the viral genome and package the genome. In addition, an adenovirus produces protein-non-coding VA RNAs, which are encoded by the VA gene. The adenoviral particles can have L2 and L3 capsids.

To date, 52 human adenovirus antigenic types have been identified and classified into 6 subgroups based on hemagglutination properties and sequence homology: Subgroup A (for example, serotypes 12, 18, and 31), Subgroup B (for example, serotypes 3, 7, 11, 14, 16, 21, 34, 35, and 50), Subgroup C (for example, serotypes 1, 2, 5, and 6), Subgroup D (for example, serotypes 8, 9, 10, 13, 15, 17, 19, 20, 22 to 30, 32, 33, 36 to 39, and 42 to 48), Subgroup E (for example, serotype 4), Subgroup F (for example, serotypes 40 and 41), and unclassified serotype groups (for example, serotypes 49 and 51).

In one embodiment, the adenovirus-derived construct plasmids contain at least one of the nucleic acid sequences required to construct an adenovirus-derived construct, and a desired gene. In one embodiment, the adenovirus-derived construct plasmid of the present disclosure contains a desired gene between a 5' ITR and a 3' ITR. In one embodiment, the adenovirus-derived construct plasmids contain a desired gene, a promoter, and a terminator between the 5' ITR and the 3' ITR. In one embodiment, the adenovirus-derived construct plasmid does not contain one or a plurality (for example, all) of the nucleic acid sequences required to construct an adenovirus-derived construct between the 5' ITR and the 3' ITR. In one embodiment, the adenovirus-derived construct plasmid is configured to function in a producer cell so that the virus-derived construct plasmid and the producer cell contain the nucleic acid sequences required to construct an adenovirus-derived construct. In one embodiment, one or a plurality (for example, all) of the genes not contained in the virus-derived construct plasmid among the nucleic acid sequences required to construct an adenovirus-derived construct may be encoded by the chromosomes of the producer cell.

In one embodiment, the nucleic acid sequence required to construct an adenovirus-derived construct can contain a gene encoding E1A, E1B, E2A, E2B, E3, E4, L1, L2, L3, L4, L5, IX, and IVa2. In one embodiment, the nucleic acid sequence required to construct an adenovirus-derived construct can be all genes of adenovirus other than E3. In one embodiment, the nucleic acid sequence required to construct an adenovirus-derived construct can contain a nucleic acid sequence encoding a structural protein (L2 or L3), a nucleic acid sequence encoding a packaging factor (E1A, E1B, E2A, E2B, or E4), two terminal repeats (5' ITR and 3' ITR), and a nucleic acid sequence encoding a functional cofactor. In one embodiment, the nucleic acid sequence encoding a functional cofactor can be all adenoviral genes other than L2, L3, E1A, E1B, E2A, E3, E2B, E4, a 5' ITR, and a 3' ITR. In one embodiment, the adenovirus-derived construct plasmid may not contain one or a plurality of VA, E1A, E1B, E2A, E2B, and E4, and in one embodiment, the nucleic acid sequence required to construct an adenovirus-derived construct contains genes. In certain embodiments, the adenovirus-derived construct plasmid does not contain E1A and E1B. In one embodiment, the adenovirus-derived construct plasmid is deficient in E1A, and a desired gene may be inserted into the deficient site. VA RNA is known to interact with exportin, RISK, Dicer, and the like in the human, and the deletion of VA from the adenovirus-derived construct plasmid can reduce side effects to adenovirus-derived construct infected cells. Since E1A, E1B, E2A, E2B, and E4 can be genes required for adenovirus replication, an adenovirus-derived construct plasmid that is deficient in these genes can have reduced replicative ability in infected cells.

In one embodiment, the adenovirus-derived construct of the present disclosure can be derived from human subgroup C, for example, serotype 2 or serotype 5. In one embodiment, the adenovirus-derived construct of the present disclosure can be derived from serotype 12 (Subgroup A), serotype 7 or serotype 35 (Subgroup B), serotype 30 or serotype 36 (Subgroup D), serotype 4 (Subgroup E), or serotype 41 (Subgroup F). Examples of the producer cells for producing an adenovirus-derived construct include cells such as HEK293, HEK293T, HEK293F, Hela, and Sf9.

### • Adeno-associated virus-derived construct

An adeno-associated virus (AAV) is a linear, a single-stranded DNA virus from the genus Dependovirus in the family Parvoviridae, with viral particles of 20 to 26 nm in diameter. Adenovirus elements are required for AAV proliferation. At the quantitative end of the AAV genome, there is a T-shaped hairpin structure called an inverted terminal repeat (ITR). A portion of the ITR serves as a starting point of replication and serves as a primer. In addition, the ITR is also required for packaging into viral particles and integration into chromosomal DNA of a host cell. In the left half of the genome, there is a rep gene encoding a non-structural protein, that is, a regulatory protein (Rep78, Rep76, Rep52, or Rep40) responsible for replication and transcription, and in the right half of the genome, there is a cap gene encoding three capsid proteins of structural proteins (VP1, VP2, and VP3).

The life cycle of AAV is divided into latent infection and lytic infection. The former is a case of infection by itself, and is characterized by being incorporated into the AAVS1 region (19q13.3-qter) of the long arm of chromosome 19 of the host cell. The incorporation is due to non-homologous recombination and involves Rep. It has been reported that Rep78/Rep76 binds to a base sequence (GAGC repeat sequence) commonly present in the AAVS1 region and the Rep binding region of ITR. Thus, it is considered that when target cells are infected with wild-type AAV, Rep binds to the ITR and AAVS1 of AAV, and site-specific integration of AAV genome into chromosome 19 occurs via Rep. In a case where a helper virus such as an adenovirus is simultaneously infected, when a cell latently infected with AAV is further coinfected with the helper virus, replication of AAV occurs, and a large amount of virus is released due to cell destruction (lytic infection).

In one embodiment, the AAV-derived construct plasmid contains at least one of the nucleic acid sequences required to construct an AAV-derived construct, and a desired gene. In one embodiment, the AAV-derived construct plasmid of the present disclosure contains a desired gene between a 5' ITR and a 3' ITR. In one embodiment, the AAV-derived construct plasmid contains a desired gene, a promoter, and a terminator between the 5' ITR and the 3' ITR. In one embodiment, the AAV-derived construct plasmid does not contain one or a plurality (for example, all) of the nucleic acid sequences required to construct an AAV-derived construct between the 5' ITR and the 3' ITR. In one embodiment, the AAV-derived construct plasmid is configured to function in a producer cell so that the virus-derived construct plasmid and the producer cell contain the nucleic acid sequences required to construct an AAV-derived construct. In one embodiment, one or a plurality (for example, all) of the genes not contained in the virus-derived construct plasmid among the nucleic acid sequences required to construct an AAV-derived construct may be encoded by the chromosomes of the producer cell.

In one embodiment, the nucleic acid sequence required to construct an AAV-derived construct can be a 5' ITR, rep, cap, an assembly activating protein (AAP), a membrane associated accessory protein (MAAP), and a 3' ITR of AAV, and the adenovirus E1A, E1B, E2A, VA, or E4. In one embodiment, the nucleic acid sequence required to construct an AAV-derived construct can include a nucleic acid sequence encoding an AAV structural protein (cap), a nucleic acid sequence encoding a packaging factor for AAV (rep), two terminal repeats of AAV (5' ITR and 3' ITR), and a nucleic acid sequence encoding a functional cofactor (adenovirus E1A, E1B, E2A, VA, or E4). In one embodiment, the AAV-derived construct plasmid may not contain one or a plurality of rep, cap, VA, E1A, E1B, E2A, and E4, and in one embodiment, the nucleic acid sequence required to construct an adenovirus-derived construct contains these sequences.

AAV serotypes 1 to 12 based on capsid have been reported. In addition, serotypes of rh10, DJ, DJ/8, PHP.eB, PHP.S, AAV2-retro, AAV2-QuadYF, AAV2.7m8, AAV6.2, rh.74, AAV2.5, AAV-TT, and Anc80 have also been reported. The AAV-derived construct of the present disclosure may be produced based on AAV of a suitable serotype depending on the target tissue. For example, the relationship of (serotype):(target tissue) may be selected as follows: (AAV1):(mmmmuscle, liver, respiratory tract, and nerve cells), (AAV2):(muscle, liver, and nerve cells), (AAV3):(muscle, liver, and nerve cells), (AAV4):(muscle and ependymal cells), (AAV5):(muscle, liver, nerve cells, glial cells, and respiratory tract), (AAV6):(muscle, liver, respiratory tract, and nerve cells), (AAV7):(muscle and liver), (AAV8):(muscle and liver), and (AAV9):(muscle, liver, and respiratory tract). Examples of the producer cells for producing an AAV-derived construct include cells such as HEK293, HEK293T, HEK293F, Hela, and Sf9. Examples of AAV capsids include wild-type as well as capsids with targeted mutations (AAV2i8, AAV2.5, AAV-TT, AAV9.HR, and the like), capsids with random mutations (AAV-PHP.B and the like), and capsids designed in silico (Anc 80 and the like), and in one embodiment, the AAV-derived construct of the present disclosure may contain these modified capsids and the AAV-derived construct plasmids of the present disclosure may be constructed to encode these modified capsids. As used herein, in a case where a nucleic acid sequence is derived from a particular serotype, it is intended that the nucleic acid sequence may encode a wild-type capsid or a capsid modified as described above based on the wild-type capsid.

### • Retrovirus-derived construct

As used herein, the term "retrovirus" generally refers to a virus of the family Retroviridae. A retrovirus is a double-stranded RNA enveloped virus primarily characterized by its ability to reverse transcribe the genome from RNA to DNA. The virion is about 100 to 120 nm in length and in diameter, and contains a dimeric genome of identical positive-stranded RNA complexed with nucleocapsid proteins. The genome is encapsulated in a capsid containing enzyme proteins required for viral infection, that is, reverse transcriptase, integrase, and protease. The matrix proteins form the outer layer of the capsid core that interacts with the envelope, which is a lipid bilayer derived from the host cell membrane, surrounding the viral nuclear particle. Viral envelope glycoproteins that recognize a specific receptor on the host cell and initiate the infection process are immobilized on the bilayer. Envelope proteins are formed by two subunits, transmembrane (TM), which immobilizes proteins within lipid membranes, and surface (SU), which binds to cellular receptors.

Examples of the retrovirus include, but are not limited to, murine leukemia virus (MLV), human immunodeficiency virus (HIV), equine infectious anemia virus (EIAV), mouse mammary tumor virus (MMTV), Rous sarcoma virus (RSV), Fujinami sarcoma virus (FSV), Moloney murine leukemia virus (Mo-MLV), FBR murine osteosarcoma virus (FBR MSV), Moloney murine sarcoma virus (Mo-MSV), Abelson murine leukemia virus (A-MLV), avian myelocytomatosis virus 29 (MC29), avian erythroblastosis virus (AEV), and a lentivirus. Lentiviruses can be divided into a "primate lentivirus" and a "non-primate lentivirus". Examples of the primate lentivirus include human immunodeficiency virus (HIV), which is the causative agent of human acquired immunodeficiency syndrome (AIDS), and simian immunodeficiency virus (SIV). The non-primate lentiviral group includes the prototype "slow virus" visna-maedi virus (VMV), and the related caprine arthritis encephalitis virus (CAEV), equine infectious anemia virus (EIAV), and the more recently described feline immunodeficiency virus (FIV) and bovine immunodeficiency virus (BIV).

During the infection process, the retrovirus first is attached to a specific cell surface receptor. Upon entry into a susceptible host cell, the retroviral RNA genome is copied into DNA by reverse transcriptase. This DNA is transported to the host cell nucleus and then integrated into the host genome, and this state is called a provirus. The provirus is stable in host chromosomes during cell division and is transcribed like other cellular proteins. The provirus encodes the proteins and packaging mechanisms required to produce more viruses, and can sprout and leave the cell. When the retrovirus emerges from host cells, the retrovirus has a host cell lipid membrane. In this way, the host cell-derived membrane protein becomes portion of the retroviral particles.

The retroviral genome contains four genes, gag (group specific antigen), pro (protease), pol (polymerase) and env (envelope). The gag sequence encodes three major structural proteins: a matrix protein, a nucleocapsid protein, and a capsid protein. The pro sequence encodes a protease responsible for cleaving Gag and Gag-Pol during particle assembly, emergence, and maturation. The pol sequence encodes the enzymes of reverse transcriptase and integrase, and the former catalyzes the reverse transcription of the viral genome from RNA to DNA during the infection process, while the latter is responsible for processing the LTR and integrating the proviral DNA into the host cell genome. The env sequence encodes both SU and TM subunits of the envelope glycoprotein. The ability of retrovirus to bind to its target host cells using specific cell surface receptors is conferred by the surface component (SU) of the Env protein, whereas the ability of retrovirus to enter cells via membrane fusion can be conferred by the membrane-anchored transmembrane component (TM). The retroviral genome contains elements required to promote gene expression, reverse transcription, and integration into the host cell chromosome, and examples of the elements include two LTRs (long terminal repeats), a packaging signal (ψ) sequence required for specific packaging of viral RNA into a newly formed virion, and a polypurine tract (PPT) that functions as a site to initiate positive-strand DNA synthesis during reverse transcription. Long terminal repeats (LTR) are about 600 nt in length, of which the U3 region is 450, the R sequence is 100, and the U5 region is about 70 nt in length.

The genome of a complex retrovirus, such as a lentivirus, can contain, in addition to gag, pro, pol, and env, accessory genes that regulate viral gene expression, infectious particle assembly, and modulate viral replication in infected cells. A representative lentivirus is HIV-1. The lentivirus can contain two regulatory genes, tat, and rev. For example, HIV-1 further contains vif, vpr, vpu, and nef. In addition, accessory genes such as vpx are present. These accessory genes are involved in the synthesis and processing of viral RNA and control of other replication functions. In particular, HIV also contains structural landmarks (TAR, RRE, PE, SLIP, CRS, and INS) and the like. The lentiviral particles can contain the capsid protein of p24.

In one embodiment, the retrovirus-derived construct plasmid contains at least one of the nucleic acid sequences required to construct a retrovirus-derived construct, and a desired gene. In one embodiment, the retrovirus-derived construct plasmid of the present disclosure contains a desired gene between a 5' LTR and a 3' LTR. In one embodiment, the retrovirus-derived construct plasmid may contain a primer binding site (PBS) and/or a polypurine tract (PPT) between the 5' LTR and the desired gene. In one embodiment, the retrovirus-derived construct plasmid may contain a woodchuck hepatitis virus post-transcriptional regulatory element (WPRE) between the desired gene and the 3' LTR. In one embodiment, the retrovirus-derived construct plasmid is configured to function in a producer cell so that the virus-derived construct plasmid and the producer cell contain the nucleic acid sequences required to construct a retrovirus-derived construct. In one embodiment, one or a plurality (for example, all) of the genes not contained in the virus-derived construct plasmid among the nucleic acid sequences required to construct a retrovirus-derived construct may be encoded by the chromosomes of the producer cell. In one embodiment, env may be replaced with a gene encoding VSV-G (glycoprotein G of vesicular stomatitis virus (VSV)) during construction of a retrovirus-derived construct. In one embodiment, the retrovirus-derived construct (including a lentivirus) plasmid may additionally contain the VSV-G gene. In one embodiment, when the retrovirus-derived construct (including a lentivirus) is constructed, a fusion glycoprotein (FuG-B) in which the glycoprotein gene of rabies virus (RV-G) or the intracellular domain of RV-G is replaced with that of the vesicular stomatitis virus glycoprotein (VSV-G) may be used in addition to or in place of env, and the retrovirus-derived construct (including a lentivirus) plasmids may contain these genes.

In one embodiment, the nucleic acid sequence required to construct a retrovirus-derived construct can contain a nucleic acid sequence encoding a structural protein (gag), a packaging factor (ψ), two terminal repeats (5' LTR and 3' LTR), and a nucleic acid sequence encoding a functional cofactor. In one embodiment, the nucleic acid sequence encoding a functional cofactor can be pro, pol, and env. In one embodiment, the retrovirus-derived construct plasmid may not contain one or a plurality of gag, pro, pol, and env, and in one embodiment, the nucleic acid sequence required to construct a retrovirus-derived construct contains these genes. Since pol can be a gene required for retrovirus replication, a retrovirus-derived construct plasmid that is deficient in these genes can have reduced replicative ability in infected cells. In one embodiment, the retrovirus-derived construct plasmid does not contain one or a plurality (for example, all) of the nucleic acid sequences required to construct a retrovirus-derived construct between the 5' LTR and the 3' LTR. In one embodiment, the retrovirus-derived construct plasmid of the present disclosure contains a desired gene between a 5' LTR and a 3' LTR. In one embodiment, the retrovirus-derived construct plasmid contains a desired gene, a promoter, and a terminator between the 5' LTR and the 3' LTR. In one embodiment, the retrovirus-derived construct plasmid may be modified to be deficient in the U3 region of the LTR. Examples of the producer cells for producing a retrovirus-derived construct include cells such as HEK293.

In one embodiment, the nucleic acid sequence required to construct a lentivirus-derived construct can contain a nucleic acid sequence encoding a structural protein (gag, pol, VSV-G, or env), a packaging factor (packaging signal (ψ) sequence), two terminal repeats (5' LTR and 3' LTR), and a nucleic acid sequence encoding a functional cofactor. In one embodiment, the nucleic acid sequence encoding a functional cofactor can be rev. In one embodiment, the lentivirus-derived construct plasmid may not contain one or a plurality of rev, gag, pro, pol, and env, and in one embodiment, the nucleic acid sequence required to construct a lentivirus-derived construct contains these genes. Since pol and rev can be a gene required for lentivirus replication, a lentivirus-derived construct plasmid that is deficient in these genes can have reduced replicative ability in infected cells. In one embodiment, the lentivirus-derived construct plasmid may contain one or a plurality of tat, vif, vpr, vpu, nef, vpx, TAR, RRE, PE, SLIP, CRS, INS, APP, MAAP, RPE, PPT, PRE, WRPE, and oPRE. In one embodiment, the lentivirus-derived construct plasmid does not contain one or a plurality (for example, all) of the nucleic acid sequences required to construct a lentivirus-derived construct between the 5' LTR and the 3' LTR. In one embodiment, the lentivirus-derived construct plasmid of the present disclosure contains a desired gene between a 5' LTR and a 3' LTR. In one embodiment, the lentivirus-derived construct plasmid contains a desired gene, a promoter, a terminator, and WPRE between the 5' LTR and the 3' LTR. In one embodiment, the lentivirus-derived construct plasmid may be modified to be deficient in the U3 region of the LTR and TAT. In one embodiment, the retrovirus-derived construct plasmid may be modified to be deficient in the U3 region of the LTR. Examples of the producer cells for producing a lentivirus-derived construct include cells such as HEK293T, HEK293, and Hela.

In one embodiment, the present disclosure provides a virus-derived construct-containing composition containing a population of virus-derived constructs of the present disclosure. In one embodiment, the virus-derived construct-containing composition may have a viral vector particle number with respect to a viral vector genome copy number (VP/VG) of 35, 30, 25, 20, 15, 10, or 5 or less. The virus-derived construct of the present disclosure and the virus-derived construct-containing composition of the present disclosure produced by the method of the present disclosure can have structural characteristics that are not fully analyzable or significantly difficult to analyze, such as in the conformation or surface modification of the virus-derived construct. Therefore, the characteristic of being produced by the method of the present disclosure is one of the characteristics of appropriately expressing the virus-derived construct of the present disclosure and the virus-derived construct-containing composition of the present disclosure.

### (Production of virus-derived construct plasmids)

In one embodiment, the present disclosure provides a method for producing a virus-derived construct plasmid of the present disclosure, the method including a step of operably linking a plurality of nucleic acid sequences. In one embodiment, the method for producing a virus-derived construct plasmid of the present disclosure can include: a step of obtaining a virus-derived construct plasmid containing at least one of nucleic acid sequences required to construct a virus-derived construct from a first microbial host cell; and ii) a step of introducing the virus-derived construct plasmid into a second microbial host cell and placing the second microbial host cell under conditions in which the virus-derived construct plasmid is amplified. In one embodiment, the first microbial host cell can be a transformed organism (Bacillus subtilis or the like). In one embodiment, the second microbial host cell can be Escherichia coli. In one embodiment, the method for producing a virus-derived construct plasmid of the present disclosure includes a step of introducing a nucleic acid containing a nucleic acid sequence for producing a virus-derived construct containing a sequence that is replicated in Bacillus subtilis into a host cell to form a plasmid in the host cell. As used herein, in one embodiment, Bacillus subtilis may have the ability to form a plasmid from a nucleic acid incorporated from the outside, and thus in the method, the nucleic acid to be introduced may not be a plasmid. For example, when Bacillus subtilis is brought into contact with a nucleic acid (for example, an acyclic nucleic acid having a tandem repeat nucleic acid sequence), Bacillus subtilis can take up the nucleic acid and form a plasmid in Bacillus subtilis (see, for example, the OGAB method described As used herein).

In one embodiment, when the virus-derived construct plasmid is produced, a nucleic acid sequence (As used herein, described as a vector partial nucleic acid sequence) other than the nucleic acid sequence required to construct the virus-derived construct can be fragmented into a plurality of fragments. In the OGAB method, a plasmid is constructed using nucleic acid fragments, and a vector portion can also be flexibly designed by fragmenting a vector partial nucleic acid sequence in combination with and/or independently of a nucleic acid sequence required to construct a virus-derived construct and subjecting the vector partial nucleic acid sequence to the OGAB method. Flexibility in the design of the vector portion may allow for miniaturization of the vector plasmid, as the vector portion that can be commonly used for construction of various viral vector plasmids may also include portions that are unnecessary for construction of individual viral vector plasmids. **In** one embodiment, the step of obtaining the virus-derived construct plasmid containing at least one of the nucleic acid sequences required to construct a virus-derived construct from the first microbial host cell includes obtaining a virus-derived construct plasmid from a plurality of nucleic acid fragments containing at least two (for example, 2 to 20, 2 to 10, 2 to 5, 3 to 20, 3 to 10, 3 to 5, 4 to 20, 4 to 10, 5 to 20, 5 to 10, or the like) nucleic acid fragments containing vector partial nucleic acid sequences.

The vector portion usually contains a nucleic acid sequence having a function different from that of an element (for example, a sequence encoding a capsid, a sequence controlling a capsid, and the like) related to the production of a virus-derived construct, and may contain an element or the like for facilitating the handling of the viral vector plasmid. **In** one embodiment, the vector partial nucleic acid sequence includes a nucleic acid sequence having the function of nucleic acid replication, drug resistance, copy number adjustment, improved stability, or improved operability by genetic engineering (design of multiple cloning sites). For example, the vector partial nucleic acid sequence may include a nucleic acid sequence of a replication starting point, a drug resistance gene, a promoter, a centromere sequence, a transgene, a replication regulatory gene, a transfer regulatory gene, a toxin gene, an antitoxin gene, a homologous recombination site, or a multiple cloning site. The vector partial nucleic acid sequence can be modularized (blocked) for each functional unit, where one module may be included in one nucleic acid fragment or may be included in a plurality of nucleic acid fragments. By preparing a plurality of types of nucleic acid fragments as modules having similar functions such as drug resistance, a novel virus-derived construct plasmid can be efficiently produced.

**In** one embodiment, the present disclosure provides a method for producing virus-derived construct plasmids of the present disclosure, the method including producing adeno-associated virus-derived construct plasmids in Bacillus subtilis, virus-derived construct plasmids produced by such a method, and a virus-derived construct. A virus-derived gene is often difficult to replicate by a host cell due to generation of mutation, inhibition of proliferation to the host cell, or the like. In particular, ITRs contained in AAV are likely to be mutated in Escherichia coli, making replication difficult. In addition, even with current techniques, replication of virus-derived construct plasmids is not readily achievable in any host cell. It is not easy to form and replicate the virus-derived construct plasmids by changing the host to Bacillus subtilis while various efforts are made in the replication of the virus-derived construct plasmids by Escherichia coli. Formation and production of virus-derived construct plasmids in Bacillus subtilis was enabled for the first time through the experiments of the present disclosure.

In one embodiment, the method for producing a virus-derived construct plasmid of the present disclosure may include a step of removing a portion of the virus-derived construct plasmid of the present disclosure (for example, obtained by the method for producing a virus-derived construct plasmid of the present disclosure) and incorporating (cloning) the portion of the virus-derived construct plasmid into another plasmid (may be the virus-derived construct plasmid of the present disclosure). A portion of the other plasmid may be removed during incorporation. For example, in a case where a nucleic acid sequence that provides a higher amount of virus-derived construct produced is found in one or a plurality of the nucleic acid sequences (A) to (D) of the nucleic acid sequences required to construct a virus-derived construct, an improvement in the amount of the virus-derived construct produced can be obtained by replacing the nucleic acid sequence with the corresponding nucleic acid sequences (A) to (D) of the existing virus-derived construct plasmid. For example, when a virus-derived construct plasmid containing a promoter that leads to excellent expression of a desired gene when located upstream of a specific desired gene is obtained, a virus-derived construct that highly expresses the desired gene can be obtained by incorporating the promoter and the nucleic acid sequence in the region of the desired gene into the existing virus-derived construct plasmid.

In one embodiment, the method for introducing a nucleic acid into Bacillus subtilis can be any method, and examples thereof include the use of competent Bacillus subtilis, an electroporation method, a method using a particle gun (gene gun), and a calcium phosphate method. The term "competent" refers to a state in which a cell becomes more permeable to exogenous substances (for example, nucleic acids). Any known method can be used to render Bacillus subtilis competent, and for example, the method described in Anagnostopoulou, C. and Spizizen, J. J. Bacteriol., 81, 741-746 (1961) can be used. In one embodiment, the virus-derived construct plasmid of the present disclosure may be produced in Bacillus subtilis and directly amplified in the same Bacillus subtilis.

When a nucleic acid is introduced into Bacillus subtilis to produce a virus-derived construct plasmid having a structure different from the nucleic acid, the nucleic acid to be introduced may contain each element of the virus-derived construct plasmid described in detail above.

### (OGAB method)

In one embodiment, the virus-derived construct plasmid of the present disclosure can be produced by the OGAB method. The Ordered Gene Assembly in Bacillus subtilis (OGAB) method is a method of incorporating an integrated nucleic acid into a transformed organism to produce circular plasmids in the organism. In the OGAB method, plasmids containing large sized nucleic acids can be conveniently prepared. The nucleic acid to be incorporated into the transformed organism is referred to as an "integrated nucleic acid" As used herein, typically, the integrated nucleic acid has a tandem repeat-like structure in which one unit of the plasmid and one set of unit nucleic acids repeatedly appear in the same direction, and the use of the integrated nucleic acid having such a structure can promote the production of a plasmid in the transformed organism. As used herein, the term "unit nucleic acid" refers to a nucleic acid molecule or a portion of a nucleic acid molecule having some sequences constituting the sequence of the integrated nucleic acid, and as described in detail below, a plurality of types of unit nucleic acids are prepared as a unit vector, and then the integrated nucleic acid is constructed.

Hereinafter, a procedure for producing an integrated nucleic acid to be incorporated into a transformed organism will be described.

### • Preparation of unit nucleic acids

A unit nucleic acid for incorporation into an integrated nucleic acid is prepared. The unit nucleic acid can be produced by any known method, for example, by polymerase chain reaction (PCR) or chemical synthesis. The unit nucleic acid can contain any desired sequence, such as a sequence encoding a desired protein (a therapeutic protein, a protein constituting a virus-derived construct, or the like) or a portion thereof, a sequence controlling a gene (a promoter, an enhancer, or the like), or a sequence for manipulating a nucleic acid (a restriction enzyme recognition sequence or the like). The ends of each unit nucleic acid can be configured to produce a specific protruding sequence so that a plurality of types of unit nucleic acids are aligned in a particular order and/or specific direction when incorporated into an integrated nucleic acid.

One or a plurality of unit nucleic acids can be designed to encode one or a plurality of genes having a long base length, as a large number of unit nucleic acids can eventually be integrated on the plasmid. Examples of the gene having a long base length include a group of genes constituting a series of metabolic pathways.

### • Preparation of unit vector

A unit vector can be prepared by linking a unit nucleic acid and an additional nucleic acid different from the unit nucleic acid. The use of the unit vector can allow for easier handling of unit nucleic acids.

The additional nucleic acid may be a linear nucleic acid or a circular plasmid. In a case where a circular plasmid is used as an additional nucleic acid, a unit vector can also have a circular structure, and thus can be used for transformation of Escherichia coli and the like, for example. In one embodiment, the additional nucleic acid can contain a replication starting point so that the unit vector is replicated in the introduced host. In one embodiment, all the unit nucleic acids for constructing an integrated nucleic acid can be linked to the same type of additional nucleic acid, thereby reducing a difference in size between the unit vectors and facilitating the handling of the plurality of types of unit vectors. In one embodiment, a unit nucleic acid for constructing an integrated nucleic acid may be linked to different types of additional nucleic acids. In one embodiment, for one or a plurality of unit nucleic acids for constructing an integrated nucleic acid, a ratio of (base length of unit nucleic acid)/(base length of unit vector) or an average thereof can be 50% or less, 40% or less, 30% or less, 20% or less, 15% or less, 10% or less, 7% or less, 5% or less, 2% or less, 1.5% or less, 1% or less, or 0.5% or less. As the size of the additional nucleic acid is larger than that of the unit nucleic acid, more uniform handling of different types of unit vectors can be possible. The linking between the unit nucleic acid and the additional nucleic acid can be performed by any method such as ligation using the DNA ligase or TA cloning method. In one embodiment, for one or the plurality of unit nucleic acids for constructing an integrated nucleic acid, the ratio of (base length of unit nucleic acid)/(base length of unit vector) or the average thereof can be 1% or more, 0.3% or more, 0.1% or more, 0.03% or more, 0.01% or more, 0.003% or more, or 0.001% or more, and the manipulation of the unit vector can be facilitated.

In one embodiment, the length of the unit nucleic acid can be 10 bp or more, 20 bp or more, 50 bp or more, 70 bp or more, 100 bp or more, 200 bp or more, 500 bp or more, 700 bp or more, 1,000 bp or more, or 1,500 bp or more, and 5,000 bp or less, 5,000 bp or less, 2,000 bp or less, 1,500 bp or less, 1,200 bp or less, 1,000 bp or less, 700 bp or less, or 500 bp or less.

In one embodiment, the integrated nucleic acid can be constructed from 2 kinds or more, 4 kinds or more, 6 kinds or more, 8 kinds or more, 10 kinds or more, 15 kinds or more, 20 kinds or more, 30 kinds or more, 40 kinds or more, 50 kinds or more, 60 kinds or more, 70 kinds or more, 80 kinds or more, 90 kinds or more, kinds or 100 kinds or more, and 1,000 kinds or less, 700 kinds or less, 500 kinds or less, 200 kinds or less, 120 kinds or less, 100 kinds or less, 80 kinds or less, 70 kinds or less, 60 kinds or less, 70 kinds or less, kinds or 50 kinds or less unit nucleic acids. By adjusting the numbers of moles of the respective unit nucleic acids (kinds or unit vectors) to be substantially the same, a desired integrated nucleic acid having a tandem repeat-like structure can be efficiently produced.

In one embodiment, the unit nucleic acid may have a base length obtained by approximately equally dividing a set of repetitive sequences in the integrated nucleic acid by the number of unit nucleic acids. This can facilitate a manipulation of aligning the numbers of moles of the respective unit nucleic acids (or unit vectors). In one embodiment, the unit nucleic acid can have an increased or decreased base length of 30% or less, 25% or less, 20% or less, 15% or less, 10% or less, 7% or less, or 5% or less from a base length obtained by approximately equally dividing a set of repetitive sequences in the integrated nucleic acid by the number of unit nucleic acids.

In one embodiment, the unit nucleic acid can be designed to have a non-palindromic sequence (a sequence that is not a palindromic sequence) at the end of the unit nucleic acid. The unit nucleic acid designed as described above can easily provide a structure in which the unit nucleic acids are linked to each other while maintaining the order in the integrated nucleic acid when the non-palindromic sequence is made into a protruding sequence.

### • Production of integrated nucleic acid

The integrated nucleic acid can be constructed by linking the unit nucleic acids to each other. In one embodiment, the unit nucleic acid can be prepared by being cut out from the unit vector by a restriction enzyme or the like. An integrated nucleic acid can contain 1 or more, 2 or more, 3 or more, 4 or more, 5 or more, 6 or more, 7 or more, 8 or more, 9 or more, or 10 or more sets of repetitive sequences. The repetitive sequence in the integrated nucleic acid can include a sequence of a unit nucleic acid, and as necessary, a sequence of an integrated vector nucleic acid. The integrated nucleic acid can contain a sequence that enables replication of the nucleic acid in a transformed organism. In one embodiment, a sequence that enables replication of the nucleic acid in a transformed organism can contain a replication starting point effective in the transformed organism (for example, Bacillus bacteria (Bacillus subtilis)). The sequence of the replication starting point effective in Bacillus subtilis is not particularly limited, and examples of a sequence having a θ-type replication mechanism include a sequence of an origin of replication contained in plasmids such as pTB19 (Imanaka, T., et al. J. Gen. Microbioi. 130, 1399-1408 (1984)) or pLS32 (Tanaka, T and Ogra, M. FEBS Lett. 422, 243-246 (1998)), and pAMβ1 (Swinfield, T. J., et al. Gene 87, 79-90 (1990)).

The integrated nucleic acid may contain an additional base sequence as necessary in addition to the unit nucleic acid. In one embodiment, the integrated nucleic acid may contain a base sequence that controls transcription translation, such as a promoter, an operator, an activator, or a terminator. Specific examples of the promoter in the case of using Bacillus subtilis as a host include Pspac (Yansura, D. and Henner, D. J. Pro. Natl. Acad. Sci, USA 81, 439-443 (1984.)) and Pr promoter (Itaya, M. Biosci. Biotechnol. Biochem. 63, 602-604 (1999)), which can be regulated in expression by isopropyl s-D-thiogalactopyranoside (IPTG).

The unit nucleic acid can form a repetitive structure that maintains a certain order and direction in the integrated nucleic acid. In one embodiment, the unit nucleic acid is constructed so that the base sequences at the protruding ends of the unit nucleic acid cut out from the unit vector are complementary to each other, such that it is possible to form a repetitive structure in which a certain order and direction in the integrated nucleic acid are maintained. In one embodiment, by making the structure of the protruding end unique for each different unit nucleic acid, it is possible to efficiently form a repetitive structure maintaining a certain order and direction. In one embodiment, the protruding end may contain a non-palindromic sequence and may be either a 5' end protruding or a 3' end protruding.

In one embodiment, a unit nucleic acid having a protruding end can be cut out from a unit vector using a restriction enzyme. In the embodiment, the unit vector can contain one or a plurality of restriction enzyme recognition sequences. In a case where the unit vector contains a plurality of restriction enzyme recognition sequences, the respective restriction enzyme recognition sequences may be recognized by the same restriction enzyme or may be recognized by different restriction enzymes. In one embodiment, the unit vector can contain a pair of regions recognized by the same restriction enzyme so that a complete unit nucleic acid region is included between these regions. The restriction enzymes used are not particularly limited, Type IIS restriction enzymes such as AarI, BbsI, BbvI, BcoDI, BfuAI, BsaI, BsaXI, BsmAI, BsmBI, BsmFI, BspMI, BspQI, BtgZI, FokI, and SfaNI can be used, and these restriction enzymes can create a protruding end at a site away from the recognition sequence outside the recognition sequence by a certain distance. When (for example, single) Type IIS restriction enzyme is used, the sequences of the protruding ends of the cut unit nucleic acid can be different for each unit nucleic acid, which can be advantageous for assembling a plurality of unit nucleic acids in a certain order and direction. In one embodiment using Type IIS restriction enzyme, the unit vector does not contain a region recognized by Type IIS restriction enzyme in the unit nucleic acid region. In an embodiment using a restriction enzyme that cleaves the recognition region, the unit vector can contain a region recognized by the restriction enzyme at the end of the unit nucleic acid region.

In one embodiment, when the same type of restriction enzyme is used to cut out the unit nucleic acid from a plurality of unit vectors, the restriction enzyme treatment can be performed in a solution containing the plurality of unit vectors, and the efficiency of the operation can be improved. The types of restriction enzymes used to produce a certain integrated nucleic acid can be, for example, 5 or less, 4 or less, 3 or less, 2 or less, or 1, and the use of a small number of restriction enzymes can reduce variations in the number of moles between the unit nucleic acids. In one embodiment, the unit nucleic acid cut out from the unit vector can be readily purified by any known fractionation method such as agarose gel electrophoresis.

The unit nucleic acid, and as necessary, the integrated vector nucleic acids can be linked (ligated) to each other using DNA ligase or the like. Accordingly, an integrated nucleic acid can be produced. For example, the linking of the unit nucleic acids, and as necessary, the integrated vector nucleic acids can be performed in the presence of components such as polyethylene glycol (for example, PEG2000, PEG4000, PEG6000, PEG8000, and the like) and salts (for example, monovalent alkali metal, sodium chloride, and the like). A concentration of each unit nucleic acid in a ligation reaction solution is not particularly limited, and can be 1 fmol/µl or more. A reaction temperature and time of the ligation are not particularly limited, and are, for example, 37°C and 30 minutes or longer. In one embodiment, before linking the unit nucleic acid, and as necessary, the integrated vector nucleic acid, a composition containing the unit nucleic acid, and as necessary, the integrated vector nucleic acid may be subjected to any conditions for inactivating the restriction enzyme (for example, phenol/chloroform treatment).

The unit nucleic acid can be adjusted to about the same number of moles using the method described in WO 2015/111248 A or the like. By adjusting the unit nucleic acids to approximately the same number of moles, a desired integrated nucleic acid having a tandem repeat-like structure can be efficiently produced. The number of moles of the unit nucleic acid can be adjusted by measuring the concentration of the unit vector or the unit nucleic acid.

### • Production of plasmids from integrated nucleic acid

Plasmids can be formed in a transformed organism by bringing an integrated nucleic acid into contact with the transformed organism. In one embodiment, examples of the transformed organism include bacteria belonging to the genus Bacillus, bacteria belonging to the genus Streptococcus, bacteria belonging to the genus Haemophilus, and bacteria belonging to the genus Neisseria. Examples of the bacteria belonging to the genus Bacillus include B. subtilis (Bacillus subtilis), B. megaterium (Bacillus megaterium), and B. stearothermophilus (Bacillus stearothermophilus). In a preferred embodiment, the transformed organism is Bacillus subtilis. In one embodiment, the transformed organism that incorporates the integrated nucleic acid is in a competent state and can actively incorporate a nucleic acid. For example, Bacillus subtilis in a competent state cleaves a double-stranded nucleic acid as a substrate on a cell, decomposes any one strand of the double-stranded nucleic acid from the cleavage point, and incorporates the other single strand into a bacterial cell. The incorporated single strand can be repaired into a circular double-stranded nucleic acid in the bacterial cell. Any known method can be used to render the transformed organism competent, and for example, Bacillus subtilis can be rendered competent using the method described in Anagnostopoulou, C. and Spizen, J. J. Bacteriol., 81, 741-746 (1961). As a method of transformation, a known method suitable for each transformed organism can be used.

In one embodiment, the virus-derived construct plasmids produced from packaging cells can be purified using any known method, and the present disclosure also provides the thus purified virus-derived construct plasmids. In one embodiment, the purified virus-derived construct plasmids containing a desired nucleic acid sequence can be confirmed by examining the size pattern of fragments generated by restriction enzyme cleavage, a PCR method, a base sequencing method, or the like. In one embodiment, the virus-derived construct plasmid-containing composition prepared by the method for producing virus-derived construct plasmids of the present disclosure may contain trace endotoxins. In one embodiment, there is provided Bacillus subtilis containing the virus-derived construct plasmids of the present disclosure. In one embodiment, the virus-derived construct plasmid of the present disclosure can be produced from at least two, at least three, at least four, at least five, or more nucleic acid fragments containing a vector partial nucleic acid sequence in a nucleic acid fragment produced by chemical synthesis or an enzymatic reaction. In one embodiment, the virus-derived construct plasmid of the present disclosure is produced by circularizing only a plurality of nucleic acid fragments produced by chemical synthesis or an enzymatic reaction. In one embodiment, the virus-derived construct plasmid of the present disclosure is produced from 3 or more, 4 or more, 5 or more, 7 or more, 10 or more, 15 or more, 20 or more nucleic acid fragments. In one embodiment, the nucleic acid fragments used to produce the virus-derived construct plasmid of the present disclosure can independently have a length of 20 kb or less, 15 kb or less, 10 kb or less, 7 kb or less, 5 kb or less, 4 kb or less, 3 kb or less, 2 kb or less, 1.5 kb or less, 1 kb or less, 0.7 kb or less, or 0.5 kb or less.

### (Composition)

In one embodiment, the present disclosure provides a composition containing the virus-derived construct plasmids or virus-derived constructs described As used herein.

### (Formulation and the like)

The composition described As used herein can be provided in a variety of forms. The form of the composition may be, for example, an injection, a capsule, a tablet, a granule, an inhalant, or the like. An aqueous solution for injection may be stored, for example, in a vial or in a stainless container. In addition, the aqueous solution for injection may contain, for example, physiological saline, sugar (for example, trehalose), NaCl, NaOH, or the like.

In one embodiment, the composition of the present disclosure contains a pharmaceutically acceptable carrier or excipient. Such a carrier can be an aseptic liquid, for example, water or oil, includes a carrier derived from petroleum, an animal, a plant, or a synthetic origin, and includes, but is not limited to, peanut oil, soybean oil, mineral oil, sesame oil, and the like. When a medicine is orally administered, water is a preferred carrier. When the pharmaceutical composition is administered intravenously, saline and aqueous dextrose are preferred carriers. Preferably, an aqueous saline solution, and aqueous dextrose and a glycerol solution are used as a liquid carrier for an injectable solution. Examples of a preferred excipient include light anhydrous silicic acid, crystalline cellulose, mannitol, starch, glucose, lactose, sucrose, gelatin, malt, rice, wheat flour, chalk, silica gel, sodium stearate, glycerol monostearate, talc, sodium chloride, powdered skim milk, glycerol, propylene, glycol, water, ethanol, carmellose calcium, carmellose sodium, hydroxypropyl cellulose, hydroxypropyl methylcellulose, polyvinyl acetal diethylamino acetate, polyvinylpyrrolidone, gelatin, medium-chain fatty acid triglyceride, polyoxyethylene hydrogenated castor oil 60, poloxamer, saccharose, carboxymethylcellulose, corn starch, and an inorganic salt. The composition can contain a small amount of a wetting agent or an emulsifier, or a pH buffer, if desired. These compositions can be in the form of a solution, suspension, emulsion, tablet, pill, capsule, powder, sustained release mixture, or the like. The composition can also be formulated as a suppository using a traditional binding agent and carrier, for example, triglyceride. Oral formulation can also contain a standard carrier such as medicine grade mannitol, lactose, starch, magnesium stearate, sodium saccharin, cellulose, or magnesium carbonate. Examples of a preferred carrier are described in E. W. Martin, Remington's Pharmaceutical Sciences (Mark Publishing Company, Easton, U.S.A). In addition, for example, a surfactant, an excipient, a coloring agent, a flavoring agent, a preservative, a stabilizer, a buffer, a suspension, an isotonizing agent, a binder, a disintegrant, a lubricant, a fluidity accelerator, a corrigent, or the like may be contained. In one embodiment, the pH of any liquid composition of the present disclosure can be about 3, about 3.5, about 4, about 4.5, about 5, about 5.5, about 6, about 6.5, about 7, about 7.5, about 8, about 8.5, about 9, about 9.5, about 10, about 10.5, about 11, or a range between any two of these values.

The optional ingredient of the composition of the present disclosure can be provided as a pharmaceutically acceptable salt, and can be, for example, a salt formed with a free carboxyl group derived from hydrochloric acid, phosphoric acid, acetic acid, oxalic acid, tartaric acid, or the like, a salt formed with a free amine group derived from isopropylamine, triethylamine, 2-ethylaminoethanol, histidine, procaine, or the like, and a salt derived from sodium, potassium, ammonium, calcium, ferric hydroxide, or the like.

In a preferred embodiment, a composition can be formulated as a pharmaceutical composition adapted for administration to humans according to a known method. Such a composition can be administered by injection. A composition for use in injection administration is typically a solution in an aseptic isotonic aqueous buffer. The composition can also contain a solubilizing agent and a local anesthetic agent such as lidocaine which alleviates the pain at the site of injection as necessary. In general, the ingredients can be supplied separately or mixed and supplied together in a unit dosage form, and supplied as a lyophilized powder or water free concentrate, for example, in a sealed container such as an ampoule or sachet showing the amount of active agent. When the composition is to be administered by injection, the composition can be distributed by using an injection bottle containing aseptic agent-grade water or saline. When the composition is to be administered by injection, aseptic water or saline ampoule for injection can also be provided so that the ingredients can be mixed prior to administration.

### (Use and application)

The virus-derived construct plasmid or virus-derived construct described As used herein, or a composition containing the same, can be used in a variety of applications, such as gene therapy, functional genomics, cancer vaccination, and/or antiviral vaccination.

When the virus-derived construct or the virus-derived construct-containing composition of the present disclosure is applied to a subject, the subject is not particularly limited, and may be a mammal (for example, a mouse, a rat, a hamster, a rabbit, a cat, a dog, a cow, sheep, a pig, a monkey, a human, or the like), a bird, a reptile, an amphibian, an arthropod, a fish, or the like.

The amount of the virus-derived construct or the virus-derived construct-containing composition of the present disclosure can vary depending on the nature of the disorder or condition to be treated or prevented, and can be determined by those skilled in the art by a standard clinical technique based on the descriptions As used herein. An in vitro assay can be used in some cases to assist the identification of the optimal dosing range. The precise dose to be used in a formulation can also vary depending on the administration route or the severity of the disease or disorder. Thus, the dose should be determined in accordance with the judgment of the attending physician or the condition of each patient. A dosage of the virus-derived construct or the virus-derived construct-containing composition of the present disclosure is not particularly limited, and may be, for example, 1 x 10⁵, 1 x 10⁶, 1 × 10⁷, 1 x 10⁸, 1 x 10⁹, 1 x 10¹⁰, 1 x 10¹¹, 1 x 10¹², 1 × 10¹³, 1 × 10¹⁴, or 1 x 10¹⁵ virus-derived constructs per dose, and may be within a range of any two of these values. The dosing interval is not particularly limited, and may be, for example, 1 or 2 administrations every 1, 7, 14, 21, or 28 days or 1 or 2 administrations per range of any two of these values. The dosage, dosing interval, and dosing method may be appropriately selected depending on the age, weight, symptom, target organ, or the like of the patient.

The administration route of the virus-derived construct or the virus-derived construct-containing composition described As used herein may be, for example, intravenous, intradermal, subcutaneous, intramuscular, intraperitoneal, intrathecal, intraventricular, intraparenchymal, pulmonary, intranasal, epidural, or oral administration. In one embodiment, the composition of the present disclosure can be used together with various delivery systems. Such systems include, for example, encapsulation in liposomes, microparticles, and microcapsules, the use of receptor-mediated endocytosis, and the like. It is also possible to administer the medicine by a suitable route, for example, by injection, bolus injection, or absorption through epithelial or mucocutaneous lining (for example, oral cavity, rectum, intestinal mucosa, or the like). In addition, an inhaler or nebulizer using an aerosolizing agent can be used as necessary. In addition, other drugs can also be administered together. The administration can also be systemic or local.

The composition of the present disclosure can be provided as a kit. In one embodiment, the present disclosure provides a kit for forming a virus-derived construct plasmid, the kit containing at least one nucleic acid including a nucleic acid sequence that promotes plasmid replication in Bacillus subtilis and a nucleic acid sequence required to constitute a virus. In one embodiment, the present disclosure provides a drug pack or kit including one or more containers filled with one or more components that can be added to the composition of the present disclosure. In some cases, information indicating approval of manufacture, use, or sale for administration to humans by a government agency regulating the manufacture, use, or sale of medicines or biological products in a stipulated form can be appended to such a container.

The formulation procedure for the composition of the present disclosure as a medicine or the like is known in the art, and is described, for example, in the Japanese Pharmacopoeia, the United States Pharmacopeia, pharmacopeia of other countries, or the like. Accordingly, those skilled in the art can determine the embodiment, such as the amount to be used, without undue experimentation from the descriptions As used herein.

As used herein, "or" is used when "at least one or more" of the items listed in the text can be employed. The same applies to "or". When explicitly described As used herein as "within the range" of "two values", the range also includes the two values themselves.

Reference literatures such as scientific literatures, patents, and patent applications cited As used herein are incorporated herein by reference to the same extent that the entirety of each document is specifically described.

The present disclosure has been described above with reference to preferred embodiments for easy understanding. Hereinafter, the present disclosure will be described based on examples, but the above description and the following examples are provided for the purpose of illustration only and not for the purpose of limiting the present disclosure. Accordingly, the scope of the present disclosure is not limited to the embodiments or examples specifically described As used herein, but is limited only by the claims.

### Examples

For reagents, the specific products described in the Examples were used. However, the reagent can be substituted with an equivalent product from another manufacturer (Sigma-Aldrich, Wako Pure Chemical Industries, Ltd., Nacalai Tesque INC., R&D Systems, USCN Life Science INC, or the like).

### (Example 1: Construction of pGETS103-AAV-Helper-RC2 by OGAB method)

### • Construction of vector DNA for OGAB integration

The plasmid pGETS103ΔAarI was cleaved with the restriction enzyme HindIII, purified by TE saturated phenol treatment, butanol extraction, and ethanol precipitation, and the linker DNA prepared by annealing the single-stranded DNAs set forth in SEQ ID NOs: 3 and 4 was inserted thereinto, thereby producing pGETS103-ΔAarI-Linker as a vector for OGAB integration. This plamid was cleaved with restriction enzyme AarI and size-fractionated by low melting point agarose gel electrophoresis, and large fragments of 15 kb were cut out from gel and purified to obtain vector fragments for OGAB integration.

### • Preparation of integrated fragments for OGAB

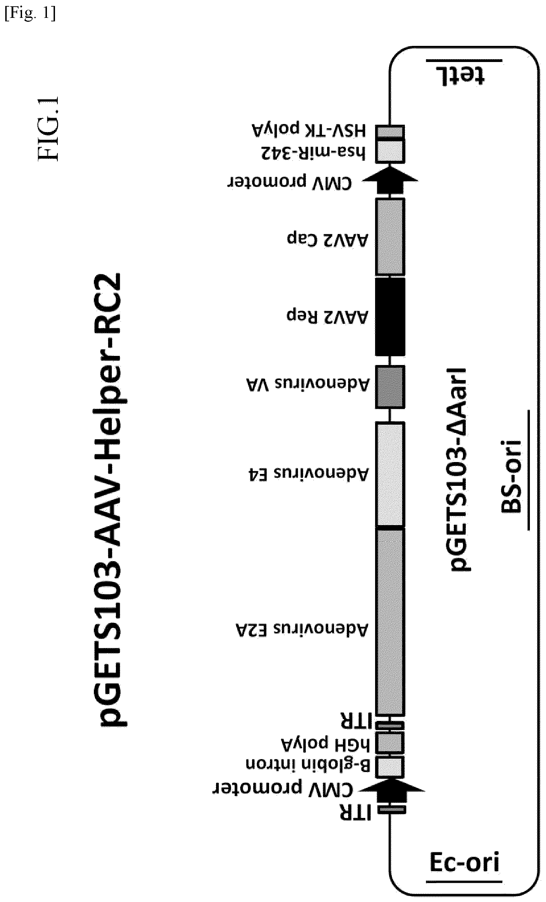

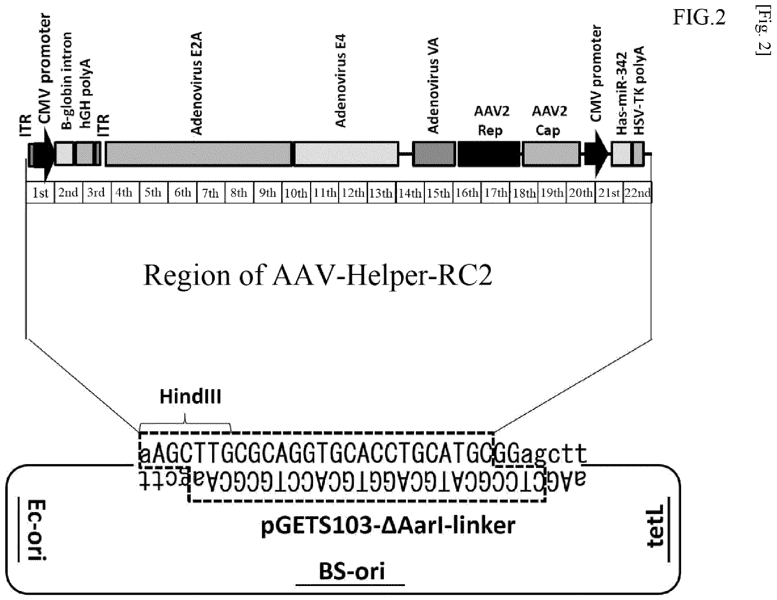

In the base sequence of pGETS103-AAV-Helper-RC2 (FIG. 1), whether construction by the OGAB method was possible for the region of AAV-Helper-RC2 (FIG. 2) excluding the base sequence of pGETS103 in the vector part was examined. First, the DNA region was designed to be divided into 22 fragments illustrated in FIG. 1. Since the 3rd fragment includes a boundary region between the AAV fragment and the Helper fragment and the 16th fragment includes a boundary region between the Helper fragment and the RC2 fragment, the 1st, 3rd, and 16th fragments were prepared by MAP method (JP 2018-93024 A) after chemically synthesizing DNA of a material as described below. Specifically, double-stranded DNAs obtained by assembly by MAP method using single-stranded DNAs of SEQ ID NOs: 5 to 10 for the 1st fragment, SEQ ID NOs: 11 to 16 for the 3rd fragment, and SEQ ID NOs: 17 to 22 for the 16th fragment were used. The remaining regions were amplified by PCR using a combination of F and R primers attached with the numbers of the respective fragments set forth in SEQ ID NOs: 23 to 66 and using pAAV-CMV Vector for the 2nd fragment, pHelper Vector for the 4th to 15th fragments, and pRC2-mi342 Vector for the 17th to 22nd fragments as templates under the following conditions. In one reaction, 10 µL of 2 × Prime Star mix (Takara Bio Inc.), 1 µL of template DNA, 1 µL of a solution containing 3.2 pmol of both primers, and 8 µL of sterilized water were added, and after 96°C for 2 minutes, 30 cycles of 98°C for 10 seconds, 55°C for 15 seconds, and 72°C for 5 seconds were performed.

### • Cloning of integrated fragment for OGAB into vector

DNA fragments were obtained by MAP method or PCR method, and then these DNAs were purified using MinElute PCR Purification Kit (Qiagen N.V.), and finally eluted from the column with 15 µL of TE buffer (Nacalai Tesque INC.). To 1.4 µL of the resulting DNA solution, 0.2 µL of 10 × Ex-Taq buffer (Takara Bio Inc.), 0.2 µL of 2 mM dNTP (Takara Bio Inc.), and 0.2 µL of 10 × A-attachment mix (Toyobo Co., Ltd.) were added, and the mixture was reacted at 60°C for 1 hour. Thereafter, 1 µL of pMD19 simple (Takara Bio Inc.) was diluted 20 times with TE buffer, 3 µL of DNA Ligation Kit <Mighty Mix> was added thereto, a ligation reaction was performed at 16°C for 3 hours, and transformation was performed using Escherichia coli JM109 competent cells (Takara Bio Inc.). After overnight culture, the base sequences of the colonies that appeared on an LB plate containing carbenicillin were confirmed, and a clone having a correct sequence for each fragment was obtained.

### • Preparation of equimolar mixture of fragments for OGAB

Escherichia coli with these clones was grown in 2 ml of LB medium, and then, plasmids were purified with automated system QIAcube by QIAcube by QIAprep Spin Miniprep Kit (Qiagen N.V.) using 900 µL of the resultant and finally eluted into 30 µL of TE buffer. TE buffer was added so that a solution containing 2.5 µg of plasmid DNAs among them was 50 µL. Further, 6 µL of 10 X Plasmid safe buffer (epicentre), 2.4 µL of 25 mM ATP, and 2 µL of Plasmid-Safe ATP-Dependent DNase (epicentre) were added thereto, and the mixture was reacted at 37°C for 1 hour and then deactivated at 70°C for 30 minutes to decompose DNA having a structure other than a circular structure. Thereafter, the reaction solution was purified using MinElute PCR Purification Kit (Qiagen N.V.) and finally eluted from the column with 15 µL of TE buffer (Nacalai Tesque INC.). The resulting DNA solution was measured using a microspectrophotometer (Nano drop One, Thermo Fisher Scientific Inc.), and diluted by adding TE buffer so as to be 100 ng/µL. Thereafter, the concentration was measured again, and the DNA volume required to fractionate exactly 500 ng of DNA was calculated to two decimal places in µL, and fractionated with a micropipette to perform equimolar fractionation of each plasmid. The fractionated DNA solutions were pooled in two 1.5 ml centrifuge tubes depending on the type of restriction enzyme to be subsequently used. Plasmids cloning the 1st, 3rd, 5th, 7th, 9th, 11th, 13th, 14th, 17th, 19th, and 21st fragments were pooled into tubes for cleavage with the restriction enzyme AarI, and plasmids cloning the 2nd, 4th, 6th, 8th, 10th, 12th, 15th, 16th, 18th, 20th, and 22nd fragments were pooled into groups for cleavage with the restriction enzyme BsaI, respectively. Thereafter, 1.94 volumes of sterilized water, 0.33 volumes of 10 × Buffer AarI (Thermo Fisher Scientific Inc.), 0.06 volumes of 50 × oligonucleotide (0.025 mM), and 0.17 volumes of AarI (Thermo Fisher Scientific Inc.) were added to the tube for cleavage with AarI when the total volume of each plasmid was 1 volume, and the mixture was reacted at 37°C for 2 hours. In addition, to the tube for cleavage with BsaI, 2 volumes of sterile water, 0.33 volumes of 10 X CutSmart buffer (NEB), and 0.17 volumes of BsaI-HFv2 were added when the total volume of each plasmid solution was 1 volume, and the mixture was reacted at 37°C for 2 hours. An equal amount of TE saturated phenol to the restriction enzyme reaction solution was added to each tube, and the mixture was well mixed to deactivate the restriction enzyme. Then, the mixture of the phenol and the DNA solution in the emulsified state was integrated into one 2 ml centrifuge tube, and centrifuged at 20,000 × g for 10 minutes to separate the phenol phase and the aqueous phase. The upper layer was transferred to a new tube, into which an equal amount of 1-butanol was added, and the mixture was well stirred and centrifuged at 20,000 x g for 1 minute. Thereafter, the upper layer was removed by suction with a pipette, an equal amount of 1-butanol was added again, centrifugation was performed, and the upper layer was discarded until the volume of the lower layer became 450 µL or less. Thereafter, 50 µL of P3 buffer was added, 900 µL of ethanol was added, and the mixture was centrifuged at 20,000 × g for 10 minutes. The supernatant was discarded so as not to lose the precipitate and then rinsed with 900 µL of 70% ethanol, and the supernatant was sucked with a pipette and discarded. Thereafter, the mixture was centrifuged again, the remaining supernatant was collected at the bottom, and the liquid was completely removed with a pipette. Immediately, 50 µL of TE was added, and the precipitate was completely dissolved by tapping for 5 minutes. Thereafter, an equimolar mixture of 22 types of fragments of about 750 bp cut out from pMD19 by electrophoresis with a low melting point agarose gel was size-fractionated, and a mixture of 22 types of fragments was purified according to the method for cutting and purifying DNA fragments from an electrophoresis gel.

### • Construction of pGETS 103-AAV-Helper-RC2 by OGAB method

Thereafter, TE buffer was added to 1 fmol of the resulting DNA solution and 1 fmol of pGETS103ΔAarI-Linker purified by cleavage with AarI so that the total amount was 4 µL, and 5 µL of 2 × ligation buffer (15% polyethylene glycol 6000, 500 mM NaCl, 132 mM Tris·HCl (pH 7.6), 13.2 mM MgCl2, 20 mM DTT, 0.2 mM ATP) was added thereto. Then, the mixture was well mixed, 1 µL of T4 DNA Ligase (Takara Bio Inc.) was added, and the mixture was incubated at 37°C for 5 hours. 100 µL of competent cells of Bacillus subtilis were added thereto, and the mixture was subjected to rotary culture at 37°C for 30 minutes using a duck rotor. Thereafter, 300 µL of LB medium was added, and rotary culture was performed at 37°C for 1 hour with a duck rotor. Thereafter, the culture solution was spread on an LB plate containing 10 µg/mL of tetracycline (Sigma-Aldrich), and culture was performed overnight at 37°C. 94 colonies were obtained.

### • Confirmation of plasmid structure of transformant

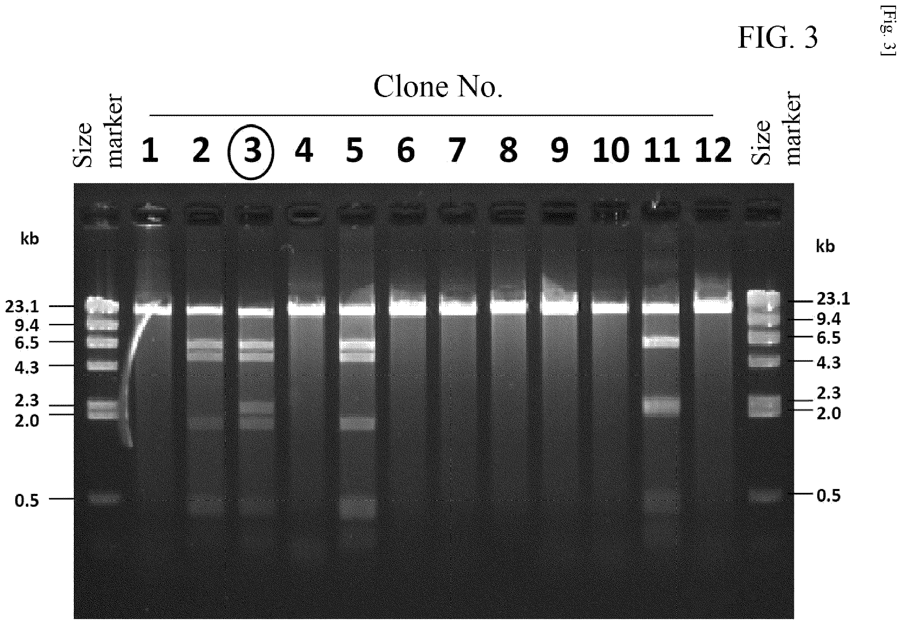

12 colonies were randomly selected and cultured overnight in 2 ml of LB medium containing 10 µg/ml of tetracycline, and in order to amplify the number of copies of the internal plasmids, IPTG was added so that a final concentration was 1 mM, and culture was further performed at 37°C for 3 hours. A small amount of plasmids was extracted from the resulting bacterial cells as follows. 900 µL of the bacterial solution was placed in a 1.5 ml centrifuge tube and centrifuged at 6,800 × g for 3 minutes, and the supernatant was removed with a micropipette. The resulting cell pellets were well suspended, 100 µL of P1 buffer containing 10 mg/ml of egg white lysozyme (wako) was added, and then the cell pellets were incubated at 37°C for 5 minutes. Then, 200 µL of P2 buffer was added and mixed by inversion 4 times. Thereafter, 150 µL of P3 was added, and mixing was performed by inversion 4 times. The resultant was centrifuged at 20,000 × g for 10 minutes to separate into a white precipitate and a supernatant. The supernatant was transferred to a new 1.5 ml centrifuge tube, into which 450 µL of TE saturated phenol (Nacalai Tesque INC.) was added, and mixed well. Then, the mixture was centrifuged at 20,000 x g for 10 minutes to separate into a phenol phase and an aqueous phase, 320 µL of the aqueous phase was transferred to a new tube, into which 900 µL of ethanol was added, and the mixture was centrifuged at 20,000 x g for 10 minutes. The supernatant was removed with a micropipette, 900 µL of 70% ethanol was added, and the entire tube was rinsed. Thereafter, 70% ethanol was removed with a micropipette, and the resulting precipitate was dissolved in 27 µL of TE buffer. 8 µL of the resulting plasmid solution was taken, 1 µL of 10 × 3.1 NEB buffer (NEB) and 1 µL of SmaI were added thereto, the mixture was reacted at 37°C for 1 hour, and then, cleaved patterns were confirmed by agarose gel electrophoresis. As illustrated in FIG. 3, one out of 12 (clone number 3) showed the expected cleavage pattern. The entire nucleotide sequence of this clone was determined, and it was confirmed that pGETS103-AAV-Helper-RC2 was constructed.

### (Example 2: Construction of various AAV All-in-One vector plasmids)

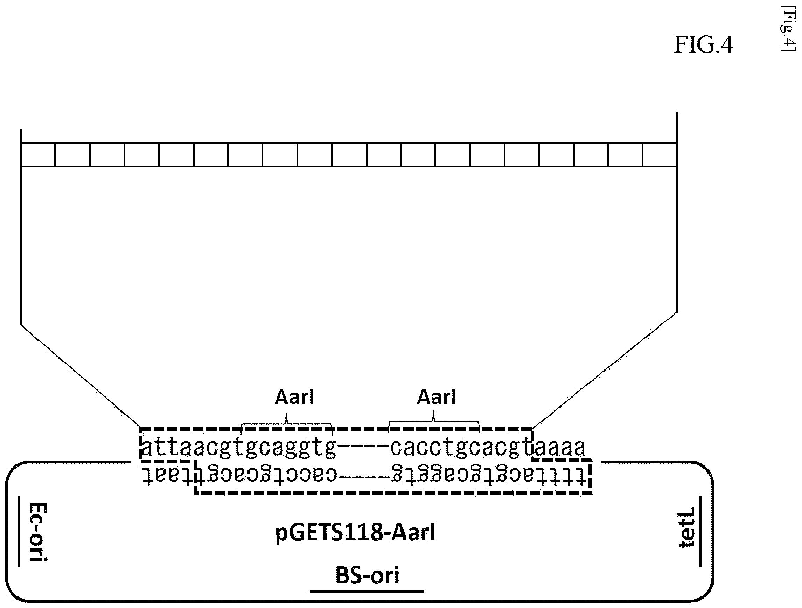

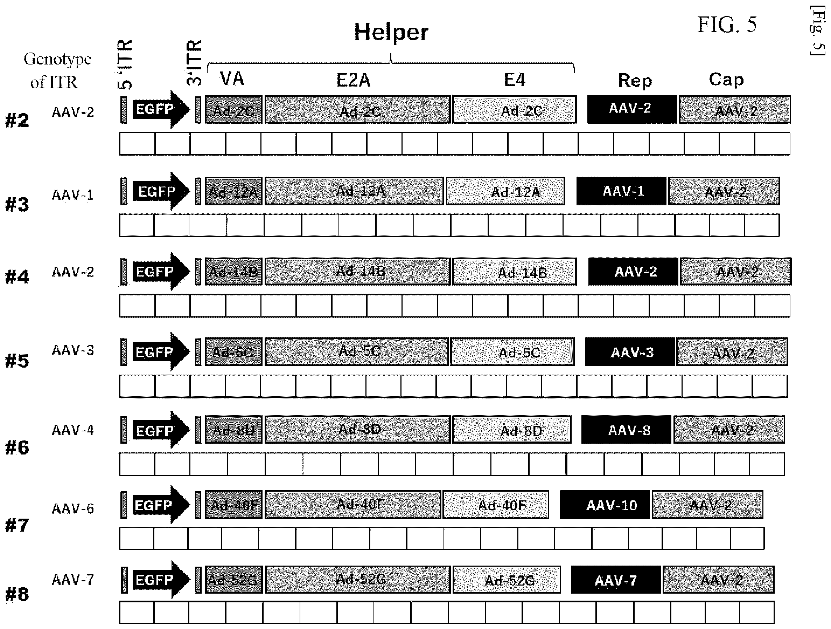

Similar to Example 1, seven types of vector plasmids of All-in-One structure for producing AAV vectors were constructed using components obtained from the AAV and adenoviral genomes of other serotypes. Each plasmid was constructed based on pGETS118-AarI (Tsuge, K., Sato, Y., Kobayashi, Y, Gondo, M., Hasebe, M., Togashi, T., Tomita, M., and Itaya, M. Method of preparing an equimolar DNA mixture for one-step DNA assembly of over 50 fragments. Sci Rep 5, 10655 (2015)) (the schematic view is illustrated in FIG. 4). The serotypes of the viral genomes from which the ITRs, Rep, Cap and Helper (VA, E2A and E4) used in each of the vector plasmids were derived are shown in the table below (see also FIG. 5).

**[Table 1]**

| Table 1: Construction of each plasmid | | | | | |
|---|---|---|---|---|---|
| Vector plasmid No. | 5' ITR | 3' **ITR** | Helper | Rep | Cap |
| 1* | Improved AAV-2 | Improved AAV-2 | Ad-2C | Improved AAV- 2 | AAV-2 |
| 2 | Improved AAV-2 | Improved AAV-2 | Ad-2C | Improved AAV-2 | AAV-2 |
| 3 | AAV-1 | AAV-1 | Ad-12A | AAV-1 | AAV-2 |
| 4 | AAV-2 | AAV-2 | Ad-14B | AAV--2 | AAV-2 |
| 5 | AAV-3 | AAV-3 | Ad-5C | AAV-3 | AAV-2 |
| 6 | AAV-4 | AAV-4 | Ad-8D | AAV-8 | AAV-2 |
| 7 | AAV-6 | AAV-6 | Ad-40F | AAV-10 | AAV-2 |
| 8 | AAV-7 | AAV-7 | Ad-52G | AAV-7 | AAV-2 |

| | | | | | |
|---|---|---|---|---|---|
| * The vector plasmid of 1 is that of Example 1 (pGETS103-AAV-Helper-RC2). AAV: Adeno-associated virus serotype, Ad: adenovirus serotype and group | | | | | |

Each of the above All-in-One nucleic acids (total length of 15 to 16 kb) was designed, and DNA fragments containing 18 or 19 unit DNA fragments (700 to 900 bp) were chemically synthesized according to their length. These DNA fragments were cut out by any one of restriction enzymes AarI, BbsI, BsaI, and BsmBI to prepare unit DNA fragments. These unit DNA fragments were ligated to AarI-cleaved pGETS118 and tandem repeats, and then circular plasmids were formed in Bacillus subtilis by the OGAB method as in Example 1. As a result, the construction of a vector plasmid having an All-in-One structure of the seven types of additional AAV vectors was confirmed.

### (Example 3: Evaluation of production of viral vector in adhesion culture (flask culture))

### • Mass culture of plasmid DNA

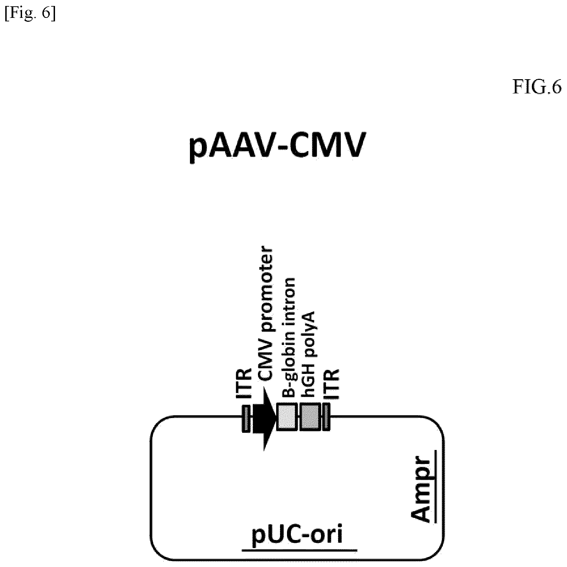

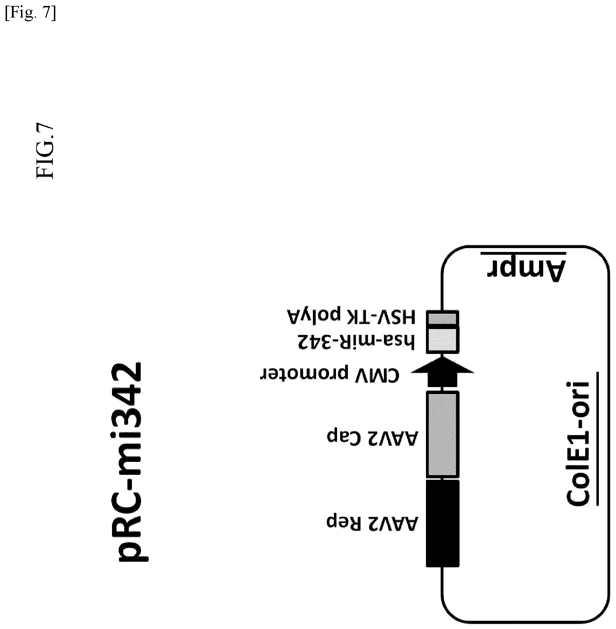

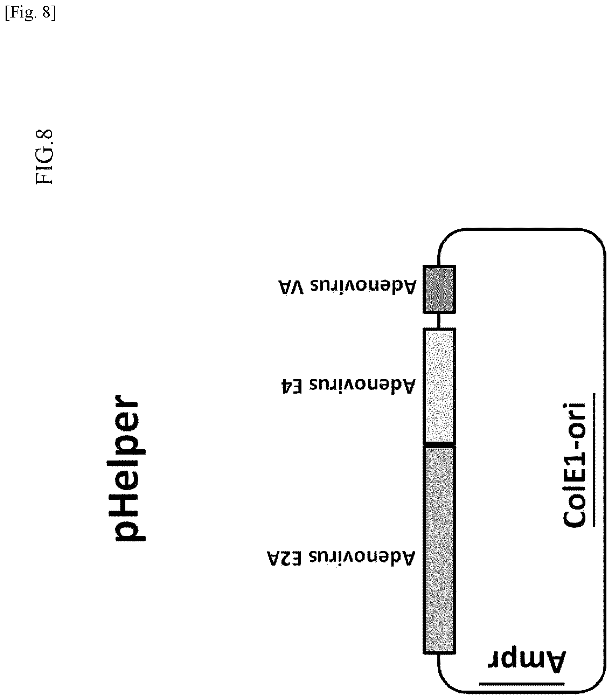

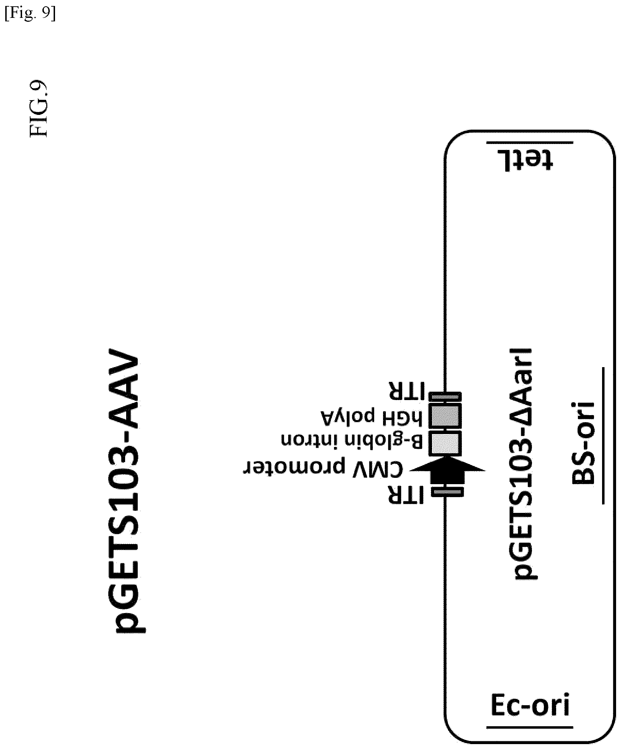

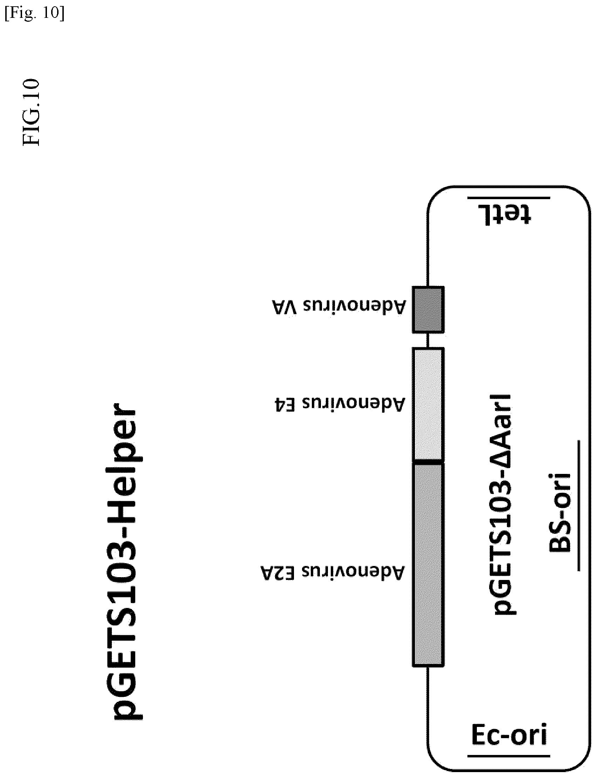

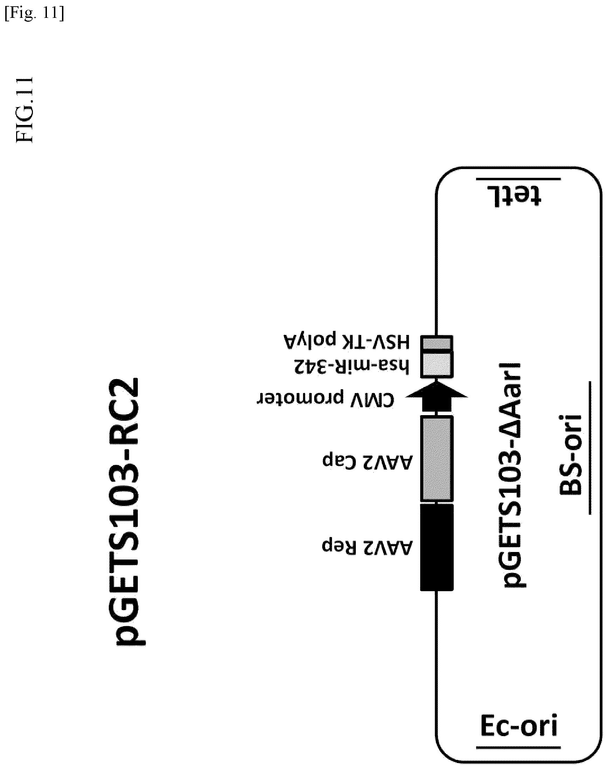

The constructed pGETS103-AAV-Helper-RC2 was transformed into Escherichia coli JM109 competent cell (purchased from Takara Bio Inc.), Bacillus subtilis strain BUSY9797 (transferred from Kobe University, Tsuge, K., Sato, Y., Kobayashi, Y, Gondo, M., Hasebe, M., Togashi, T., Tomita, M., Itaya, M. (2015) Method of preparing an equimolar DNA mixture for one-step DNA assembly of over 50 fragments, Scientific Reports, 5, 10655), and mass-cultured in each of Bacillus subtilis and Escherichia coli. 200 mL of a mixture obtained by adding an antibiotic (tetracycline) to LB medium (10 g of Bactotryptone, 5 g of Yeast extract, and 5 g of sodium chloride were dissolved in 1 L of water) was prepared, 100 ml of the mixture was placed in a 500 mL Erlenmeyer flask to inoculate Escherichia coli or Bacillus subtilis plasmid-retaining strain, and culture was performed overnight at 37°C (in the case of culturing a larger amount, the flask culture was repeated). Centrifugation was performed to collect bacteria from the culture solution, and after lysis, the bacteria were purified by cesium chloride density gradient ultracentrifugation (cesium chloride density: about 0.84 g/mL) to recover supercoil-shaped plasmid DNA. The concentration of the recovered plasmid DNA was measured using an ultraviolet-visible spectrophotometer. pAAV-CMV (FIG. 6), pHelper (FIG. 7), and pRC2-mi342 (FIG. 8) were purchased from Takara Bio Inc. and cultured in a large amount in Escherichia coli in the same manner. In addition, pGETS103-AAV (FIG. 9), pGETS103-Helper (FIG. 10), and pGETS103-RC2 (FIG. 11) were plasmid DNAs obtained by subjecting pAAV-CMV, pHelper, and pRC2-mi342pGETS103 to a restriction enzyme treatment, and cloning each gene required for production of a viral vector into pGETS-103. In the same manner, a large amount of plasmid DNAs were cultured in Escherichia coli and Bacillus subtilis.

**[Table 2]**

| Table 2: Summary of plasmid DNA used | | | | |
|---|---|---|---|---|
| **rAAV #** | Transfected pDNA | Number of transfected pDNAs | Length [bp] | Host using mass culture |
| 1 | pGETS103-AAV-Helper-RC2 | 1 | 31884 | Bacillus subtilis |
| 2 | ·pGETS103-AAV | 3 | 17722 | Bacillus subtilis |
| | ·pGETS103-Helper | | 24812 | |
| | ·pGETS103-RC2 | | 20982 | |
| 3 | pGETS103-AAV-Helper-RC2 | 1 | 31884 | Escherichia coli |
| 4 | ·pGETS103-AAV | 3 | 17722 | Escherichia coli |
| | ·pGETS103-Helper | | 24812 | |
| | ·pGETS103-RC2 | | 20982 | |
| 5 | ·pAAV-CMV | 3 | 5031 | Escherichia coli |
| | ·pHelper | | 11635 | |
| | ·pRC2-mi342 | | 8189 | |

### • Production of rAAV

1.5 × 10⁷ 293T cells were seeded in a 15 cm-dish the day before transfection. After 22 hours, the medium was replaced with 19 mL of DMEM medium (2% FBS, 1% penicillin/streptomycin), and culture was performed at 37°C and 5% CO₂ for 2 hours. To 293T cells in a confluent state (the number of cells: 3 × 10⁷), a transfection solution in which 500 µL of DMEM medium containing 2.7 pmol of the plasmids and 500 µL of DMEM medium containing PEIpro (registered trademark) in an amount of 1-fold of DNA were mixed was added.

After culturing at 37°C and 5% CO₂ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 1.2 mL of Lysis buffer (2 mM MgCl₂, 150 mM NaCl, 50 mM Tris, 0.1% Triton (registered trademark) X-100; pH 8.5), benzonase (registered trademark) (50 units/mL) was further added thereto, and the mixture was reacted at 37°C for 30 minutes. Thereafter, 2/100 volume of a 1.9 M MgSO₄ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

Density gradient centrifugation in which three types of cesium chloride solutions (1.50 g/cm³, 1.30 g/cm³, and 1.25 g/cm³) were stacked was performed at 226,000 × g and 18°C for 1.5 hours, and about 3.5 mL of rAAV was recovered from about 1.30 g/cm³ of the solution.

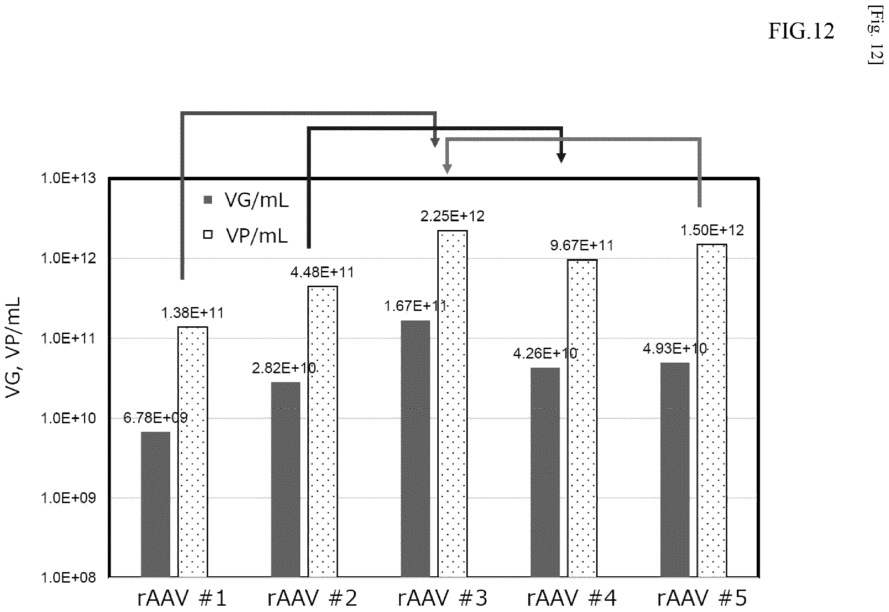

### • Evaluation of rAAV (FIG. 12)

In order to quantify the genome titer of rAAV, DNaseI treatment was performed for 30 minutes, and a sample reacted at 95°C for 10 minutes was diluted 1,000 times and subjected to qPCR. As primer sequences, 5'-GGAACCCCTAGTGATGGAGTT-3' (SEQ ID NO: 1) and 5'-CGGCCTCAGTGAGCGA-3' (SEQ ID NO: 2) were used. As the PCR conditions, after 10 minutes at 95°C, 40 cycles of 95°C/15 seconds, 55°C/5 seconds, and 72°C/30 seconds were repeated.

The particle titer was quantified by ELISA method. For the quantification kit, AAV2 Titration ELISA 2.0R was used, and the test was performed based on the protocol. To a 96-well plate on which the anti-AAV2 antibody contained in the kit was immobilized, 100 µL each of rAAV and a standard (empty capsid reagent contained in the kit) was added. After standing at 37°C for 1 hour, the liquid was discarded, and washing was performed 3 times with 200 µL of an assay buffer. 100 µL of a biotin-conjugated anti-AAV2 antibody was added, the mixture was allowed to stand at 37°C for 1 hour, the liquid was discarded, and then, washing was performed 3 times with 200 µL of an assay buffer. Further, 100 µL of HRP-labeled streptavidin was added, the mixture was allowed to stand at 37°C for 1 hour, the liquid was discarded, and washing was performed 3 times with 200 µL of an assay buffer. Finally, 100 µL of TMB was added, the mixture was allowed to stand at room temperature for 15 minutes, a reaction stopping reagent was added thereto, and absorbance (450 nm, 650 nm) was measured with a microplate reader. The VP/VG ratio was calculated by dividing the particle titer by the genome titer. The VG/cell ratio was calculated by dividing the genome titer by the number of cells in the confluent state.

**[Table 3]**

| Table 3: Results of viral vector production | | | | | |
|---|---|---|---|---|---|
| **rAAV #** | Genome titer [VG/mL] | Total amount of genome | Particle titer [VP/mL] | VP/VG ratio | VG/cell ratio |
| | | [VG] | | | |
| 1 | 6.78E+09 | 2.37E+10 | 1.38E+11 | 20 | 7.90E+02 |
| 2 | 2.82E+10 | 9.87E+10 | 4.48E+11 | 16 | 3.29E+ 03 |
| 3 | 1.6 7E+11 | 5.85E+11 | 2.25E+12 | 13 | 1.95E+04 |
| 4 | 4.26E+10 | 1.49E+11 | 9.67E+11 | 23 | 4.97E+403 |
| 5 | 4.93E+10 | 1.72E+11 | 1.50E+12 | 30 | 5.75E+03 |

### (Example 4: Evaluation of production of viral vector in adhesion culture (jar culture))

### • Mass culture of plasmid DNA

pGETS103-AAV-Helper-RC2 was transformed into Escherichia coli JM109 competent cell and Bacillus subtilis BUSY9797 strain, and each cells were cultured in a large amount in a jar fermenter (Biot, BMS-P). As a pre-culture, 50 mL of LB medium was added to a 500 mL flask, and after inoculation, batch culture was performed. A portion of the liquid cultured overnight was collected and inoculated into 4 L of an initial medium in a 10 L jar fermenter to start the main culture (batch culture). The culture was continued while the dissolved oxygen (Escherichia coli: 3.6 to 7.3 ppm, Bacillus subtilis: 5.4 to 6.9 ppm) and the pH (6.95 to 7.05) were kept constant, and after it was confirmed that the turbidity (OD600 value) was a certain value (Escherichia coli: 21, Bacillus subtilis: 38) or more, the culture was terminated. Centrifugation was performed to collect bacteria from the culture solution, and after lysis, the bacteria were purified by cesium chloride density gradient ultracentrifugation (cesium chloride density: about 0.84 g/mL) to recover supercoil-shaped plasmid DNA. The concentration of the recovered plasmid DNA was measured using an ultraviolet-visible spectrophotometer.

**[Table 4]**

| Table 4: Construction of plasmid DNA used | | | | |
|---|---|---|---|---|
| **rAAV #** | Transfected pDNA | Number of transfected pDNAs | Length [bp] | Host using mass culture |
| 6 | pGETS103-AAV-Helper-RC2 | 1 | 31884 | Escherichia coli |
| 7 | pGETS103-AAV-Helper-RC2 | 1 | 31884 | Bacillus subtilis |

### • Production of rAAV

3 × 10⁶ 293T cells were seeded in a 10 cm-dish the day before transfection. After 22 hours, the medium was replaced with 9.5 mL of DMEM medium (2% FBS, 1% penicillin/streptomycin), and culture was performed at 37°C and 5% CO₂ for 2 hours. To 293T cells in a confluent state (the number of cells: 6 × 10⁶), a transfection solution in which 250 µL of DMEM medium containing 11.53 µg of the plasmids and 250 µL of DMEM medium containing PEIpro (registered trademark) (PolyPlus-transfection SAS) in an amount of 1-fold of DNA were mixed was added.

After culturing at 37°C and 5% CO₂ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 400 µL of Lysis buffer (2 mM MgCl₂, 150 mM NaCl, 50 mM Tris, 0.1% Triton (registered trademark) X-100 (Nacalai Tesque INC.); pH 8.5), benzonase (registered trademark) (50 units/mL, Novagen) was further added thereto, and the mixture was reacted at 37°C for 30 minutes. Thereafter, 2/100 volume of a 1.9 M MgSO₄ solution was added, and the mixture was allowed to stand at room temperature for 10 minutes. The supernatant was recovered by centrifugation at 12,000 x g and 4°C for 10 minutes, and Pluronic (registered trademark) F-68 (Thermo Fisher Scientific Inc.) was added at a final concentration of 0.001%.

Density gradient centrifugation in which three types of cesium chloride solutions (1.50 g/cm³, 1.30 g/cm³, and 1.25 g/cm³) were stacked was performed at 226,000 × g and 18°C for 1.5 hours, and about 3.5 mL of rAAV was recovered from about 1.30 g/cm³ of the solution.

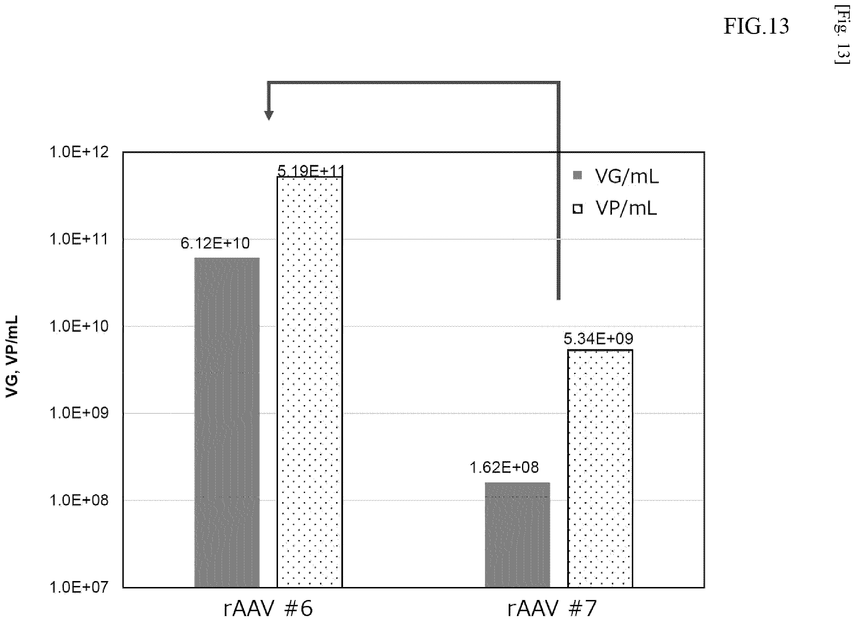

### • Evaluation of rAAV (FIG. 13)

In order to quantify the genome titer of rAAV, DNaseI treatment was performed for 30 minutes, and a sample reacted at 95°C for 10 minutes was diluted 1,000 times and subjected to qPCR. As primer sequences, 5'-GGAACCCCTAGTGATGGAGTT-3' (SEQ ID NO: 1) and 5'-CGGCCTCAGTGAGCGA-3' (SEQ ID NO: 2) were used. As the PCR conditions, after 10 minutes at 95°C, 40 cycles of 95°C/15 seconds, 55°C/5 seconds, and 72°C/30 seconds were repeated.

The particle titer was quantified by ELISA method. For the quantification kit, AAV2 Titration ELISA 2.0R (PROGEN) was used, and the test was performed based on the protocol. To a 96-well plate on which the anti-AAV2 antibody contained in the kit was immobilized, 100 µL each of rAAV and a standard (empty capsid reagent contained in the kit) was added. After standing at 37°C for 1 hour, the liquid was discarded, and washing was performed 3 times with 200 µL of an assay buffer. 100 µL of a biotin-conjugated anti-AAV2 antibody was added, the mixture was allowed to stand at 37°C for 1 hour, the liquid was discarded, and then, washing was performed 3 times with 200 µL of an assay buffer. Further, 100 µL of HRP-labeled streptavidin was added, the mixture was allowed to stand at 37°C for 1 hour, the liquid was discarded, and washing was performed 3 times with 200 µL of an assay buffer. Finally, 100 µL of TMB was added, the mixture was allowed to stand at room temperature for 15 minutes, a reaction stopping reagent was added thereto, and absorbance (450 nm, 650 nm) was measured with a microplate reader.

The VP/VG ratio was calculated by dividing the particle titer by the genome titer. The VG/cell ratio was calculated by dividing the genome titer by the number of cells in the confluent state.

**[Table 5]**

| Table 5: Results of viral vector production | | | | | |
|---|---|---|---|---|---|
| **rAAV #** | Genome titer [VG/mL] | Total amount of genome | Particle titer [VP/mL] | VP/VG ratio | VG/cell ratio |
| | | [VG] | | | |
| 6 | 6.12E+10 | 1.84E+11 | 5.19E+11 | 8 | 3.06E+04 |
| 7 | 1.62E+08 | 4.85E+08 | 5.34E+09 | 33 | 8.08E+01 |

### (Example 5: Viral vector production using different plasmid vector plasmids)

### • Mass culture of plasmid DNA

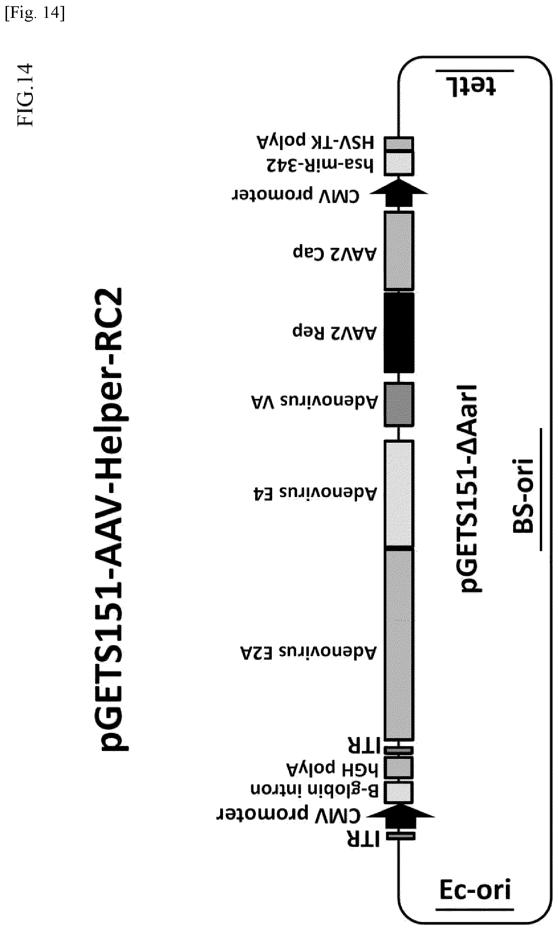

In the base sequence of pGETS103-AAV-Helper-R2, a region of AAV-Helper-RC2 (FIG. 2) excluding the base sequence of pGETS103 of the vector portion was cloned into pGETS151 to construct pGETS151-AAV-Helper-RC2 (FIG. 14). pGETS151-AAV-Helper-RC2 was transformed into Escherichia coli JM109 competent cell and Bacillus subtilis MI112 strain, and each cells were cultured in a large amount in a jar fermenter (Biot, BMS-P). As pre-culture, 50 mL of LB medium (a medium in which the concentration of Tryptone and YE was doubled was used for Bacillus subtilis) was added to a 500 mL flask, and after inoculation, batch culture was performed. A portion of the liquid cultured overnight was collected and inoculated into 4 L of an initial medium in a 10 L jar fermenter to start the main culture (fed-batch culture). The culture was continued while the dissolved oxygen (4.3 to 7.3 ppm) and the pH (6.95 to 7.1) were kept constant, and after it was confirmed that the turbidity (OD600 value) was 50 or more, the culture was terminated. Centrifugation was performed to collect bacteria from the culture solution, and after lysis, the bacteria were purified by cesium chloride density gradient ultracentrifugation (cesium chloride density: about 0.84 g/mL) to recover supercoil-shaped plasmid DNA. The concentration of the recovered plasmid DNA was measured using an ultraviolet-visible spectrophotometer.

**[Table 6]**

| Table 6: Plasmid DNA used in Example 5 | | | | |
|---|---|---|---|---|
| **rAAV #** | Transfected pDNA | Number of transfected pDNAs | Length [bp] | Host using mass culture |
| 8 | pGETS151-AAV-Helper-RC2 | 1 | 22749 | Escherichia coli |
| 9 | pGETS151-AAV-Helper-RC2 | 1 | 22749 | Bacillus subtilis |

### • Production of rAAV

1.5 × 10⁷ 293T cells were seeded in a 15 cm-dish the day before transfection. After 22 hours, the medium was replaced with 19 mL of DMEM medium (2% FBS, 1% penicillin/streptomycin), and culture was performed at 37°C and 5% CO₂ for 2 hours. To 293T cells in a confluent state (the number of cells: 3 × 10⁷), a transfection solution in which 500 µL of DMEM medium containing 2.1 pmol of the plasmids and 500 µL of DMEM medium containing PEIpro (registered trademark) in an amount of 1-fold of DNA were mixed was added.

After culturing at 37°C and 5% CO₂ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 1.2 mL of Lysis buffer (2 mM MgCl₂, 150 mM NaCl, 50 mM Tris, 0.1% Triton (registered trademark) X-100; pH 8.5), benzonase (registered trademark) (50 units/mL) was further added thereto, and the mixture was reacted at 37°C for 30 minutes. Thereafter, 2/100 volume of a 1.9 M MgSO₄ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

Density gradient centrifugation in which three types of cesium chloride solutions (1.45 g/cm³, 1.27 g/cm³, and 1.25 g/cm³) were stacked was performed at 226,000 × g and 18°C for 1.5 hours, and about 2 mL of rAAV was recovered from about 1.27 g/cm³ of the solution.

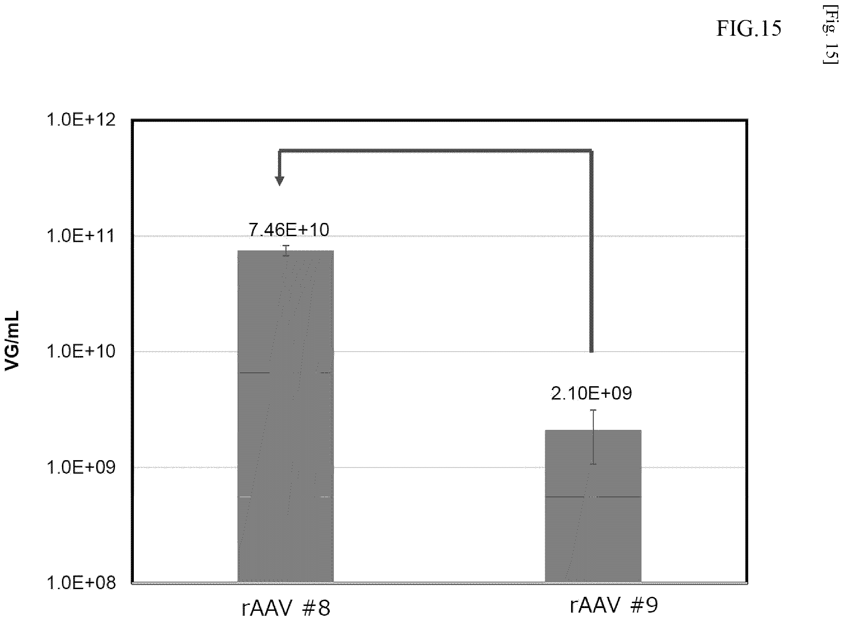

### • Evaluation of rAAV (FIG. 15)

In order to quantify the genome titer of rAAV, DNaseI treatment was performed for 30 minutes, and a sample reacted at 95°C for 10 minutes was diluted 1,000 times and subjected to qPCR. As primer sequences, 5'-GGAACCCCTAGTGATGGAGTT-3' (SEQ ID NO: 1) and 5'-CGGCCTCAGTGAGCGA-3' (SEQ ID NO: 2) were used. As the PCR conditions, after 10 minutes at 95°C, 40 cycles of 95°C/15 seconds, 55°C/5 seconds, and 72°C/30 seconds were repeated. The VG/cell ratio was calculated by dividing the genome titer by the number of cells in the confluent state.

**[Table 7]**

| Table 7: Results of viral vector production | | | |
|---|---|---|---|
| **rAAV #** | Genome titer [VG/mL] | Total amount of genome | VG/cell ratio |
| | | [VG] | |
| 8 | 7.46E+10 | 1.49E+11 | 4.97E+03 |
| 9 | 2.10E+09 | 4.20E+09 | 1.40E+02 |

### (Example 6: Evaluation of production of viral vector in suspension culture)

### • Mass culture of plasmid DNA

pGETS151-AAV-Helper-RC2 was transformed into Escherichia coli JM109 competent cell and Bacillus subtilis MI112 strain, and each cells were cultured in a large amount in a jar fermenter (Biot, BMS-P). As pre-culture, 50 mL of LB medium (a medium in which the concentration of Tryptone and YE was doubled was used for Bacillus subtilis) was added to a 500 mL flask, and after inoculation, batch culture was performed. A portion of the liquid cultured overnight was collected and inoculated into 4 L of an initial medium in a 10 L jar fermenter to start the main culture (fed-batch culture). The culture was continued while the dissolved oxygen (4.3 to 7.3 ppm) and the pH (6.95 to 7.1) were kept constant, and after it was confirmed that the turbidity (OD600 value) was 50 or more, the culture was terminated. Centrifugation was performed to collect bacteria from the culture solution, and after lysis, the bacteria were purified by cesium chloride density gradient ultracentrifugation (cesium chloride density: about 0.84 g/mL) to recover supercoil-shaped plasmid DNA. The concentration of the recovered plasmid DNA was measured using an ultraviolet-visible spectrophotometer.

**[Table 8]**

| Table 8: Construction of plasmid DNA used | | | | |
|---|---|---|---|---|
| **rAAV #** | Transfected pDNA | Number of transfected pDNAs | Length [bp] | Host using mass culture |
| 10 | pGETS151-AAV-Helper-RC2 | 1 | 22.749 | Escherichia coli |
| 11 | pGETS151-AAV-Helper-RC2 | 1 | 22.749 | Bacillus subtilis |

### • Production of rAAV

On the day of transfection, 9.0 × 10⁷ cells of VPC2.0 (registered trademark) cells (floating cells derived from HEK293) were seeded in a 125 mL Erlenmeyer flask. After 1 hour, 3 mL of AAV-MAX Enhancer was added, and culture was performed at 37°C and 8% CO₂ for 2 hours. To VPC2.0 (registered trademark) cells (number of cells: 9.0 × 10⁷), a transfection solution obtained by mixing 3 mL of Viral-Plex^{™} Complexation Buffer containing 3.0 pmol of the plasmids, 90 µL of AAV-MAX Transfection Booster, and 180 µL of AAV-MAX Transfection Reagent was added.

After culturing at 37°C and 8% CO₂ for 72 hours, the cell suspension was recovered and centrifuged at 180 × g for 5 minutes to completely remove the supernatant. The cell pellets were suspended in 3.6 mL of Lysis buffer (2 mM MgCl₂, 150 mM NaCl, 50 mM Tris, 0.1% Triton (registered trademark) X-100; pH 8.5), benzonase (registered trademark) (167 units/mL) was further added thereto, and the mixture was reacted at 37°C for 30 minutes. Thereafter, 2/100 volume of a 1.9 M MgSO₄ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

Density gradient centrifugation in which three types of cesium chloride solutions (1.35 g/cm³, 1.27 g/cm³, and 1.25 g/cm³) were stacked was performed at 226,000 × g and 18°C for 1.5 hours, and about 2.0 mL of rAAV was recovered from about 1.27 g/cm³ of the solution.

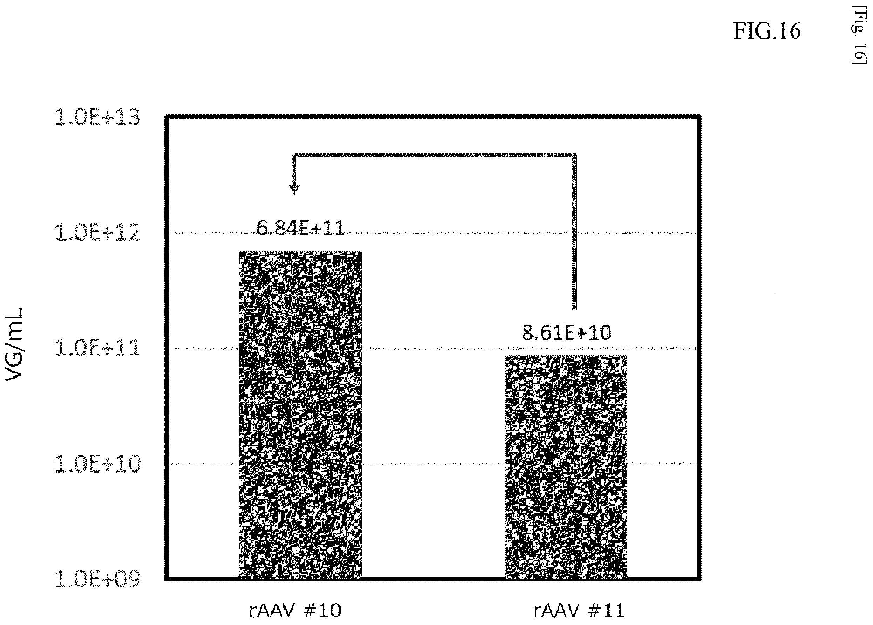

### • Evaluation of rAAV (FIG. 16)

In order to quantify the genome titer of rAAV, DNaseI treatment was performed for 30 minutes, and a sample reacted at 95°C for 10 minutes was diluted 1,000 times and subjected to qPCR. As primer sequences, 5'-GGAACCCCTAGTGATGGAGTT-3' (SEQ ID NO: 1) and 5'-CGGCCTCAGTGAGCGA-3' (SEQ ID NO: 2) were used. As the PCR conditions, after 10 minutes at 95°C, 40 cycles of 95°C/15 seconds, 55°C/5 seconds, and 72°C/30 seconds were repeated. The VG/cell ratio was calculated by dividing the genome titer by the number of cells in the confluent state.

**[Table 9]**

| Table 9: Results of viral vector production | | | |
|---|---|---|---|
| **rAAV #** | Genome titer [VG/mL] | Total amount of genome | VG/cell ratio |
| | | [VG] | |
| 10 | 6.84E+11 | 1.37E+12 | 9.87E+3 |
| 11 | 8.61E+10 | 1.72E+11 | 1.05E+3 |

### (Example 7: Construction of adenovirus All-in-One vector plasmid)

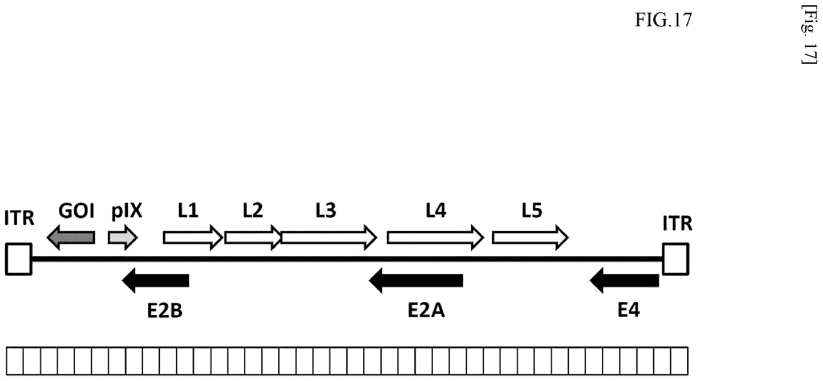

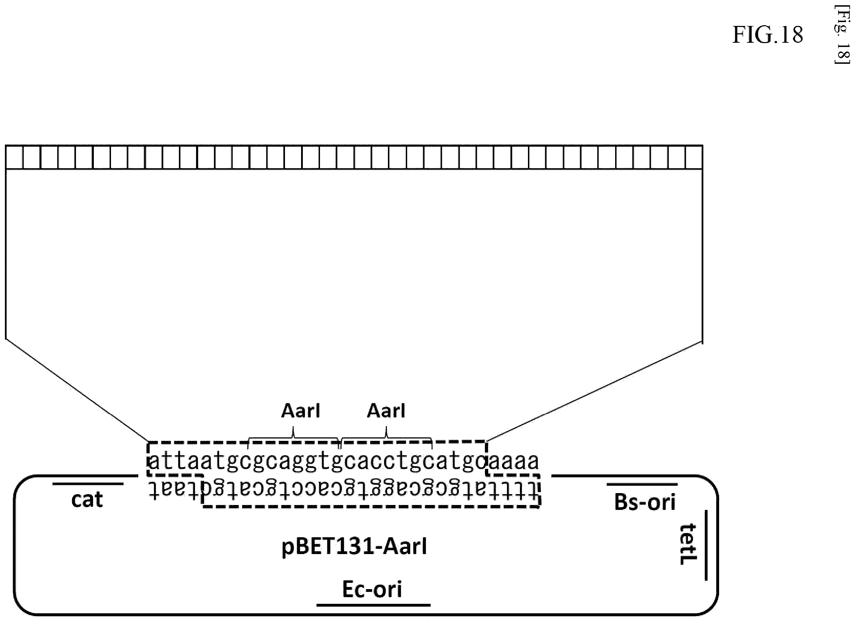

For the adenoviral vector Ad5, an All-in-One structure for producing an adenoviral vector having a total length of about 36 kb in which a GIO gene was introduced into pAxCAwtit2 manufactured by Takara Bio Inc. was designed (FIG. 17). The structure was divided into 40 pieces of about 800 bp, and DNA fragments containing a unit DNA fragment were prepared by PCR or chemical synthesis. These DNA fragments were cut out by any one of restriction enzymes AarI, BbsI, BsaI, and BsmBI to prepare unit DNA fragments. Thereafter, a linker DNA was introduced into BamHI site of pGETS118-AarI or pBET131 (Tanaka, T., and M. Ogura. 1998. A novel Bacillus natto plasmid pLS32 capable of replication in Bacillus subtilis. FEBS Lett. 422: 243-246) cleaved with AarI, pBET131-AarI (FIG. 18) into which two new AarI sites were introduced was cleaved with AarI to obtain two large fragments and tandem repeats, and then a circular plasmid was formed in Bacillus subtilis by the OGAB method in the same manner as in the above Examples. As a result, the construction of a clone having an All-in-One structure for producing an adenoviral vector was confirmed.

### (Example 8: Another structural example of AAV All-in-One vector plasmid)

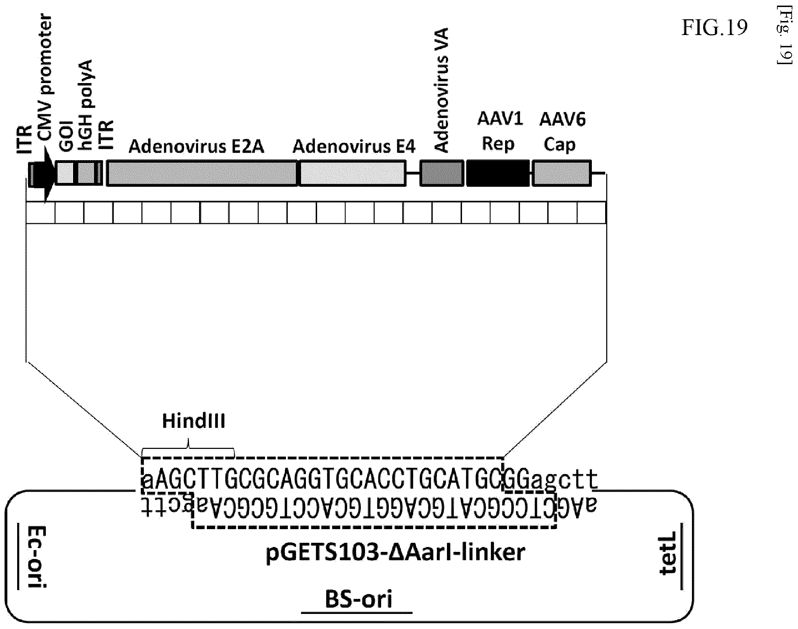

AAV All-in-One plasmids illustrated in FIGS. 19 to 24 are also considered.
- FIG. 19 is a construction example using Rep for AAV1 and Cap for AAV6 based on pGETS103-ΔAarI.

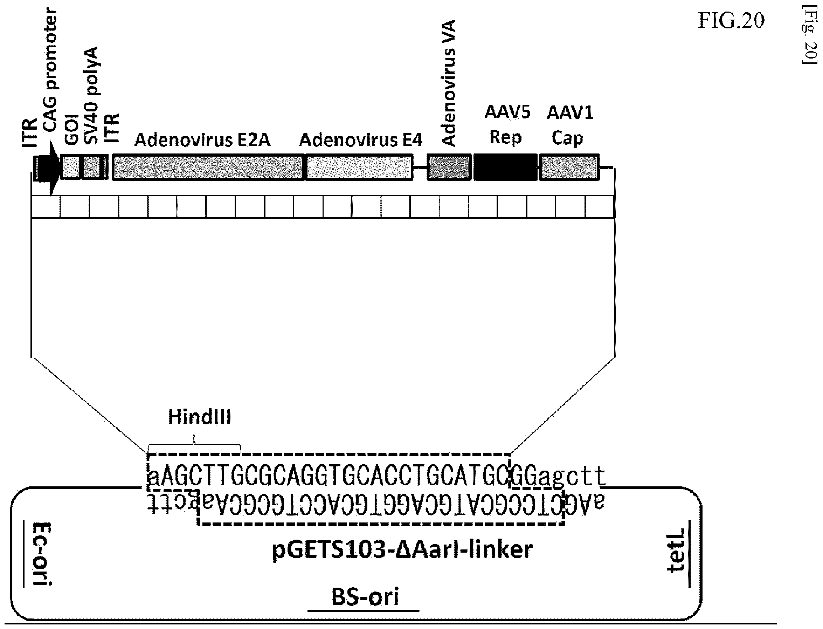

- FIG. 20 is a construction example using CAG promoter, Rep for AAV5, and Cap for AAV1 based on pGETS103-ΔAarI.

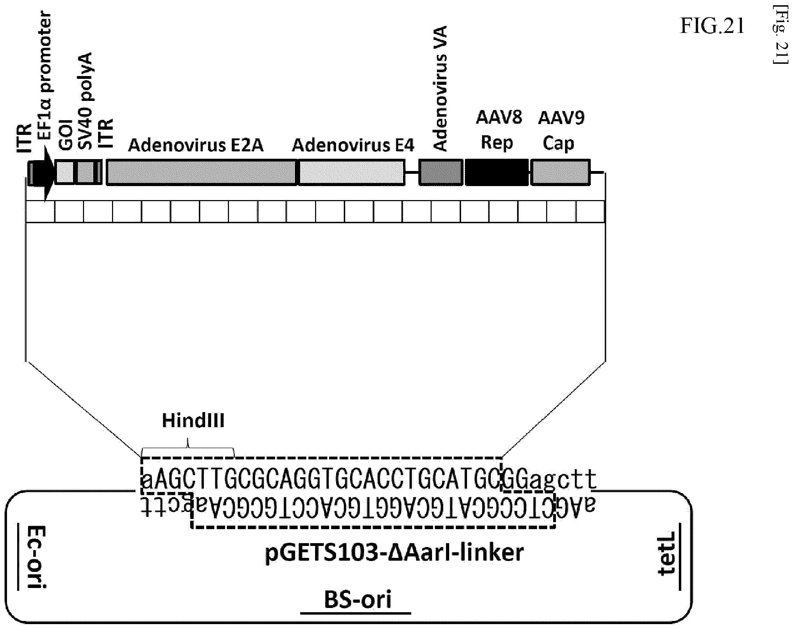

- FIG. 21 is a construction example using EF1α promoter, Rep for AAV8, and Cap for AAV9 based on pGETS103-ΔAarI.

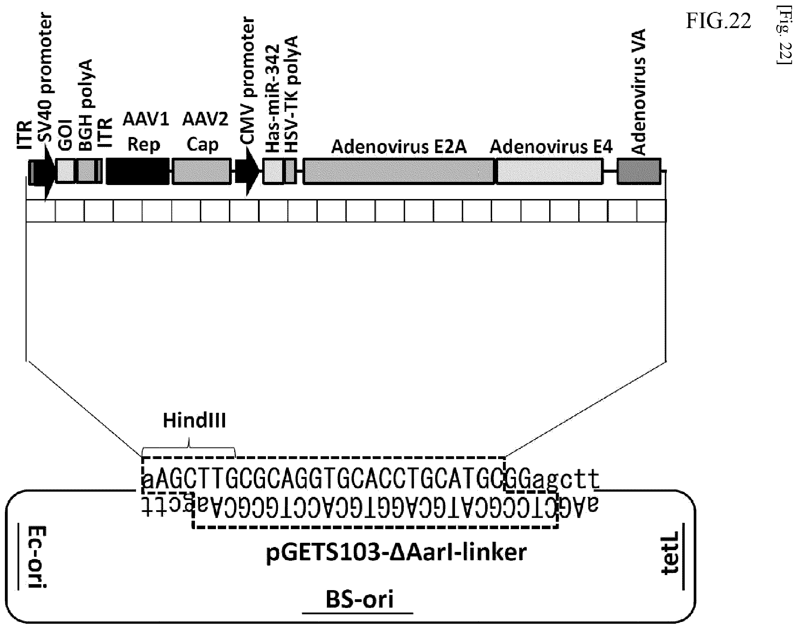

- FIG. 22 is a construction example in which SV40 promoter based on pGETS103-ΔAarI is used, and Rep, Cap, and Helper are arranged in different orders.

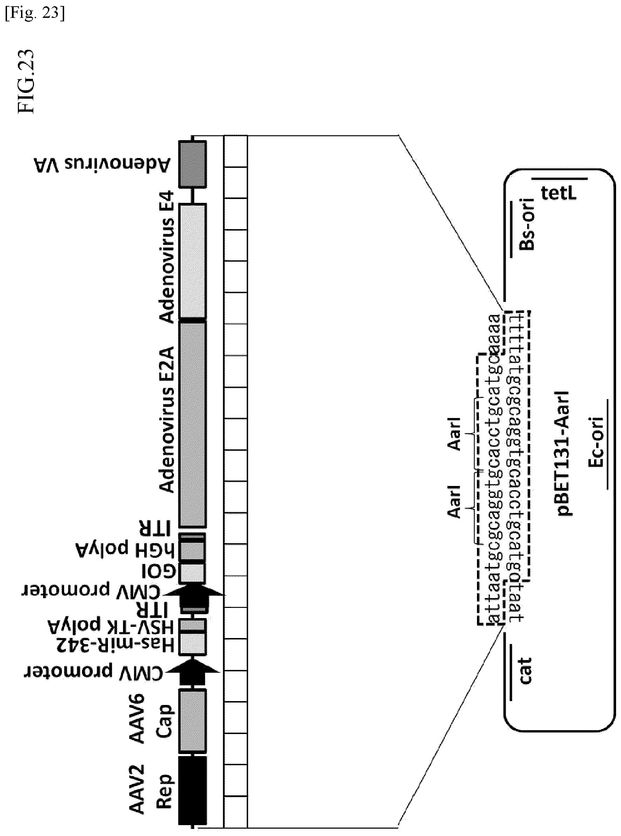

- FIG. 23 illustrates a construction example in which Rep, Cap, and Helper based on pGETS131-AarI are arranged in different orders.

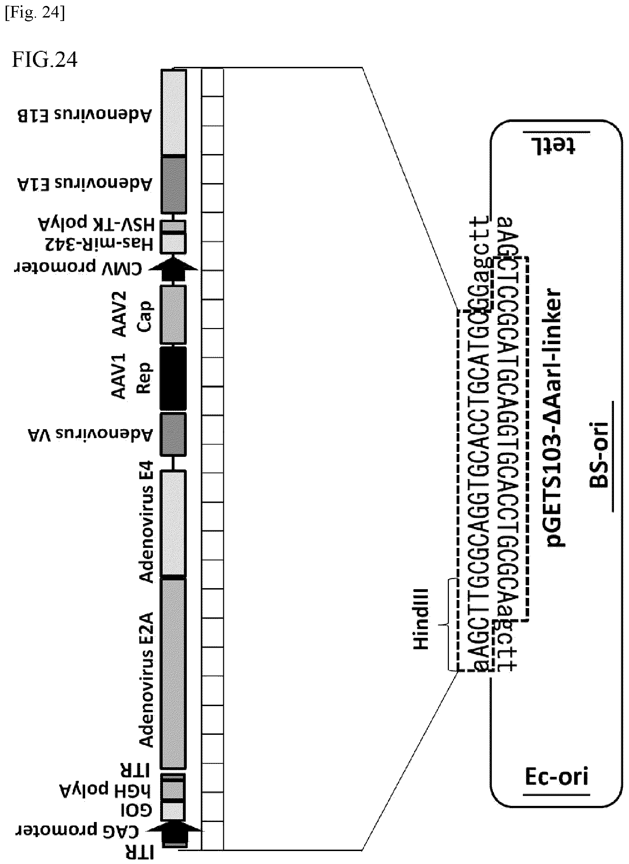

- FIG. 24 is a construction example in which the element of the Helper gene based on pBETS103-ΔAarI is modified (adenoviruses E1A and E1B are added separately from other elements).

### (Example 9: Construction of viral vector plasmids based on various viruses)

AAV vector plasmids can be constructed to further contain E1A and E1B genes in addition to the structures of the Examples described above when using producer cells other than HEK293 (NIH3T3, HT1080, A549, HeLa, HEK 293T, and the like).

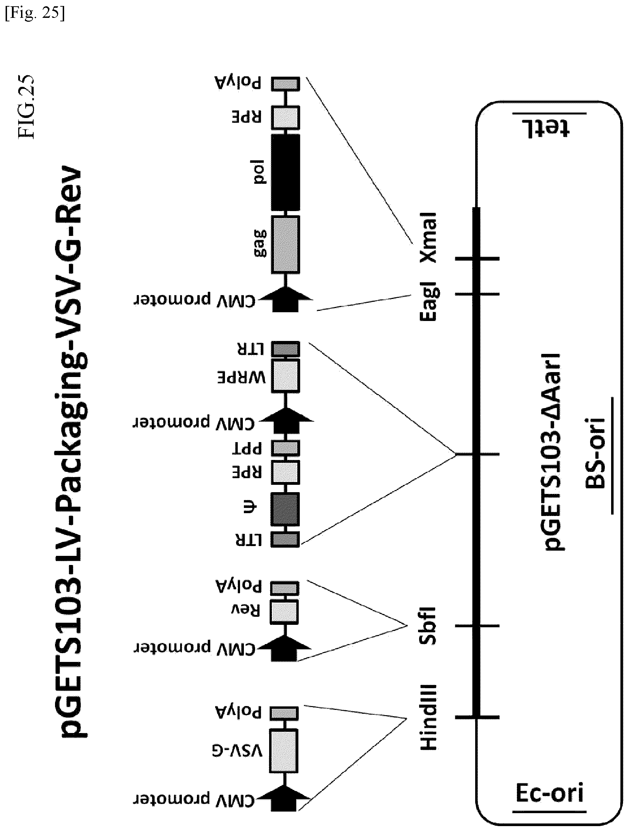

For example, as a specific configuration of the retroviral vector plasmid, the lentiviral vector plasmid, or the adenoviral vector plasmid, FIGS. 25 to 27 are assumed based on pGETS103ΔAarI of the Examples described above.

### Lentiviral vector plasmid (FIG. 25)

The following nucleic acid sequences are added based on pGETS103ΔAarI to construct a vector plasmid.
- CMV promoter, VSV-G (glycoprotein G of vesicular stomatitis virus), polyA·CMV promoter, rev, polyA
- 5' LTR, Ψ (packaging signal sequence), RPE, PPT, CMV promoter, WPRE, 3' LTR
- CMV promoter, gag, pol, RPE, polyA

### (The desired gene can be located downstream of the promoter between the LTRs.)

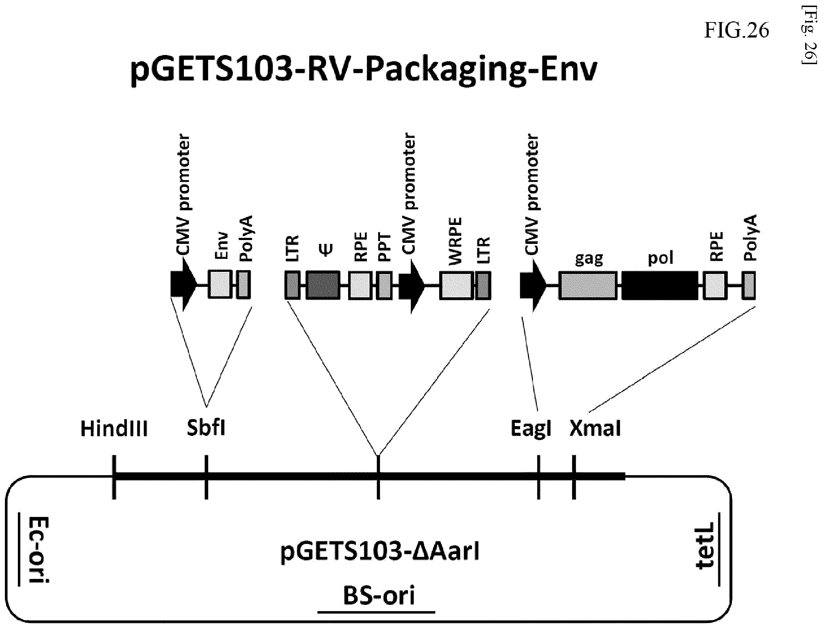

### Retroviral vector plasmid (FIG. 26)

The following nucleic acid sequences are added based on pGETS103ΔAarI to construct a vector plasmid.
- CMV promoter, env, poly A
- 5' LTR, Ψ (packaging signal sequence), RPE, PPT, CMV promoter, WPRE, 3' LTR
- CMV promoter, gag, pol, RPE, polyA
(The desired gene can be located downstream of the promoter between the LTRs.)

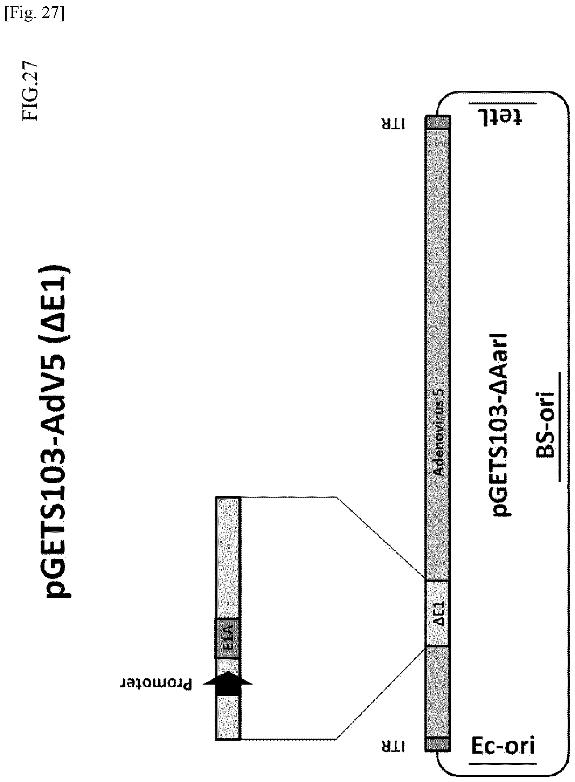

### Adenoviral vector plasmid (FIG. 27)

The following nucleic acid sequences are added based on pGETS103ΔAarI to construct a vector plasmid.
- 5' ITR, adenovirus serotype 5 genome (replacing the site of E1 gene with promoter and E1A gene), 3' ITR

(In addition to the E1A gene, the desired gene may also be located downstream of the above or other promoters.)

### (Example 10: Construction of entire viral vector plasmids by OGAB method)

Entire viral vector plasmids were prepared by the OGAB method using DNA fragments encoding elements of other vector plasmids, such as drug resistance genes (vector portions and those described As used herein), in addition to the elements related to AAV vector production. The vector portion is also prepared by the OGAB method, such that the portion of the vector plasmid not directly related to the production of the viral vector can be designed more flexibly. When the vector portion is adapted to various vector plasmids, it can be required to contain a large number of elements in the vector portion. However, when the vector portion is designed for each vector plasmid, unnecessary portions for the vector plasmid can be omitted, such that the vector plasmid can be reduced in size.

### • Preparation of integrated fragments for OGAB

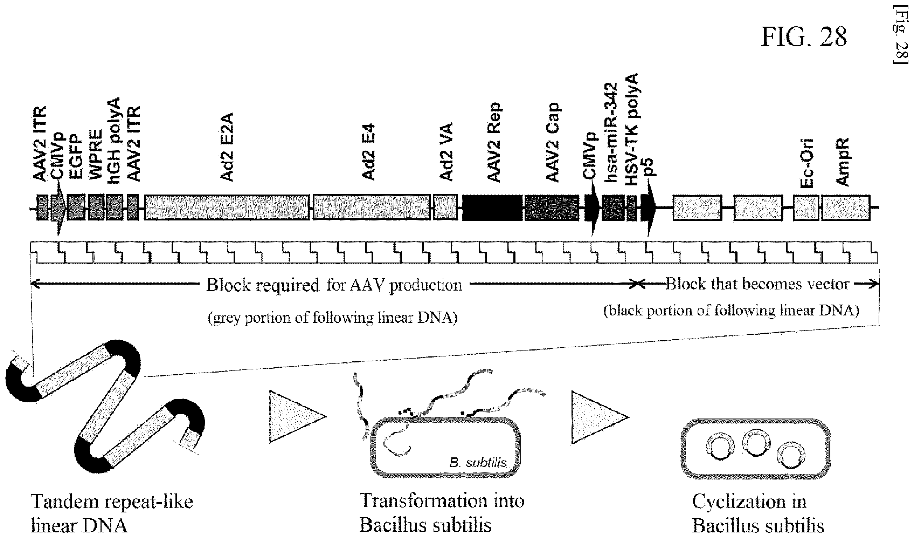

As illustrated in FIG. 28, each fragment was designed so as to divide the entire sequence including the vector region into 30 pieces (SEQ ID NOs: 67 to 96). For each of the 2nd to 4th, 22nd, 23rd, 28th, and 30th fragments, material DNA containing the sequences of SEQ ID NOs: 97 to 138 was chemically synthesized, and then prepared by MAP method (JP 2019-198236 A). Specifically, a single-stranded DNA constituting each fragment was assembled by MAP method to prepare a double-stranded DNA. The remaining fragments were amplified by a PCR method using pGETS151-AAV-Helper-RC2 as a template using a combination of F and R primers with the numbers of the respective fragments set forth in SEQ ID NOs: 139 to 184 under the following conditions. In each reaction, 2 µL of 10 × ExTaq Buffer (Takara Bio Inc.), 1.6 µL of 2.5 mM dNTP (Takara Bio Inc.), 1 µL of template DNA (1 ng), 1 µL of a solution containing 3.2 pmol each of primers of both F and R, and 13.3 µL of sterile water were added, and the mixture was kept at a temperature of 94°C for 2 minutes and then subjected to 30 cycles of 98°C for 20 seconds, 58°C for 30 seconds, and 72°C for 1 minute.

### • Cloning of integrated fragment for OGAB into vector

The double-stranded DNA fragment was purified using MinElute PCR Purification Kit (Qiagen N.V.), and finally eluted from the column with 15 µL of TE buffer (Nacalai Tesque INC.). To 1.4 µL of the resulting DNA solution, 0.2 µL of 10 × Ex-Taq Buffer (Takara Bio Inc.), 0.2 µL of 2 mM dNTP (Takara Bio Inc.), and 0.2 µL of 10 × A-attachment mix (Toyobo Co., Ltd.) were added, and the mixture was reacted at 60°C for 1 hour. Thereafter, 0.5 µL of pBR322-ΔTypeIIS (4 ng/µL) and 2.5 µL of DNA Ligation Kit <Mighty Mix> were added thereto, a ligation reaction was performed at 16°C overnight, and transformation was performed using Escherichia coli JM109 competent cell (Takara Bio Inc.). By confirming the base sequence of the colonies appearing on the LB plate containing carbenicillin cultured at 37°C overnight, a clone having a correct sequence for each fragment was obtained.

### • Preparation of equimolar mixture of fragments for OGAB

Escherichia coli with these clones was grown in 2 mL of LB medium, and then, plasmids were purified using automated system QIAcube by QIAcube by QIAprep Spin Miniprep Kit (Qiagen N.V.) using 900 µL of the resultant and finally eluted into 30 µL of TE buffer. TE buffer was added to a solution containing 4 µg of plasmid DNAs among them until a liquid volume reached 50 µL. Further, 6 µL of 10 X Plasmid Safe Buffer (epicentre), 2.4 µL of 25 mM ATP, and 2 µL of Plasmid-Safe ATP-Dependent DNase (epicentre) were added thereto, and the mixture was reacted at 37°C for 1 hour and then deactivated at 75°C for 30 minutes to decompose DNA having a structure other than a circular structure. Thereafter, the reaction solution was purified using MinElute PCR Purification Kit (Qiagen N.V.) and finally eluted from the column with 15 µL of TE buffer (Nacalai Tesque INC.). The resulting DNA solution was measured using a microspectrophotometer Nano drop One (Thermo Fisher Scientific Inc.), and diluted by adding TE buffer so as to be 100 ng/µL. Thereafter, the concentration was measured again using a microspectrophotometer Lunatic (Unchained Lab, LLC), and the DNA volume (µL) required to accurately fractionate 500 ng of DNA was calculated to 2 two decimal places, and the volume was fractionated with a micropipette to perform equimolar fractionation of each plasmid. The fractionated DNA solutions were pooled in each of two 1.5 mL centrifuge tubes depending on the type of restriction enzyme to be subsequently used. Plasmids cloning the 1st, 3rd, 4th, 6th, 7th, 9th, 10th, 13th, 17th, 19th, 20th, 23rd, 25th, 27th, and 29th fragments were pooled into tubes for cleavage with the restriction enzyme AarI, and plasmids cloning the 2nd, 5th, 8th, 11th, 12th, 14th, 15th, 16th, 18th, 21st, 22nd, 24th, 26th, 28th, and 30th fragments were pooled into tubes for cleavage with the restriction enzyme BbsI, respectively. Thereafter, 2 volumes of sterilized water, 0.33 volumes of 10 × Buffer AarI (Thermo Fisher Scientific Inc.), 0.06 volumes of 50 × oligonucleotide (0.025 mM), and 0.17 volumes of AarI (Thermo Fisher Scientific Inc.) were added to the tube for cleavage with AarI when the total volume of the preparative solution of each plasmid was 1 volume, and the mixture was reacted at 37°C for 2 hours. In addition, to the tube for cleavage with BbsI, 2 volumes of sterile water, 0.33 volumes of 10 X Cut Smart Buffer (NEB), and 0.17 volumes of BbsI-HF were added when the total volume of the preparative solution of each plasmid solution was 1 volume, and the mixture was reacted at 37°C for 2 hours. An equal amount of TE saturated phenol to the restriction enzyme reaction solution was added to each tube, and the mixture was mixed well to deactivate the restriction enzyme. Thereafter, the mixture of the phenol and the DNA solution in the emulsified state was integrated into one 2 mL centrifuge tube, centrifuged at 15,000 x g for 5 minutes, and separated into a phenol phase and an aqueous phase. The upper layer was transferred to a new tube, 50 µL of P3 buffer was added thereto, 900 µL of ethanol was added thereto, and centrifugation was performed at 15,000 x g for 10 minutes. The supernatant was discarded so that no precipitate was lost, the precipitate was rinsed with 900 µL of 70% ethanol, and then, the liquid portion was removed with a pipette. Thereafter, the mixture was centrifuged again, the liquid remaining was collected at the bottom, and the liquid was completely removed with a pipette to obtain a precipitate. Thereafter, the precipitate was dissolved in 30 µL of TE buffer.

An equimolar mixture of 25 types of fragments of about 850 bp cut out from pBR322 was size-fractionated by electrophoresis with low melting point agarose gel electrophoresis, and a DNA fragment region was cut out from the gel after the electrophoresis and the DNA fragment was purified to obtain a DNA solution as a purified product of a mixture of 25 types of DNA fragments.

• Construction of pGETS151-AAV-EGFP-WPRE-Helper-RC2 by OGAB method

To 6.4 µL (300 ng) of the resulting DNA solution, 5.6 µL of sterilized water and 15 µL of 2 x ligation buffer (15% polyethylene glycol 6,000, 500 mM NaCl, 132 mM Tris

·HCl (pH 7.6), 13.2 mM MgCl₂, 20 mM DTT, 0.2 mM ATP) were added and mixed well, and thereafter, to 18 µL of the resultant, 2 µL of T4 DNA Ligase (Takara Bio Inc.) was added, and the mixture was incubated at 37°C for 5 and a half hours. The solution was divided into two parts each having a volume of 10 µL, 100 µL of each of the competent cells of Bacillus subtilis was added thereto, and rotary culture was performed at 37°C for 30 minutes by a duck rotor. The resulting culture medium was spread on an LB plate containing 10 µg/mL of tetracycline (Nacalai Tesque INC.) and cultured at 37°C overnight.

### • Confirmation of plasmid structure of transformant

Six colonies were randomly selected and cultured at 37°C overnight in 2 mL of medium containing 10 µg/mL of tetracycline. A small amount of plasmids was extracted from the resulting bacterial cells as follows.

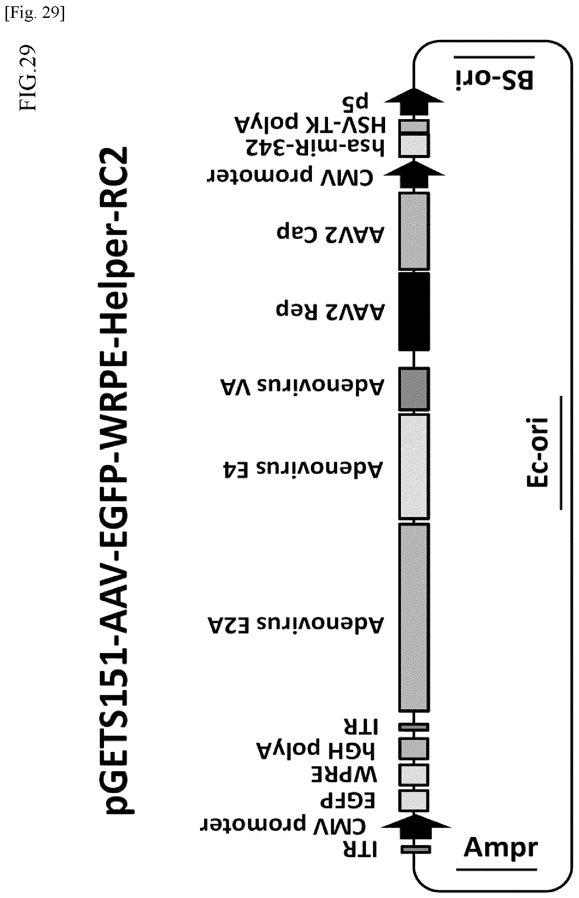

First, 900 µL of the bacterial solution was placed in a 1.5 mL tube and centrifuged at 6,800 × g for 3 minutes, and the supernatant was removed with a micropipette. The resulting cell pellets were well suspended, 100 µL of a P1 buffer containing 10 mg/mL of egg white lysozyme (Wako Pure Chemical Industries, Ltd.) was added, and the cell pellets were suspended with a vortex mixer and then incubated at 37°C for 5 minutes. Then, 200 µL of P2 buffer was added and mixed by inversion. Thereafter, 150 µL of P3 buffer was added, and the mixture was mixed by vigorously shaking the tube 3 times in the vertical axis direction. The resultant was centrifuged at 15,000 rpm for 5 minutes to separate into a white precipitate and a supernatant. The supernatant was transferred to a new 1.5 mL centrifuge tube, into which 450 µL of TE saturated phenol (Nacalai Tesque INC.) was added, and mixed well. Then, the mixture was centrifuged at 10,000 rpm for 10 minutes to separate into a phenol phase and an aqueous phase. 300 µL of the aqueous phase was transferred to a new tube, 900 µL of ethanol was added thereto, and the mixture was centrifuged at 10,000 rpm for 10 minutes. The supernatant was removed with a micropipette, 900 µL of 70% ethanol was added, and the entire tube was rinsed. Thereafter, 70% ethanol was removed with a micropipette and centrifuged again at 10,000 rpm for 1 minute. The supernatant was completely removed with a micropipette, and the precipitate was dissolved with 27 µL of TE buffer containing 30 µg/mL of RNaseA. 8 µL of the resulting plasmid solution was taken, and 1 µL of 10 X CutSmart Buffer (NEB) and 0.5 µL of SacI-HF or SfiI were added thereto. The mixture was incubated at 37°C for 15 minutes, and the enzyme was deactivated by a heat treatment at 65°C for 5 minutes. The restriction enzyme cleavage pattern was confirmed by agarose gel electrophoresis of the restriction enzyme treated product. The entire base sequence of the clone showing the correct cleavage pattern was determined, and it was confirmed that pGETS151-AAV-EGFP-WPRE-Helper-RC2 (FIG. 29) was constructed.

### • Production of rAAV using All-in-One plasmid (pGETS151-AAV-EGFP-WPRE-Helper-RC2)

The influence on viral particle production was evaluated using an All-in-One plasmid with the same genes as the three plasmids used for Triple-transfection.

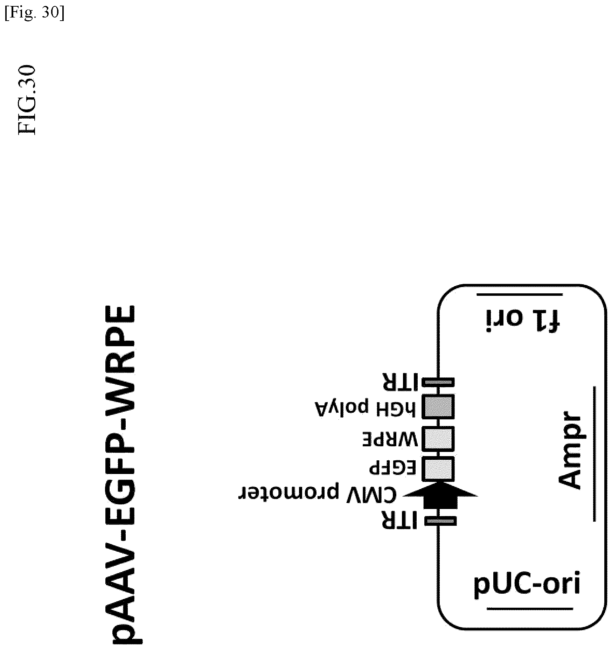

1 x 10⁶ HEK293 cells were seeded in a 6-well plate the day before transfection. After 22 hours, the medium was replaced with 1.9 mL of DMEM medium (2% FBS, 1% penicillin/streptomycin), and culture was performed at 37°C and 5% CO₂ for 2 hours. In Triple-transfection, 0.48 µg of pAAV-EGFP-WPRE plasmid (FIG. 30), 0.52 µg of pRC-mi342 plasmid (FIG. 7), 0.95 µg of pHelper plasmid (FIG. 8), and 100 µL of a transfection solution obtained by mixing PEI pro in an amount of 1-fold and DMEM medium were added thereto. In Single-transfection, 1.95 µg of pGETS151-AAV-EGFP-WPRE-Helper-RC2 plasmid (FIG. 29), and 100 µL of a transfection solution obtained by mixing PEI pro in an amount of 1-fold and DMEM medium were added. After culturing at 37°C and 5% CO₂ for 72 hours, 1/80 volume of 0.5M EDTA (pH 8.0) was added, and the mixture was allowed to stand at room temperature for 10 minutes. The detached cell suspension was recovered and centrifuged at 180 × g for 5 minutes to completely remove the supernatant. The cell pellets were suspended in 200 µL of Lysis buffer (2 mM MgCl₂, 150 mM NaCl, 50 mM Tris, 0.1% Triton (registered trademark) X-100; pH 8.5), benzonase (25 units) was further added thereto, and the mixture was reacted at 37°C for 30 minutes. Thereafter, 2/100 volume of a 1.9 M MgSO₄ solution was added, and the mixture was allowed to stand at room temperature for 10 minutes. The supernatant was recovered by centrifugation at 12,000 x g and 4°C for 10 minutes, and Pluronic (registered trademark) F-68 was added at a final concentration of 0.001%.

### • Evaluation of rAAV

A sample obtained by subjecting the supernatant to DNaseI treatment for 30 minutes and reacting the treated supernatant at 95°C for 10 minutes was diluted 1,000 times and subjected to qPCR. Primer sequences of 5'-CATCAATGGGCGTGGATAGC-3' (SEQ ID NO: 185) and 5'-GGAGTTGTTACGACATTTTGGAAA-3' (SEQ ID NO: 186) were used for targeting the CMV promoter. As the reaction conditions, after 10 minutes at 95°C, 40 cycles of 95°C/15 seconds, 55°C/5 seconds, and 72°C/30 seconds were repeated.

Each plasmid used in Examples above was obtained or produced as follows.
- 6230_pAAV-CMV
   (6230_pAAV-CMV) purchased from Takara Bio Inc.
- 6230_pRC2-mi342
   (6230_pRC2-mi342) purchased from Takara Bio Inc.
- 6230_pHelper
   (6230_pHelper) purchased from Takara Bio Inc.
- SYNp204_pAAV-EGFP-WPRE

pAAV-CMV plasmid (Takara Bio Inc.: 6230) was treated with a restriction enzyme (AflII, BamHI). The following fragments were inserted therein using InFusion. EGFP-WPRE fragments were prepared using ppGETS151-AAV-EGFP-WPRE-Helper-RC2 as a template using the following primers:primer sequences 5'-GAGCTGGTTTAGTGGATATC-3' (SEQ ID NO: 187) and 5'-CAGGGATGCCACCCGTTTAATTA-3' (SEQ ID NO: 188).

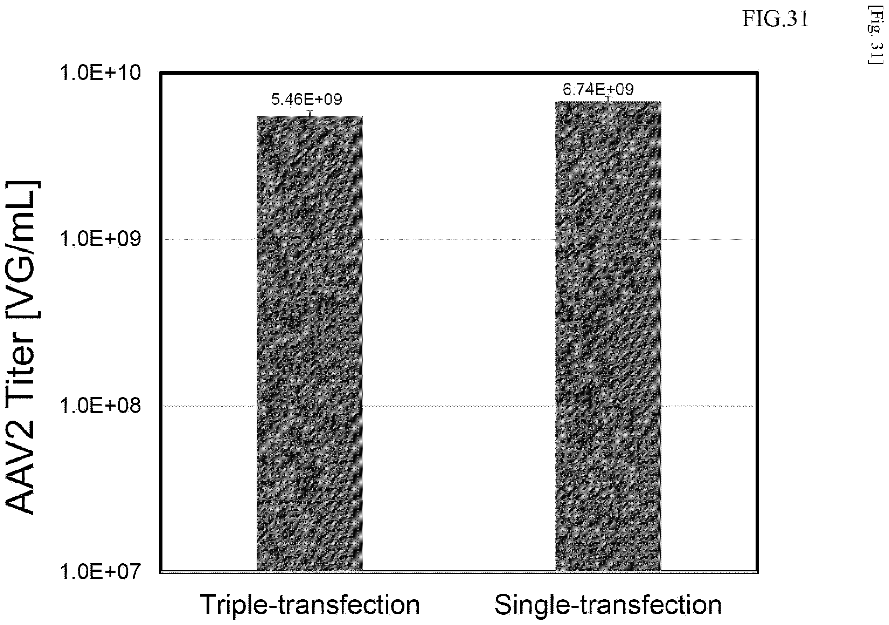

The amounts of viral vectors produced under the conditions of Triple-transfection and Single-transfection are illustrated in FIG. 31. It was confirmed that all of the viral vector plasmids were designed and prepared by the OGAB method, and the viral vector was highly produced from the viral vector plasmid thus produced.

### (Note)

Although the present disclosure has been illustrated using preferred embodiments of the present disclosure as described above, it is understood that the scope of the present disclosure should be interpreted only by the claims. It is understood that the patents, patent applications, and documents cited As used herein are to be incorporated by reference As used herein in the same manner as the contents are specifically described As used herein.

The present application claims priority to Japanese Patent Application No. 2022-76060 filed to the Japan Patent Office on May 2, 2022. The entire content thereof is incorporated herein by reference.

### Industrial Applicability

The present disclosure provides a virus-derived construct plasmid having a novel structure and can allow for the supply of a virus-derived construct based on the virus-derived construct plasmid.

### [Sequence Listing Free Text]

SEQ ID NO: 1: Primer
   GGAACCCCTAGTGATGGAGTT
SEQ ID NO: 2: Primer
   CGGCCTCAGTGAGCGA
SEQ ID NO: 3: Linker F
   AGCTTGCGCAGGTGCACCTGCATGCGG
SEQ ID NO: 4: Linker R
   AGCTCCGCATGCAGGTGCACCTGCGCA
SEQ ID NO: 5: 1-1th 
TAGAGGCAAGGGTTGTTTTTATTGACTACACCTGCGATCAAGCTTGCCT
GCAGGCAGCTGCGCGCTCGCTCGCTCACTGAGGCCGCCCGGGCAAAGCCCGGG
CGTCGGGCGACCTTTGGTCGCCCGGCCTCAGTGAGCGAGCGAGCGCGCAGAGA
GGGAGTGGCCAACTCCATCACTAGG
SEQ ID NO: 6: 1-2th 
GGTCGTTGGGCGGTCAGCCAGGCGGGCCATTTACCGTAAGTTATGTAA
CGCGGAACTCCATATATGGGCTATGAACTAATGACCCCGTAATTGATTACTATTAA
AGCTTGATATCGATAGGAACCCCTAGTGATGGAGTTGGCCACTCCCTCTCT
SEQ ID NO: 7: 1-3th 
ATGGCCCGCCTGGCTGACCGCCCAACGACCCCCGCCCATTGACGTCAA
TAATGACGTATGTTCCCATAGTAACGCCAATAGGGACTTTCCATTGACGTCAATG
GGTGGAGTATTTACGGTAAACTGCCCACTTGGCAGTACATCAAGTGTATCAT
SEQ ID NO: 8: 1-4th 
GTAATAGCGATGACTAATACGTAGATGTACTGCCAAGTAGGAAAGTCCC
ATAAGGTCATGTACTGGGCATAATGCCAGGCGGGCCATTTACCGTCATTGACGTC
AATAGGGGGCGTACTTGGCATATGATACACTTGATGTACTGCCAAGTGGGC
SEQ ID NO: 9: 1-5th 
GTACATCTACGTATTAGTCATCGCTATTACCATGGTGATGCGGTTTTGGC
AGTACATCAATGGGCGTGGATAGCGGTTTGACTCACGGGGATTTCCAAGTCTCC
ACCCCATTGACGTCAATGGGAGTTTGTTTTGGCACCAAAATCAACGGGACT
SEQ ID NO: 10: 1-6th 
TACTGGTTGGAAGAAAGACCTCTATCACCTGCGATCCTGGGCCCTTAA
GGATATCCACTAAACCAGCTCTGCTTATATAGACCTCCCACCGTACACGCCTACC
GCCCATTTGCGTCAATGGGGCGGAGTTGTTACGACATTTTGGAAAGTCCCGTTG
ATTTTGGTGCCAAAACAAAC
SEQ ID NO: 11: 3-1th 
TAGAGGCAAGGGTTGTTTTTATTGACTACACCTGCGATCGGAACCAAG
CTGGAGTGCAGTGGCACAATCTTGGCTCACTGCAATCTCCGCCTCCTGGGTTCA
AGCGATTCTCCTGCCTCAGCCTCCCGAGTTGTTGGGATTCCAGGCATGCATGACC
AGGCTCAGCTAATTTTTGTT
SEQ ID NO: 12: 3-2th 
AGGGAGCAGTGGTTCACGCCTGTAATCCCAGCAATTTGGGAGGCCAAG
GTGGGTAGATCACCTGAGATTAGGAGTTGGAGACCAGCCTGGCCAATATGGTGA
AACCCCGTCTCTACCAAAAAAACAAAAATTAGCTGAGCCTGGTCATGC
SEQ ID NO: 13: 3-3th 
GGATTACAGGCGTGAACCACTGCTCCCTTCCCTGTCCTTATCGATAGAT
CTAGGAACCCCTAGTGATGGAGTTGGCCACTCCCTCTCTGCGCGCTCGCTCGCTC
ACTGAGGCCGGGCGACCAAAGGTCGCCCGACGCCCGGGCTTTGCCCGG
SEQ ID NO: 14: 3-4th 
GGAAGCTGTAGAGCTGTTCCTGGTTGCGACGCAGGGTGGGCTGTACCT
GGGGACTGTTAAGCATGGAGTTGGGTACCGGATCTGCCTGCAGGCAGCTGCGCG
CTCGCTCGCTCACTGAGGCCGCCCGGGCAAAGCCCGGGCGTCGGGCGACC
SEQ ID NO: 15: 3-5th 
CGCAACCAGGAACAGCTCTACAGCTTCCTGGAGCGCCACTCGCCCTAC
TTCCGCAGCCACAGTGCGCAGATTAGGAGCGCCACTTCTTTTTGTCACTTGAAA
AACATGTAAAAATAATGTACTAGGAGACACTTTCAATAAAGGCAAATGT
SEQ ID NO: 16: 3-6th 
TACTGGTTGGAAGAAAGACCTCTATCACCTGCGATCTGTCCCTGCCAGT
GGCGCATAGCGATGCGCGGCAGAACCCCTTTGATTTTTAAACGGCGCAGACGGC
AAGGGTGGGGGGTAAATAATCACCCGAGAGTGTACAAATAAAAACATTTGCCTT
TATTGAAAGTGTCTCCT
SEQ ID NO: 17: 16-1th 
TAGAGGCAAGGGTTGTTTTTATTGACTAGGTCTCGCAGGTACATCTACG

GATTACGTCGACGTTTAAACCATATGAGCGGCCGCTCTAGAACTAGTGGATCCCC

CGGAAGATCAGAAGTTCCTATTCCGAAGTTCCTATTCTCTAGAAAGTATAGGAAC

TTCTGATCTGCGCAGCCGCC
SEQ ID NO: 18: 16-2th 
CAGAATCTGGCGGCAACTCCCATTCCTTCTCGGCCACCCAGTTCACAA
AGCTGTCAGAAATGCCGGGCAGATGCTCGTCAAGGTCGCTGGGGACCTTAATCA
CAATCTCGTAAAACCCCGGCATGGCGGCTGCGCAGATCAGAAGTTCCTATAC
SEQ ID NO: 19: 16-3th 
AGAAGGAATGGGAGTTGCCGCCAGATTCTGACATGGATCTGAATCTGA
TTGAGCAGGCACCCCTGACCGTGGCCGAGAAGCTGCAGCGCGACTTTCTGACG
GAATGGCGCCGTGTGAGTAAGGCCCCGGAGGCCCTTTTCTTTGTGCAAT
SEQ ID NO: 20: 16-4th CCGCGGTAAATTCTCTGAATCAGTTTTTCGCGAATCTGACTCAGGAAAC 
GTCCCAAAACCATGGATTTCACCCCGGTGGTTTCCACGAGCACGTGCATGTGGA

AGTAGCTCTCTCCCTTCTCAAATTGCACAAAGAAAAGGGCCTCCGGGGCCT
SEQ ID NO: 21: 16-5th 
CGAAAAACTGATTCAGAGAATTTACCGCGGGATCGAGCCGACTTTGCC
AAACTGGTTCGCGGTCACAAAGACCAGAAATGGCGCCGGAGGCGGGAACAAG
GTGGTGGATGAGTGCTACATCCCCAATTACTTGCTCCCCAAAACCCAGCCTGAG
SEQ ID NO: 22: 16-6th 
TACTGGTTGGAAGAAAGACCTCTATGGTCTCCCTGCTCCTGCGTCTGCG
ACACGTGCGTCAGATGCTGCGCCACCAACCGTTTACGCTCCGTGAGATTCAAAC
AGGCGCTTAAATACTGTTCCATATTAGTCCACGCCCACTGGAGCTCAGGCTGGGT
TTTGGGGAGCAAGTAATT
SEQ ID NO: 23: 1-F
   TAGCACCTGCATGCAAGCTTGCCTGCAGGCA
SEQ ID NO: 24: 1-R
   TAGCACCTGCGCATCTGGGCCCTTAAGGATATC
SEQ ID NO: 25: 2-F
   TAGGGTCTCGCCAGCCGGCC
SEQ ID NO: 26: 2-R
   TAGGGTCTCCTTCCCAATAGACCCCGCA
SEQ ID NO: 27: 3-F
   TAGCACCTGCATGCGGAACCAAGCTGGAGTG
SEQ ID NO: 28: 3-R
   TAGCACCTGCGCATTGTCCCTGCCAGTGG
SEQ ID NO: 29: 4-F
   TAGGGTCTCGGACACGTTGCGATACTGGT
SEQ ID NO: 30: 4-R
   TAGGGTCTCCCACGAGCCCACGG
SEQ ID NO: 31: 5-F
   TAGCACCTGCATGCCGTGGTGCTTGTAGGTTAC
SEQ ID NO: 32: 5-R
   TAGCACCTGCGCATGAGCGCGGACGC
SEQ ID NO: 33: 6-F
   TAGGGTCTCGGCTCGGGGGTGGT
SEQ ID NO: 34: 6-R
   TAGGGTCTCCTTGCCGCGCGTTTCTC
SEQ ID NO: 35: 7-F
   TAGCACCTGCATGCGCAAACGCTCTGCAACAAGA
SEQ ID NO: 36: 7-R
   TAGCACCTGCGCATGTCCGCCAGGTGC
SEQ ID NO: 37: 8-F
   TAGGGTCTCGGGACATTATCTTCCCCGAAC
SEQ ID NO: 38: 8-R
   TAGGGTCTCCGTGGCGGCGGC
SEQ ID NO: 39: 9-F
   TAGCACCTGCATGCCCACCCACGGACGA
SEQ ID NO: 40: 9-R
   TAGCACCTGCGCATGAGGGAGCGCAGAGA
SEQ ID NO: 41: 10-F
   TAGGGTCTCGCCTCACCCGCAGCTG
SEQ ID NO: 42: 10-R
   TAGGGTCTCCGACTAAAAAATGACGTAACGGTTAAAGTC
SEQ ID NO: 43: 11-F
   TAGCACCTGCATGCAGTCCTATATATACTCGCTCTGTACT
SEQ ID NO: 44: 11-R
   TAGCACCTGCGCATGGAGCTATGCTAACCAGC
SEQ ID NO: 45: 12-F
   TAGGGTCTCGCTCCGAGTATGCGTGTCA
SEQ ID NO: 46: 12-R
   TAGGGTCTCCGTAGTTGTAGTATATCCACTCTCTCA
SEQ ID NO: 47: 13-F
   TAGCACCTGCATGCCTACTACACAGAGCGAGCT
SEQ ID NO: 48: 13-R
   TAGCACCTGCGCATGCACAGCACCACAATATTGTTCA
SEQ ID NO: 49: 14-F
   TAGCACCTGCATGCGTGCTGCAGTTACTGTGCT
SEQ ID NO: 50: 14-R
   TAGCACCTGCGCATTAGCGAGGTAAGCACTTACTCT
SEQ ID NO: 51: 15-F
   TAGGGTCTCGGCTAGTTTCTGTGGATTCACTAGA
SEQ ID NO: 52: 15-R
   TAGGGTCTCCCCTGGACATCCAGGTGA
SEQ ID NO: 53: 16-F
   TAGGGTCTCGCAGGTACATCTACGGATTACGT
SEQ ID NO: 54: 16-R
   TAGGGTCTCCCTGCTCCTGCGTCTG
SEQ ID NO: 55: 17-F
   TAGCACCTGCATGCGCAGAACAAAGAGAATCAGAATCC
SEQ ID NO: 56: 17-R
   TAGCACCTGCGCATGGTGAGTTCAAATTTGAACATCCG
SEQ ID NO: 57: 18-F
   TAGGGTCTCGCACCCGCCGTCTG
SEQ ID NO: 58: 18-R
   TAGGGTCTCCCGAGGGCCGCG
SEQ ID NO: 59: 19-F
   TAGCACCTGCATGCCTCGAGCACGACAAAGC
SEQ ID NO: 60: 19-R
   TAGCACCTGCGCATTGACTTGAATGTTAAAGAGCTTGAAGTTGA
SEQ ID NO: 61: 20-F
   TAGGGTCTCGGTCAAAGAGGTCACGCAGA
SEQ ID NO: 62: 20-R
   TAGGGTCTCCTTGGTTGTCCTGATTTCCTCTTC
SEQ ID NO: 63: 21-F
   TAGCACCTGCATGCCCAATCCCGTGGCTAC
SEQ ID NO: 64: 21-R
   TAGCACCTGCGCATGCATATGATACACTTGATGTACTGC
SEQ ID NO: 65: 22-F
   TAGGGTCTCGATGCCAAGTACGCCCCCT
SEQ ID NO: 66: 22-R
   TAGGGTCTCCCTCCCGGGCTGTAGT
SEQ ID NO: 67: Fragment 1 
TAGAGGCAAGGGTTGTTTTTATTGACTACACCTGCGATCAAAAGTGCC
ACCTGACGTCTAAGAAACCATTATTATCATGACATTAACCTATAAAAATAGGCGTA
TCACGAGGCCCTTTCGTCTTCAAGAATTCTCATGTTTGACAGCTTATCATCGATAA
GCTTGCCTGCAGGCAGCTGCGCGCTCGCTCGCTCACTGAGGCCGCCCGGGCAA
AGCCCGGGCGTCGGGCGACCTTTGGTCGCCCGGCCTCAGTGAGCGAGCGAGCG
CGCAGAGAGGGAGTGGCCAACTCCATCACTAGGGGTTCCTATCGATATCAAGCT
TTAATAGTAATCAATTACGGGGTCATTAGTTCATAGCCCATATATGGAGTTCCGCG
TTACATAACTTACGGTAAATGGCCCGCCTGGCTGACCGCCCAACGACCCCCGCC
 
CATTGACGTCAATAATGACGTATGTTCCCATAGTAACGCCAATAGGGACTTTCCAT
TGACGTCAATGGGTGGAGTATTTACGGTAAACTGCCCACTTGGCAGTACATCAA
GTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATC
TACGTATTAGTCATCGCTATTACCATGGTGATGCGGTTTTGGCAGTACATCAATGG
GCGTGGATAGCGGTTTGACTCACGGGGATTTCCAAGTCTCCACCCCATTGACGT
CAATGGGAGTTTGTTTTGGCACCAAAATCAACGGGACTTTCCAAAATGTCGTAA
CAACTCCGCCCCATTGACGGATCGCAGGTGATAGAGGTCTTTCTTCCAACCAGTA
SEQ ID NO: 68: Fragment 2 
TAGAGGCAAGGGTTGTTTTTATTGACTAGAAGACGAGACGCAAATGGG
CGGTAGGCGTGTACGGTGGGAGGTCTATATAAGCAGAGCTGGTTTAGTGGATATC
GTATACGCCACCATGGTGAGCAAGGGCGAGGAGCTGTTCACCGGGGTGGTGCCC
ATCCTGGTCGAGCTGGACGGCGACGTAAACGGCCACAAGTTCAGCGTGTCCGG
CGAGGGCGAGGGCGATGCCACCTACGGCAAGCTGACCCTGAAGTTCATCTGCA
CCACCGGCAAGCTGCCCGTGCCCTGGCCCACCCTCGTGACCACCCTGACCTACG
GCGTGCAGTGCTTCAGCCGCTACCCCGACCACATGAAGCAGCACGACTTCTTCA
AGTCCGCCATGCCCGAAGGCTACGTCCAGGAGCGCACCATCTTCTTCAAGGACG
ACGGCAACTACAAGACCCGCGCCGAGGTGAAGTTCGAGGGCGACACCCTGGTG
AACCGCATCGAGCTGAAGGGCATCGACTTCAAGGAGGACGGCAACATCCTGGG
GCACAAGCTGGAGTACAACTACAACAGCCACAACGTCTATATCATGGCCGACAA
GCAGAAGAACGGCATCAAGGTGAACTTCAAGATCCGCCACAACATCGAGGACG
GCAGCGTGCAGCTCGCCGACCACTACCAGCAGAACACCCCCATCGGCGACGGC
CCCGTGCTGCTGCCCGACAACCACTACCTGAGCACCCAGTCCGCCCTGAGCAAA
GACCCCAACGAGAAGCGCGATCACATGGTCCTGCTGGAGTTCGTGACCGCCGCC
GGGATCACTCTCGGCATGGACGAGCTGTACAAGGAGTCTTCATAGAGGTCTTTC
TTCCAACCAGTA
SEQ ID NO: 69: Fragment 3 
TAGAGGCAAGGGTTGTTTTTATTGACTACACCTGCGATCCAAGTAAGTA
TACCGATAATCAACCTCTGGATTACAAAATTTGTGAAAGATTGACTGGTATTCTTA
ACTATGTTGCTCCTTTTACGCTATGTGGATACGCTGCTTTAATGCCTTTGTATCATG
CTATTGCTTCCCGTATGGCTTTCATTTTCTCCTCCTTGTATAAATCCTGGTTGCTGT
CTCTTTATGAGGAGTTGTGGCCCGTTGTCAGGCAACGTGGCGTGGTGTGCACTG
TGTTTGCTGACGCAACCCCCACTGGTTGGGGCATTGCCACCACCTGTCAGCTCC
TTTCCGGGACTTTCGCTTTCCCCCTCCCTATTGCCACGGCGGAACTCATCGCCGC
CTGCCTTGCCCGCTGCTGGACAGGGGCTCGGCTGTTGGGCACTGACAATTCCGT
GGTGTTGTCGGGGAAGCTGACGTCCTTTCCATGGCTGCTCGCCTGTGTTGCCAC
CTGGATTCTGCGCGGGACGTCCTTCTGCTACGTCCCTTCGGCCCTCAATCCAGCG
GACCTTCCTTCCCGCGGCCTGCTGCCGGCTCTGCGGCCTCTTCCGCGTCTTCGCC
TTCGCCCTCAGACGAGTCGGATCTCCCTTTGGGCCGCCTCCCCGCATCGGTTAAT
TAAACGGGTGGCATCCCTGTGACCCCTCCCCAGTGCCTCTCCTGGCCCTGGAAG
TTGCCACTCCAGTGCCCACCAGCCTTGTCCTAATAAAATTAAGTTGCATCATTTT
GTCTGACTAGGTGTCCTTCTATAATATTATGGGGTGGAGGGGGGTGGTATGGAGC
AAGGGGCAAGTTGGGAAGATCGCAGGTGATAGAGGTCTTTCTTCCAACCAGTA
SEQ ID NO: 70: Fragment 4 
TAGAGGCAAGGGTTGTTTTTATTGACTACACCTGCGATCGGAAGACAA
CCTGTAGGGCCTGCGGGGTCTATTGGGAACCAAGCTGGAGTGCAGTGGCACAAT
CTTGGCTCACTGCAATCTCCGCCTCCTGGGTTCAAGCGATTCTCCTGCCTCAGCC
TCCCGAGTTGTTGGGATTCCAGGCATGCATGACCAGGCTCAGCTAATTTTTGTTT
TTTTGGTAGAGACGGGGTTTCACCATATTGGCCAGGCTGGTCTCCAACTCCTAAT
CTCAGGTGATCTACCCACCTTGGCCTCCCAAATTGCTGGGATTACAGGCGTGAAC
CACTGCTCCCTTCCCTGTCCTTATCGATAGATCTAGGAACCCCTAGTGATGGAGTT
GGCCACTCCCTCTCTGCGCGCTCGCTCGCTCACTGAGGCCGGGCGACCAAAGGT
CGCCCGACGCCCGGGCTTTGCCCGGGCGGCCTCAGTGAGCGAGCGAGCGCGCA
GCTGCCTGCAGGCAGATCCCCTCGAGGGGTACCCAACTCCATGCTTAACAGTCC
 
CCAGGTACAGCCCACCCTGCGTCGCAACCAGGAACAGCTCTACAGCTTCCTGGA
GCGCCACTCGCCCTACTTCCGCAGCCACAGTGCGCAGATTAGGAGCGCCACTTC
TTTTTGTCACTTGAAAAACATGTAAAAATAATGTACTAGGAGACACTTTCAATAA
AGGCAAATGTTTTTATTTGTACACTCTCGGGTGATTATTTACCCCCCACCCTTGCC
GTCTGCGCCGTTTAAAAATCAAAGGGGTTCTGCCGCGCATCGCTATGCGCCACT
GGCAGGGACACGTTGCGATACTGGTGGATCGCAGGTGATAGAGGTCTTTCTTCC
AACCAGTA
SEQ ID NO: 71: Fragment 5 
TAGAGGCAAGGGTTGTTTTTATTGACTAGAAGACGAGGTGTTTAGTGCT
CCACTTAAACTCAGGCACAACCATCCGCGGCAGCTCGGTGAAGTTTTCACTCCA
CAGGCTGCGCACCATCACCAACGCGTTTAGCAGGTCGGGCGCCGATATCTTGAA
GTCGCAGTTGGGGCCTCCGCCCTGCGCGCGCGAGTTGCGATACACAGGGTTGCA
GCACTGGAACACTATCAGCGCCGGGTGGTGCACGCTGGCCAGCACGCTCTTGTC
GGAGATCAGATCCGCGTCCAGGTCCTCCGCGTTGCTCAGGGCGAACGGAGTCA
ACTTTGGTAGCTGCCTTCCCAAAAAGGGTGCATGCCCAGGCTTTGAGTTGCACT
CGCACCGTAGTGGCATCAGAAGGTGACCGTGCCCGGTCTGGGCGTTAGGATACA
GCGCCTGCATGAAAGCCTTGATCTGCTTAAAAGCCACCTGAGCCTTTGCGCCTT
CAGAGAAGAACATGCCGCAAGACTTGCCGGAAAACTGATTGGCCGGACAGGCC
GCGTCATGCACGCAGCACCTTGCGTCGGTGTTGGAGATCTGCACCACATTTCGG
CCCCACCGGTTCTTCACGATCTTGGCCTTGCTAGACTGCTCCTTCAGCGCGCGCT
GCCCGTTTTCGCTCGTCACATCCATTTCAATCACGTGCTCCTTATTTATCATAATGC
TCCCGTGTAGACACTTAAGCTCGCCTTCGATCTCAGCGCAGCGGTGCAGCCACA
ACGCGCAGCCCGTGGGCTCGTGGTGCTTGTAGGTTACCTCTGCAAACGACTGCA
GGTACGCCTGCAGGAATCGCCCCATCGAGTCTTCATAGAGGTCTTTCTTCCAACC
AGTA
SEQ ID NO: 72: Fragment 6 TAGAGGCAAGGGTTGTTTTTATTGACTACACCTGCGATCCATCATCGTC 
ACAAAGGTCTTGTTGCTGGTGAAGGTCAGCTGCAACCCGCGGTGCTCCTCGTTT
AGCCAGGTCTTGCATACGGCCGCCAGAGCTTCCACTTGGTCAGGCAGTAGCTTG
AAGTTTGCCTTTAGATCGTTATCCACGTGGTACTTGTCCATCAACGCGCGCGCAG
CCTCCATGCCCTTCTCCCACGCAGACACGATCGGCAGGCTCAGCGGGTTTATCA
CCGTGCTTTCACTTTCCGCTTCACTGGACTCTTCCTTTTCCTCTTGCGTCCGCATA
CCCCGCGCCACTGGGTCGTCTTCATTCAGCCGCCGCACCGTGCGCTTACCTCCCT
TGCCGTGCTTGATTAGCACCGGTGGGTTGCTGAAACCCACCATTTGTAGCGCCA
CATCTTCTCTTTCTTCCTCGCTGTCCACGATCACCTCTGGGGATGGCGGGCGCTC
GGGCTTGGGAGAGGGGCGCTTCTTTTTCTTTTTGGACGCAATGGCCAAATCCGC
CGTCGAGGTCGATGGCCGCGGGCTGGGTGTGCGCGGCACCAGCGCATCTTGTGA
CGAGTCTTCTTCGTCCTCGGACTCGAGACGCCGCCTCAGCCGCTTTTTTGGGGG
CGCGCGGGGAGGCGGCGGCGACGGCGACGGGGACGACACGTCCTCCATGGTTG
GTGGACGTCGCGCCGCACCGCGTCCGCGCTCGGGGGTGGTTTCGCGCTGCTCCT
CTTCCCGACTGGCCATTTCCTTCTCCTATAGGCAGAAAAAGATCATGGAGTCAGT
CGAGAAGGAGGACAGCCTAGATCGCAGGTGATAGAGGTCTTTCTTCCAACCAGT
A
SEQ ID NO: 73: Fragment 7 
TAGAGGCAAGGGTTGTTTTTATTGACTACACCTGCGATCCCTAACCGCC
CCCTTTGAGTTCGCCACCACCGCCTCCACCGATGCCGCCAACGCGCCTACCACC
TTCCCCGTCGAGGCACCCCCGCTTGAGGAGGAGGAAGTGATTATCGAGCAGGA
CCCAGGTTTTGTAAGCGAAGACGACGAGGATCGCTCAGTACCAACAGAGGATA
AAAAGCAAGACCAGGACGACGCAGAGGCAAACGAGGAACAAGTCGGGCGGGG
GGACCAAAGGCATGGCGACTACCTAGATGTGGGAGACGACGTGCTGTTGAAGC
ATCTGCAGCGCCAGTGCGCCATTATCTGCGACGCGTTGCAAGAGCGCAGCGATG
TGCCCCTCGCCATAGCGGATGTCAGCCTTGCCTACGAACGCCACCTGTTCTCACC
GCGCGTACCCCCCAAACGCCAAGAAAACGGCACATGCGAGCCCAACCCGCGCC
TCAACTTCTACCCCGTATTTGCCGTGCCAGAGGTGCTTGCCACCTATCACATCTTT
 
TTCCAAAACTGCAAGATACCCCTATCCTGCCGTGCCAACCGCAGCCGAGCGGAC
AAGCAGCTGGCCTTGCGGCAGGGCGCTGTCATACCTGATATCGCCTCGCTCGAC
GAAGTGCCAAAAATCTTTGAGGGTCTTGGACGCGACGAGAAACGCGCGGCAAA
CGCTCTGCAACAAGAAAACAGCGAAAATGAAAGTCACTGTGGAGTGCTGGTGG
AACTTGAGGGTGACAACGCGCGCCTAGCCGTGCTGAAACGCAGCATCGAGGTC
ACCCACTTTGCCTACCCGGCACTTAACCTACCCCCCAAGATCGCAGGTGATAGA
GGTCTTTCTTCCAACCAGTA
SEQ ID NO: 74: Fragment 8 
TAGAGGCAAGGGTTGTTTTTATTGACTAGAAGACGACCAAGGTTATGA
GCACAGTCATGAGCGAGCTGATCGTGCGCCGTGCACGACCCCTGGAGAGGGAT
GCAAACTTGCAAGAACAAACCGAGGAGGGCCTACCCGCAGTTGGCGATGAGCA
GCTGGCGCGCTGGCTTGAGACGCGCGAGCCTGCCGACTTGGAGGAGCGACGCA
AGCTAATGATGGCCGCAGTGCTTGTTACCGTGGAGCTTGAGTGCATGCAGCGGT
TCTTTGCTGACCCGGAGATGCAGCGCAAGCTAGAGGAAACGTTGCACTACACCT
TTCGCCAGGGCTACGTGCGCCAGGCCTGCAAAATTTCCAACGTGGAGCTCTGCA
ACCTGGTCTCCTACCTTGGAATTTTGCACGAAAACCGCCTCGGGCAAAACGTGC
TTCATTCCACGCTCAAGGGCGAGGCGCGCCGCGACTACGTCCGCGACTGCGTTT
ACTTATTTCTGTGCTACACCTGGCAAACGGCCATGGGCGTGTGGCAGCAATGCCT
GGAGGAGCGCAACCTAAAGGAGCTGCAGAAGCTGCTAAAGCAAAACTTGAAG
GACCTATGGACGGCCTTCAACGAGCGCTCCGTGGCCGCGCACCTGGCGGACATT
ATCTTCCCCGAACGCCTGCTTAAAACCCTGCAACAGGGTCTGCCAGACTTCACC
AGTCAAAGCATGTTGCAAAACTTTAGGAACTTTATCCTAGAGCGTTCAGGAATTC
TGCCCGCCACCTGCTGTGCGCTTCCTAGCGACTTTGTGCCCATTAAGTACCGTGA
ATGCCCTCCGCCGCTTTGGGGTCACTGCTACCTTCGAGTCTTCATAGAGGTCTTT
CTTCCAACCAGTA
SEQ ID NO: 75: Fragment 9 TAGAGGCAAGGGTTGTTTTTATTGACTACACCTGCGATCCTTCTGCAGC 
TAGCCAACTACCTTGCCTACCACTCCGACATCATGGAAGACGTGAGCGGTGACG
GCCTACTGGAGTGTCACTGTCGCTGCAACCTATGCACCCCGCACCGCTCCCTGG
TCTGCAATTCGCAACTGCTTAGCGAAAGTCAAATTATCGGTACCTTTGAGCTGCA
GGGTCCCTCGCCTGACGAAAAGTCCGCGGCTCCGGGGTTGAAACTCACTCCGG
GGCTGTGGACGTCGGCTTACCTTCGCAAATTTGTACCTGAGGACTACCACGCCC
ACGAGATTAGGTTCTACGAAGACCAATCCCGCCCGCCAAATGCGGAGCTTACCG
CCTGCGTCATTACCCAGGGCCACATCCTTGGCCAATTGCAAGCCATCAACAAAG
CCCGCCAAGAGTTTCTGCTACGAAAGGGACGGGGGTTACCTGGACCCCCAGT
CCGGCGAGGAGCTCAACCCAATCCCCCCGCCGCCGCAGCCCTATCAGCAGCCGC
GGGCCCTTGCTTCCCAGGATGGCACCCAAAAAGAAGCTGCAGCTGCCGCCGCC
GCCACCCACGGACGAGGAGGAATACTGGGACAGTCAGGCAGAGGAGGTTTTGG
ACGAGGAGGAGGAGATGATGGAAGACTGGGACAGCCTAGACGAAGCTTCCGAG
GCCGAAGAGGTGTCAGACGAAACACCGTCACCCTCGGTCGCATTCCCCTCGCCG
GCGCCCCAGAAATTGGCAACCGTTCCCAGCATCGCTACAACCTCCGCTCCTCAG
GCGCCGCCGGCACTGCCTGTTCGCCGACCGATCGCAGGTGATAGAGGTCTTTCT
TCCAACCAGTA
SEQ ID NO: 76: Fragment 10 
TAGAGGCAAGGGTTGTTTTTATTGACTACACCTGCGATCGACCCAACCG
TAGATGGGACACCACTGGAACCAGGGCCGGTAAGTCTAAGCAGCCGCCGT
TAGCCCAAGAGCAACAACAGCGCCAAGGCTACCGCTCGTGGCGCGGGCACAAG
AACGCCATAGTTGCTTGCTTGCAAGACTGTGGGGGCAACATCTCCTTCGCCCGC
CGCTTTCTTCTCTACCATCACGGCGTGGCCTTCCCCCGTAACATCCTGCATTACTA
CCGTCATCTCTACAGCCCCTACTGCACCGGCGGCAGCGGCAGCGGCAGCAACAG
CAGCGGTCACACAGAAGCAAAGGCGACCGGATAGCAAGACTCTGACAAAGCCC
AAGAAATCCACAGCGGCGGCAGCAGCAGGAGGAGGAGCGCTGCGTCTGGCGC
CCAACGAACCCGTATCGACCCGCGAGCTTAGAAATAGGATTTTTCCCACTCTGTA
TGCTATATTTCAACAAAGCAGGGGCCAAGAACAAGAGCTGAAAATAAAAAACA
 
GGTCTCTGCGCTCCCTCACCCGCAGCTGCCTGTATCACAAAAGCGAAGATCAGC
TTCGGCGCACGCTGGAAGACGCGGAGGCTCTCTTCAGCAAATACTGCGCGCTGA
CTCTTAAGGACTAGTTTCGCGCCCTTTCTCAAATTTAAGCGCGAAAACTACGTCA
TCTCCAGCGGCCACACCCGGCGCCAGCACCTGTCGTCAGCGCCATTATGAGCAA
GGAAATTCCCACGCCCTACATGTGGAGTTACCAGCCACAAATGGGACTTGCGGC
TGGAGCTGCCCAAGACTACTCAACCCGAATAAACTACGATCGCAGGTGATAGAG
GTCTTTCTTCCAACCAGTA
SEQ ID NO: 77: Fragment 11 
TAGAGGCAAGGGTTGTTTTTATTGACTAGAAGACGACTACATGAGCGC
GGGACCCCACATGATATCCCGGGTCAACGGAATCCGCGCCCACCGAAACCGAAT
TCTCCTCGAACAGGCGGCTATTACCACCACACCTCGTAATAACCTTAATCCCCGT
AGTTGGCCCGCTGCCCTGGTGTACCAGGAAAGTCCCGCTCCCACCACTGTGGTA
CTTCCCAGAGACGCCCAGGCCGAAGTTCAGATGACTAACTCAGGGGCGCAGCT
TGCGGGCGGCTTTCGTCACAGGGTGCGGTCGCCCGGGCGTTTTAGGGCGGAGTA
ACTTGCATGTATTGGGAATTGTAGTTTTTTTAAAATGGGAAGTGACGTATCGTGG
GAAAACGGAAGTGAAGATTTGAGGAAGTTGTGGGTTTTTTGGCTTTCGTTTCTG
GGCGTAGGTTCGCGTGCGGTTTTCTGGGTGTTTTTTGTGGACTTTAACCGTTACG
TCATTTTTTAGTCCTATATATACTCGCTCTGTACTTGGCCCTTTTTACACTGTGACT
GATTGAGCTGGTGCCGTGTCGAGTGGTGTTTTTTAATAGGTTTTTTTACTGGTAA
GGCTGACTGTTATGGCTGCCGCTGTGGAAGCGCTGTATGTTGTTCTGGAGCGGG
AGGGTGCTATTTTGCCTAGGCAGGAGGGTTTTTCAGGTGTTTATGTGTTTTTCTCT
CCTATTAATTTTGTTATACCTCCTATGGGGGCTGTAATGTTGTCTCTACGCCTGCG
GGTATGTATTCCCCCGGGCTATTTCGGTCGCTTTTTAGCACTGACCGATGTTAACC
AACCTGGAGTCTTCATAGAGGTCTTTCTTCCAACCAGTA
SEQ ID NO: 78: Fragment 12 
TAGAGGCAAGGGTTGTTTTTATTGACTAGAAGACGACCTGATGTGTTTA
CCGAGTCTTACATTATGACTCCGGACATGACCGAGGAACTGTCGGTGGTGCTTTT
 
TAATCACGGTGACCAGTTTTTTTACGGTCACGCCGGCATGGCCGTAGTCCGTCTT
ATGCTTATAAGGGTTGTTTTTCCTGTTGTAAGACAGGCTTCTAATGTTTAAATGTT
TTTTTTTTTGTTATTTTATTTTGTGTTTAATGCAGGAACCCGCAGACATGTTTGAG
AGAAAAATGGTGTCTTTTTCTGTGGTGGTTCCGGAACTTACCTGCCTTTATCTGC
ATGAGCATGACTACGATGTGCTTGCTTTTTTGCGCGAGGCTTTGCCTGATTTTTTG
AGCAGCACCTTGCATTTTATATCGCCGCCCATGCAACAAGCTTACATAGGGGCTA
CGCTGGTTAGCATAGCTCCGAGTATGCGTGTCATAATCAGTGTGGGTTCTTTTGTC
ATGGTTCCTGGCGGGGAAGTGGCCGCGCTGGTCCGTGCAGACCTGCACGATTAT
GTTCAGCTGGCCCTGCGAAGGGACCTACGGGATCGCGGTATTTTTGTTAATGTTC
CGCTTTTGAATCTTATACAGGTCTGTGAGGAACCTGAATTTTTGCAATCATGATTC
GCTGCTTGAGGCTGAAGGTGGAGGGCGCTCTGGAGCAGATTTTTACAATGGCCG
GACTTAATATTCGGGATTTGCTTAGAGACATATTGATAAGGTGGCGAGATGAAAA
TTATTTGGGCATGGTTGAAGGTGCTGGAATGTTTATAGAGGAGATTCACCCTGAA
GGGTTTAGCCTTTACGTCCGAGTCTTCATAGAGGTCTTTCTTCCAACCAGTA
SEQ ID NO: 79: Fragment 13 
TAGAGGCAAGGGTTGTTTTTATTGACTACACCTGCGATCGTCCACTTGG
ACGTGAGGGCAGTTTGCCTTTTGGAAGCCATTGTGCAACATCTTACAAATGCCAT
TATCTGTTCTTTGGCTGTAGAGTTTGACCACGCCACCGGAGGGGAGCGCGTTCA
CTTAATAGATCTTCATTTTGAGGTTTTGGATAATCTTTTGGAATAAAAAAAAAAA
AACATGGTTCTTCCAGCTCTTCCCGCTCCTCCCGTGTGTGACTCGCAGAACGAAT
GTGTAGGTTGGCTGGGTGTGGCTTATTCTGCGGTGGTGGATGTTATCAGGGCAGC
GGCGCATGAAGGAGTTTACATAGAACCCGAAGCCAGGGGGCGCCTGGATGCTTT
GAGAGAGTGGATATACTACAACTACTACACAGAGCGAGCTAAGCGACGAGACCG
GAGACGCAGATCTGTTTGTCACGCCCGCACCTGGTTTTGCTTCAGGAAATATGA
CTACGTCCGGCGTTCCATTTGGCATGACACTACGACCAACACGATCTCGGTTGTC
TCGGCGCACTCCGTACAGTAGGGATCGCCTACCTCCTTTTGAGACAGAGACCCG
CGCTACCATACTGGAGGATCATCCGCTGCTGCCCGAATGTAACACTTTGACAATG
 
CACAACGTGAGTTACGTGCGAGGTCTTCCCTGCAGTGTGGGATTTACGCTGATTC
AGGAATGGGTTGTTCCCTGGGATATGGTTCTGACGCGGGAGGAGCTTGTAATCCT
GAGGAAGTGTATGCACGTGTGCCTGTGTTGTGCCAACATTGATATCATGACGAGC
ATGATGATCCATGGTTACGGATCGCAGGTGATAGAGGTCTTTCTTCCAACCAGTA
SEQ ID NO: 80: Fragment 14 
TAGAGGCAAGGGTTGTTTTTATTGACTAGAAGACGATACGAGTCCTGG
GCTCTCCACTGTCATTGTTCCAGTCCCGGTTCCCTGCAGTGCATAGCCGGCGGGC
AGGTTTTGGCCAGCTGGTTTAGGATGGTGGTGGATGGCGCCATGTTTAATCAGAG
GTTTATATGGTACCGGGAGGTGGTGAATTACAACATGCCAAAAGAGGTAATGTTT
ATGTCCAGCGTGTTTATGAGGGGTCGCCACTTAATCTACCTGCGCTTGTGGTATG
ATGGCCACGTGGGTTCTGTGGTCCCCGCCATGAGCTTTGGATACAGCGCCTTGC
ACTGTGGGATTTTGAACAATATTGTGGTGCTGTGCTGCAGTTACTGTGCTGATTT
AAGTGAGATCAGGGTGCGCTGCTGTGCCCGGAGGACAAGGCGTCTCATGCTGC
GGGCGGTGCGAATCATCGCTGAGGAGACCACTGCCATGTTGTATTCCTGCAGGA
CGGAGCGGCGGCGGCAGCAGTTTATTCGCGCGCTGCTGCAGCACCACCGCCCTA
TCCTGATGCACGATTATGACTCTACCCCCATGTAGGCGTGGACTTCCCCTTCGCC
GCCCGTTGAGCAACCGCAAGTTGGACAGCAGCCTGTGGCTCAGCAGCTGGACA
GCGACATGAACTTAAGCGAGCTGCCCGGGGAGTTTATTAATATCACTGATGAGCG
TTTGGCTCGACAGGAAACCGTGTGGAATATAACACCTAAGAATATGTCTGTTACC
CATGATATGATGCTTTTTAAGGCCAGCCGGGGAGAAAGGACTGTGTACTCTGTGT
GTTGGGAGGGAGGTGGCAGGAGTCTTCATAGAGGTCTTTCTTCCAACCAGTA
SEQ ID NO: 81: Fragment 15 
TAGAGGCAAGGGTTGTTTTTATTGACTAGAAGACGAGCAGGTTGAATA

CTAGGGTTCTGTGAGTTTGATTAAGGTACGGTGATCAATATAAGCTATGTGGTGG

TGGGGCTATACTACTGAATGAAAAATGACTTGAAATTTTCTGCAATTGAAAAATA

AACACGTTGAAACATAACATGCAACAGGTTCACGATTCTTTATTCCTGGGCAATG

TAGGAGAAGGTGTAAGAGTTGGTAGCAAAAGTTTCAGTGGTGTATTTTCCACTT
 
TCCCAGGACCATGTAAAAGACATAGAGTAAGTGCTTACCTCGCTAGTTTCTGTGG
ATTCACTAGAATCGATGTAGGATGTTGCCCCTCCTGACGCGGTAGGAGAAGGGG
AGGGTGCCCTGCATGTCTGCCGCTGCTCTTGCTCTTGCCGCTGCTGAGGAGGGG
GGCGCATCTGCCGCAGCACCGGATGCATCTGGGAAAAGCAAAAAAGGGGCTCG
TCCCTGTTTCCGGAGGAATTTGCAAGCGGGGTCTTGCATGACGGGGAGGCAAAC
CCCCGTTCGCCGCAGTCCGGCCGGCCCGAGACTCGAACCGGGGGTCCTGCGAC
TCAACCCTTGGAAAATAACCCTCCGGCTACAGGGAGCGAGCCACTTAATGCTTT
CGCTTTCCAGCCTAACCGCTTACGCCGCGCGCGGCCAGTGGCCAAAAAAGCTAG
CGCAGCAGCCGCCGCGCCTGGAAGGAAGCCAAAAGGAGCGCTCCCCCGTTGTC
TGACGTCGCACACCTGGGTTCGACACGCGGGCGGTAACCGCATGGATCACGGC
GGACGGCCGGATCCGGGGTTCGAACGAGTCTTCATAGAGGTCTTTCTTCCAACC
AGTA
SEQ ID NO: 82: Fragment 16 
TAGAGGCAAGGGTTGTTTTTATTGACTAGAAGACGAGAACCCCGGTCG
TCCGCCATGATACCCTTGCGAATTTATCCACCAGACCACGGAAGAGTGCCCGCTT
ACAGGCTCTCCTTTTGCACGGTCTAGAGCGTCAACGACTGCGCACGCCTCACCG
GCCAGAGCGTCCCGACCATGGAGCACTTTTTGCCGCTGCGCAACATCTGGAACC
GCGTCCGCGACTTTCCGCGCGCCTCCACCACCGCCGCCGGCATCACCTGGATGT
CCAGGTACATCTACGGATTACGTCGACGTTTAAACCATATGAGCGGCCGCTCTAG
AACTAGTGGATCCCCCGGAAGATCAGAAGTTCCTATTCCGAAGTTCCTATTCTCT
AGAAAGTATAGGAACTTCTGATCTGCGCAGCCGCCATGCCGGGGTTTTACGAGA
TTGTGATTAAGGTCCCCAGCGACCTTGACGAGCATCTGCCCGGCATTTCTGACA
GCTTTGTGAACTGGGTGGCCGAGAAGGAATGGGAGTTGCCGCCAGATTCTGAC
ATGGATCTGAATCTGATTGAGCAGGCACCCCTGACCGTGGCCGAGAAGCTGCAG
CGCGACTTTCTGACGGAATGGCGCCGTGTGAGTAAGGCCCCGGAGGCCCTTTTC
TTTGTGCAATTTGAGAAGGGAGAGAGCTACTTCCACATGCACGTGCTCGTGGAA
ACCACCGGGGTGAAATCCATGGTTTTGGGACGTTTCCTGAGTCAGATTCGCGAA
 
AAACTGATTCAGAGAATTTACCGCGGGATCGAGCCGACTTTGCCAAACTGGTTC
GCGGTCACAAAGACCAGAAATGGCGCCGGAGGAGTCTTCATAGAGGTCTTTCTT
CCAACCAGTA
SEQ ID NO: 83: Fragment 17 
TAGAGGCAAGGGTTGTTTTTATTGACTACACCTGCGATCGGAGGCGGG
AACAAGGTGGTGGATGAGTGCTACATCCCCAATTACTTGCTCCCCAAAACCCAG
CCTGAGCTCCAGTGGGCGTGGACTAATATGGAACAGTATTTAAGCGCCTGTTTGA
ATCTCACGGAGCGTAAACGGTTGGTGGCGCAGCATCTGACGCACGTGTCGCAGA
CGCAGGAGCAGAACAAAGAGAATCAGAATCCCAATTCTGATGCGCCGGTGATCA
GATCAAAAACTTCAGCCAGGTACATGGAGCTGGTCGGGTGGCTCGTGGACAAG
GGGATTACCTCGGAGAAGCAGTGGATCCAGGAGGACCAGGCCTCATACATCTCC
TTCAATGCGGCCTCCAACTCGCGGTCCCAAATCAAGGCTGCCTTGGACAATGCG
GGAAAGATTATGAGCCTGACTAAAACCGCCCCCGACTACCTGGTGGGCCAGCAG
CCCGTGGAGGACATTTCCAGCAATCGGATTTATAAAATTTTGGAACTAAACGGGT
ACGATCCCCAATATGCGGCTTCCGTCTTTCTGGGATGGGCCACGAAAAAGTTCG
GCAAGAGGAACACCATCTGGCTGTTTGGGCCTGCAACTACCGGGAAGACCAAC
ATCGCGGAGGCCATAGCCCACACTGTGCCCTTCTACGGGTGCGTAAACTGGACC
AATGAGAACTTTCCCTTCAACGACTGTGTCGACAAGATGGTGATCTGGTGGGAG
GAGGGGAAGATGACCGCCAAGGTCGTGGAGTCGGCCAAAGCCATTCTCGGAGG
AAGCAAGGTGCGCGTGGACCAGAAATGCAAGTCCTCGATCGCAGGTGATAGAG
GTCTTTCTTCCAACCAGTA
SEQ ID NO: 84: Fragment 18 
TAGAGGCAAGGGTTGTTTTTATTGACTAGAAGACGACCTCGGCCCAGA
TAGACCCGACTCCCGTGATCGTCACCTCCAACACCAACATGTGCGCCGTGATTG
ACGGGAACTCAACGACCTTCGAACACCAGCAGCCGTTGCAAGACCGGATGTTC
AAATTTGAACTCACCCGCCGTCTGGATCATGACTTTGGGAAGGTCACCAAGCAG
GAAGTCAAAGACTTTTTCCGGTGGGCAAAGGATCACGTGGTTGAGGTGGAGCA
 
TGAATTCTACGTCAAAAAGGGTGGAGCCAAGAAAAGACCCGCCCCCAGTGACG
CAGATATAAGTGAGCCCAAACGGGTGCGCGAGTCAGTTGCGCAGCCATCGACGT
CAGACGCGGAAGCTTCGATCAACTACGCAGACAGGTACCAAAACAAATGTTCTC
GTCACGTGGGCATGAATCTGATGCTGTTTCCCTGCAGACAATGCGAGAGAATGA
ATCAGAATTCAAATATCTGCTTCACTCACGGACAGAAAGACTGTTTAGAGTGCTT
TCCCGTGTCAGAATCTCAACCCGTTTCTGTCGTCAAAAAGGCGTATCAGAAACT
GTGCTACATTCATCATATCATGGGAAAGGTGCCAGACGCTTGCACTGCCTGCGAT
CTGGTCAATGTGGATTTGGATGACTGCATCTTTGAACAATAAATGATTTAAATCA
GGTATGGCTGCCGATGGTTATCTTCCAGATTGGCTCGAGGACACTCTCTCTGAAG
GAATAAGACAGTGGTGGAAGCTCAAACCTGGCCCACCACCACCAAAGCCCGCA
GAGCGGCATAAGGACGACAGCGAGTCTTCATAGAGGTCTTTCTTCCAACCAGTA
SEQ ID NO: 85: Fragment 19 
TAGAGGCAAGGGTTGTTTTTATTGACTACACCTGCGATCCAGCAGGGG
TCTTGTGCTTCCTGGGTACAAGTACCTCGGACCCTTCAACGGACTCGACAAGGG
AGAGCCGGTCAACGAGGCAGACGCCGCGGCCCTCGAGCACGACAAAGCCTACG
ACCGGCAGCTCGACAGCGGAGACAACCCGTACCTCAAGTACAACCACGCCGAC
GCGGAGTTTCAGGAGCGCCTTAAAGAAGATACGTCTTTTGGGGGCAACCTCGGA
CGAGCAGTCTTCCAGGCGAAAAAGAGGGTTCTTGAACCTCTGGGCCTGGTTGA
GGAACCTGTTAAGACGGCTCCGGGAAAAAAGAGGCCGGTAGAGCACTCTCCTG
TGGAGCCAGACTCCTCCTCGGGAACCGGAAAGGCGGGCCAGCAGCCTGCAAGA
AAAAGATTGAATTTTGGTCAGACTGGAGACGCAGACTCAGTACCTGACCCCCAG
CCTCTCGGACAGCCACCAGCAGCCCCCTCTGGTCTGGGAACTAATACGATGGCT
ACAGGCAGTGGCGCACCAATGGCAGACAATAACGAGGGCGCCGACGGAGTGGG
TAATTCCTCGGGAAATTGGCATTGCGATTCCACATGGATGGGCGACAGAGTCATC
ACCACCAGCACCCGAACCTGGGCCCTGCCCACCTACAACAACCACCTCTACAAA
CAAATTTCCAGCCAATCAGGAGCCTCGAACGACAATCACTACTTTGGCTACAGC
ACCCCTTGGGGGTATTTTGACTTCAACAGATTCCACTGCCACTTTTCACCACGTG
 
ACTGGCAAAGACTCATCAACAACAACTGGGGATTCCGACGATCGCAGGTGATAG

AGGTCTTTCTTCCAACCAGTA
SEQ ID NO: 86: Fragment 20 
TAGAGGCAAGGGTTGTTTTTATTGACTACACCTGCGATCCGACCCAAG
AGACTCAACTTCAAGCTCTTTAACATTCAAGTCAAAGAGGTCACGCAGAATGAC
GGTACGACGACGATTGCCAATAACCTTACCAGCACGGTTCAGGTGTTTACTGACT
CGGAGTACCAGCTCCCGTACGTCCTCGGCTCGGCGCATCAAGGATGCCTCCCGC
CGTTCCCAGCAGACGTCTTCATGGTGCCACAGTATGGATACCTCACCCTGAACA
ACGGGAGTCAGGCAGTAGGACGCTCTTCATTTTACTGCCTGGAGTACTTTCCTTC
TCAGATGCTGCGTACCGGAAACAACTTTACCTTCAGCTACACTTTTGAGGACGTT
CCTTTCCACAGCAGCTACGCTCACAGCCAGAGTCTGGACCGTCTCATGAATCCT
CTCATCGACCAGTACCTGTATTACTTGAGCAGAACAAACACTCCAAGTGGAACC
ACCACGCAGTCAAGGCTTCAGTTTTCTCAGGCCGGAGCGAGTGACATTCGGGAC
CAGTCTAGGAACTGGCTTCCTGGACCCTGTTACCGCCAGCAGCGAGTATCAAAG
ACATCTGCGGATAACAACAACAGTGAATACTCGTGGACTGGAGCTACCAAGTAC
CACCTCAATGGCAGAGACTCTCTGGTGAATCCGGGCCCGGCCATGGCAAGCCAC
AAGGACGATGAAGAAAAGTTTTTTCCTCAGAGCGGGGTTCTCATCTTTGGGAAG
CAAGGCTCAGAGAAAACAAATGTGGACATTGAAAAGGTCATGATTACAGACGA
AGAGGAAATCAGGACAACCAATCCCGTGGGATCGCAGGTGATAGAGGTCTTTCT
TCCAACCAGTA
SEQ ID NO: 87: Fragment 21 
TAGAGGCAAGGGTTGTTTTTATTGACTAGAAGACGAGTGGCTACGGAG
CAGTATGGTTCTGTATCTACCAACCTCCAGAGAGGCAACAGACAAGCAGCTACC
GCAGATGTCAACACACAAGGCGTTCTTCCAGGCATGGTCTGGCAGGACAGAGAT
GTGTACCTTCAGGGGCCCATCTGGGCAAAGATTCCACACACGGACGGACATTTT
CACCCCTCTCCCCTCATGGGTGGATTCGGACTTAAACACCCTCCTCCACAGATTC
TCATCAAGAACACCCCGGTACCTGCGAATCCTTCGACCACCTTCAGTGCGGCAA
 
AGTTTGCTTCCTTCATCACACAGTACTCCACGGGACAGGTCAGCGTGGAGATCG
AGTGGGAGCTGCAGAAGGAAAACAGCAAACGCTGGAATCCCGAAATTCAGTAC
ACTTCCAACTACAACAAGTCTGTTAATGTGGACTTTACTGTGGACACTAATGGCG
TGTATTCAGAGCCTCGCCCCATTGGCACCAGATACCTGACTCGTAATCTGTAATT
GCTTGTTAATCAATAAACCGTTTAATTCGTTTCAGTTGAACTTTGGTCTCTGCGTA
TTTCTTTCTTATCTAGTTTCCATGGCTACTAAATGGCCCGCCTGGCTGACCGCCCA
ACGACCCCCGCCCATTGACGTCAATAATGACGTATGTTCCCATAGTAACGCCAAT
AGGGACTTTCCATTGACGTCAATGGGTGGAGTATTTACGGTAAACTGCCCACTTG
GCAGTACATCAAGTGTATCATATGCCAAGTACGCCCCCTATTGACGTCAATGACG
GTAAATGGCCCGCCTGGGAGTCTTCATAGAGGTCTTTCTTCCAACCAGTA
SEQ ID NO: 88: Fragment 22 
TAGAGGCAAGGGTTGTTTTTATTGACTAGAAGACGACTGGCATTATGCC
CAGTACATGACCTTATGGGACTTTCCTACTTGGCAGTACATCTACGTATTAGTCAT
CGCTATTACCATGGTGATGCGGTTTTGGCAGTACATCAATGGGCGTGGATAGCGG
TTTGACTCACGGGGATTTCCAAGTCTCCACCCCATTGACGTCAATGGGAGTTTGT
TTTGGCACCAAAATCAACGGGACTTTCCAAAATGTCGTAACAACTCCGCCCCAT
TGACGCAAATGGGCGGTAGGCGTGTACGGTGGGAGGTCTATATAAGCAGAGCTG
GTTTAGTGAACCGGATCCTGCCATTGCATCCTTCTCTGCAGACTAAGATGGAGTT
CCTGAACCAAGACCGCTTGCTGGCCAACCTGTGAAACTGGGCTCAAGGTGAGG
GGTGCTATCTGTGATTGAGGGACATGGTTAATGGAATTGTCTCACACAGAAATCG
CACCCGTCACCTTGGCCTACTTATCACCACCCCAAACAGAGGAACACGCCTTCT
CCAGCCACAGCCTATGGAAGGGCCTTCAGCTGCTGTGGCCCCGAGGTAAGCTTG
GGGGAGGCTAACTGAAACACGGAAGGAGACAATACCGGAAGGAACCCGCGCTA
TGACGGCAATAAAAAGACAGAATAAAACGCACGGTGTTGGGTCGTTGTAGATAA
GTAGCATGGCGGGTTAATCATTAACTACAGCCCGGGGTTTAAACAGCGGGCGGA
GGGGTGGAGTCGTGACGTGAATTACGTCATAGGGTTAGGGAGGTCCTGTATTAG
AGGTCACGTGAGTGTTTTGCGAGTCTTCATAGAGGTCTTTCTTCCAACCAGTA
SEQ ID NO: 89: Fragment 23 
TAGAGGCAAGGGTTGTTTTTATTGACTACACCTGCGATCTTGCGACATT
TTGCGACACCATGTGGTCTCGCTGGGGGGGGGGGCCCGAGTGAGCACGCAGGG
TCTCCATTTTGAAGCGGGAGGTTTGAACGAGCGCTGGCGCGCTCCGTCGACGAG
CTTTAATGCGGTAGTTTATCACAGTTAAATTGCTAACGCAGTCAGGCACCGTGTA
TGAAATCTAACAATGCGCTCATCGTCATCCTCGGCACCGTCACCCTGGATGCTGT
AGGCATAGGCTTGGTTATGCCGGTACTGCCGGGCCTCTTGCGGGATATCGTCCAT
TCCGACAGCATCGCCAGTCACTATGGCGTGCTGCTAGCGCTATATGCGTTGATGC
AATTTCTATGCGCACCCGTTCTCGGAGCACTGTCCGACCGCTTTGGCCGCCGCCC
AGTCCTGCTCGCTTCGCTACTTGGAGCCACTATCGACTACGCGATCATGGCGACC
ACACCCGTCCTGTGGATCCTCTACGCCGGACGCATCGTGGCCGGCATCACCGGC
GCCACAGGTGCGGTTGCTGGCGCCTATATCGCCGACATCACCGATGGGGAAGAT
CGGGCTCGCCACTTCGGGCTCATGAGCGCTTGTTTCGGCGTGGGTATGGTGGCA
GGCCCCGTGGCCGGGGGACTGTTGGGCGCCATCTCCTTGCATGCACCATTCCTT
GCGGCGGCGGTGCTCAACGGCCTCAACCTACTACTGGGCTGCTTCCTAATGCAG
GAGTCGCATAAGGGAGAGCGTCGACCGATGCCCTTGAGAGCCTTCAACCCAGTC
AGCTCCTTCCGGTGGGCGCGGGGCATGACGATCGCAGGTGATAGAGGTCTTTCT
TCCAACCAGTA
SEQ ID NO: 90: Fragment 24 
TAGAGGCAAGGGTTGTTTTTATTGACTAGAAGACGATGACTAGATCCAC
CTGCACGTAAAAGGCCTTCTTGGCCGCCCTTCCCGGTCGATATGAACAGCTTATT
TACATAATTCACGTTATTGGTAGTTATAAATGAAATTCCTAATATCGGTTATGAAGT
GAAATTGAATTTCTACTTGATCTTTCTCTCTATTTTTGTAAAATAAAATTAAGAATA
TTTAAATATTCAATGATTCATTTTTGCAGAAATCGGAGGAAGAAGAATATATGAA
AACATTTAACATTTCTCAACAAGATCCCCCCATATTGTTGTATAAGTGATGAAATA
CTGAATTTAAAACTTAGTTTATATGTGGTAAAATGTTTTAATCAAGTTTAGGAGGA
ATTAATTATGAAGTGTAATGAATAATGAATGTAACAGGGTTCAATTAAAAGAGGG
 
AAGCGTATCATTAACCCTATAAACTACGTCTGCCCTCATTATTGGAGGGTGAAATG
TGAATACATCCTATTCACAATCGAATTTACGACACAACCAAATTTTAATTTGGCTT
TGCATTTTATCTTTTTTTAGCGTATTAAATGAAATGGTTTTGAACGTCTCATTACCT
GATATTGCAAATGATTTTAATAAACCACCAGCGAGTACAAACTGGGTGAACACA
GCCTTTATGTTAACCTTTTCCATTGGAACAGCTGTATATGGAAAGCTATCTGATCA
ATTAGGCATCAAAAGGTTACTCCTATTTGGAATTATAATAAATTGTTTCGGGTCGG
TAATTGGGTTTGTTGGCCATTCTTTCTTTTCCTTACTTATTATGGCTCGAGTCTTCA
TAGAGGTCTTTCTTCCAACCAGTA
SEQ ID NO: 91: Fragment 25 
TAGAGGCAAGGGTTGTTTTTATTGACTACACCTGCGATCGCTCGTTTTAT
TCAAGGGGCTGGTGCAGCTGCATTTCCAGCACTCGTAATGGTTGTAGTTGCGCG
CTATATTCCAAAGGAAAATAGGGGTAAAGCATTTGGTCTTATTGGATCGATAGTA
GCCATGGGAGAAGGAGTCGGTCCAGCGATTGGTGGAATGATAGCCCATTATATTC
ATTGGTCCTATCTTCTACTCATTCCTATGATAACAATTATCACTGTTCCGTTTCTTAT
GAAATTATTAAAGAAAGAAGTAAGGATAAAAGGTCATTTTGATATCAAAGGAATT
ATACTAATGTCTGTAGGCATTGTATTTTTTATGTTGTTTACAACATCATATAGCATT
TCTTTTCTTATCGTTAGCGTGCTGTCATTCCTGATATTTGTAAAACATATCAGGAA
AGTAACAGATCCTTTTGTTGATCCCGGATTAGGGAAAAATATACCTTTTATGATTG
GAGTTCTTTGTGGGGGAATTATATTTGGAACAGTAGCAGGGTTTGTCTCTATGGT
TCCTTATATGATGAAAGATGTTCACCAGCTAAGTACTGCCGAAATCGGAAGTGTA
ATTATTTTCCCTGGAACAATGAGTGTCATTATTTTCGGCTACATTGGTGGGATACT
TGTTGATAGAAGAGGTCCTTTATACGTGTTAAACATCGGAGTTACATTTCTTTCTG
TTAGCTTTTTAACTGCTTCCTTTCTTTTAGAAACAACATCATGGTTCATGACAATT
ATAATCGTATTTGTTTTAGGTGGGCTTTCGTTCACCAAAACAGTTATATCAACAAT
TGTTTCAAGTAGCTTGATCGCAGGTGATAGAGGTCTTTCTTCCAACCAGTA
SEQ ID NO: 92: Fragment 26 TAGAGGCAAGGGTTGTTTTTATTGACTAGAAGACGAGCTTGAAACAGC 
AGGAAGCTGGTGCTGGAATGAGTTTGCTTAACTTTACCAGCTTTTTATCAGAGGG
AACAGGTATTGCAATTGTAGGTGGTTTATTATCCATACCCTTACTTGATCAAAGGT
TGTTACCTATGGAAGTTGATCAGTCAACTTATCTGTATAGTAATTTGTTATTACTTT
TTTCAGGAATCATTGTCATTAGTTGGCTGGTTACCTTGAATGTATATAAACATTCT
CAAAGGGATTTCTAAATCGTTAAGGGATCAACTTTGGGAGAGAGTTCAAAATTG
ATCCTTTTTTTATAACAGGAATTGGGCATCAAATAAAACGAAAGGCTCAGTCGAA
AGACTGGGCCTTTCGTTTTATCTGTTGTTTGTCGGTGAACGCTCTCCTGAGTAGG
ACAAATCCGCCGGGAGCGGATTTGAACGTTGCGAAGCAACGGCCCGGAGGGTG
GCGGGCAGGACGCCCGCCATAAACTGCCAGGCATCAAATTAAGCAGAAGGCCAT
CCTGACGGATGGCCTTTTTGCGTTTCTACAAACTCTTCCTGTCGTCATATCTACAA
TTCTACACAGCCCAGTCCAGACTATTGAATTGTATCACGTTTTTGATATCCTACCA
ATAACAAATTGATTGGAGGAATGCAAAGTGAATAATGAACCAGTAAAACGTGGT
AAGAAGAACAGATGGGAATTAAACCTACCTATAATGACTTATGTAGTAGCTGATG
ATTGGATTGATAAACTAGGACACGAAACGTTTACTTTATGGTTGAGGTTCCATAC
TTGGGTAGAGTCTTCATAGAGGTCTTTCTTCCAACCAGTA
SEQ ID NO: 93: Fragment 27 
TAGAGGCAAGGGTTGTTTTTATTGACTACACCTGCGATCGGTAGATAGA
GAAGATGAACTCCGAGATTATGATCGCATACCTAGAAGTTTTGAGAACATATATA
AAAAGACACTAGGAATCTCAAAAAGTAAGTTTTATAGATTGATAAAACCTTTATG
GGAATATGGATTAATAGACATCATAGAATACGAAGAATCTAACCGTAATTCTACTA
AACCTAAAAATATAATTGTTTATGAGTATCCTTTACACGAAATAGAAAGAAAGTAT
AAACCACTAGAAAAATTAAGAGATTGGGATAAAGACTATAATTCCGTTTCTAAAG
AATTAGGTAAAACAGGTGGTAGACCAAGGAAAAAAGATAGTGAAGAAGAACCC
GAAAAGAAACCCGAAGAAGTAACTAAAAAGAAACGTAAATATAAGTTAAAAAG
AGTTATCCACAACGGTTTCAAAAATGAAACGGTGGAGGGTTTCAAAAATGAAAC
GGTGGAGGGTTTCAAAAATGAAACGGTGACCGTTTCAAAAATAAAACCCAATAA
TTATTCAAATATCTTTAATAACTTATCAAATATTTCTACTAATGTTTCAAATAATTTA
 
TTAATTGATGATGATGAGGAAATCGAAAATGAACCAACTGGTCGTACAATAAATA
GGTCATTACTTTTTTCACAAGAAGATATTAAACAGGCCTATCAATTTATTAATAGA
TTTTCAGTTATACAGTTACGTGAAAACTTTAGCTTTGATAAACACTTTGAAGAAC
GGTTGGTATGTTATTTATGGAAAGCAGGGATTTCTACTTTTTACACGCACGAAATC
AGTAAAATGATAAAAAAAATAGCGATCGCAGGTGATAGAGGTCTTTCTTCCAAC
CAGTA
SEQ ID NO: 94: Fragment 28 
TAGAGGCAAGGGTTGTTTTTATTGACTAGAAGACGATAGCAGACTATGA
AAAATCTAAAAAAGGTAGATTAAACCCAATACGTGACCGAGCCTTATATATGGTA
AATGGTCTTGTAATGAATAGAGCTTCTTCCCAAAGTGAACATGCTACTTATAAAC
TAAACCAATATAAAAAACAGAAGGAACAGGAAAAACAACAACAGGAGCAACA
AAGATCAAGAGTACCGTTCTATAATTGGTTGGAGGAAAGAGAAGAACAAACCG
AAGGTCAACTACCCACCACTTAAGCGGCCGCAAGCTTGAAGAGCTCTTCTTTCA
GAACGCTCGGTTGCCGCCGGGCGTTTTTTATGAGACGTCTCGGCCTGTTTGGCC
ATTATACTGGCTTAACTATGCGGCATCAGAGCAGATTGTACTGAGAGTGCACCAT
ATGCGGTGTGAAATACCGCACAGATGCGTAAGGAGAAAATACCGCATCAGGCGC
TCTTCCGCTTCCTCGCTCACTGACTCGCTGCGCTCGGTCGTTCGGCTGCGGCGA
GCGGTATCAGCTCACTCAAAGGCGGTAATACGGTTATCCACAGAATCAGGGGAT
AACGCAGGAAAGAACATGTGAGCAAAAGGCCAGCAAAAGGCCAGGAACCGTA
AAAAGGCCGCGTTGCTGGCGTTTTTCCATAGGCTCCGCCCCCCTGACGAGCATC
ACAAAAATCGACGCTCAAGTCAGAGGTGGCGAAACCCGACAGGACTATAAAGA
TACCAGGCGTTTCCCCCTGGAAGCTCCCTCGTGCGCTCTCCTGTTCCGACCCTGC
CGCTTACCGGATACCTGTCCGCCTTTCTCGAGTCTTCATAGAGGTCTTTCTTCCA
ACCAGTA
SEQ ID NO: 95: Fragment 29 
TAGAGGCAAGGGTTGTTTTTATTGACTACACCTGCGATCTCTCCCTTCG
GGAAGCGTGGCGCTTTCTCATAGCTCACGCTGTAGGTATCTCAGTTCGGTGTAGG
 
TCGTTCGCTCCAAGCTGGGCTGTGTGCACGAACCCCCCGTTCAGCCCGACCGCT
GCGCCTTATCCGGTAACTATCGTCTTGAGTCCAACCCGGTAAGACACGACTTATC
GCCACTGGCAGCAGCCACTGGTAACAGGATTAGCAGAGCGAGGTATGTAGGCG
GTGCTACAGAGTTCTTGAAGTGGTGGCCTAACTACGGCTACACTAGAAGGACAG
TATTTGGTATCTGCGCTCTGCTGAAGCCAGTTACCTTCGGAAAAAGAGTTGGTAG
CTCTTGATCCGGCAAACAAACCACCGCTGGTAGCGGTGGTTTTTTTGTTTGCAA
GCAGCAGATTACGCGCAGAAAAAAAGGATCTCAAGAAGATCCTTTGATCTTTTC
TACGGGGTCTGACGCTCAGTGGAACGAAAACTCACGTTAAGGGATTTTGGTCAT
GAGATTATCAAAAAGGATCTTCACCTAGATCCTTTTAAATTAAAAATGAAGTTTT
AAATCAATCTAAAGTATATATGAGTAAACTTGGTCTGACAGTTACCAATGCTTAAT
CAGTGAGGCACCTATCTCAGCGATCTGTCTATTTCGTTCATCCATAGTTGCCTGAC
TCCCCGTCGTGTAGATAACTACGATACGGGAGGGCTTACCATCTGGCCCCAGTGC
TGCAATGATACCGCGAGACCCACGCTCACCGGCTCCAGATTTATCAGCAATAAAC
CAGCCAGCCGGAAGGGCCGAGGATCGCAGGTGATAGAGGTCTTTCTTCCAACC
AGTA
SEQ ID NO: 96: Fragment 30 
TAGAGGCAAGGGTTGTTTTTATTGACTAGAAGACGACGAGCGCAGAAG
TGGTCCTGCAACTTTATCCGCCTCCATCCAGTCTATTAATTGTTGCCGGGAAGCTA
GAGTAAGTAGTTCGCCAGTTAATAGTTTGCGCAACGTTGTTGCCATTGCTGCAGG
CATCGTGGTGTCACGCTCGTCGTTTGGTATGGCTTCATTCAGCTCCGGTTCCCAA
CGATCAAGGCGAGTTACATGATCCCCCATGTTGTGCAAAAAAGCGGTTAGCTCC
TTCGGTCCTCCGATCGTTGTCAGAAGTAAGTTGGCCGCAGTGTTATCACTCATGG
TTATGGCAGCACTGCATAATTCTCTTACTGTCATGCCATCCGTAAGATGCTTTTCT
GTGACTGGTGAGTACTCAACCAAGTCATTCTGAGAATAGTGTATGCGGCGACCG
AGTTGCTCTTGCCCGGCGTCAACACGGGATAATACCGCGCCACATAGCAGAACT
TTAAAAGTGCTCATCATTGGAAAACGTTCTTCGGGGCGAAAACTCTCAAGGATC
TTACCGCTGTTGAGATCCAGTTCGATGTAACCCACTCGTGCACCCAACTGATCTT
 
CAGCATCTTTTACTTTCACCAGCGTTTCTGGGTGAGCAAAAACAGGAAGGCAAA
ATGCCGCAAAAAAGGGAATAAGGGCGACACGGAAATGTTGAATACTCATACTCT
TCCTTTTTCAATATTATTGAAGCATTTATCAGGGTTATTGTCTCATGAGCGGATACA
TATTTGAATGTATTTAGAAAAATAAACAAATAGGGGTTCCGCGGCGATCGCCACA
TTTCCCCGAAAAGAGTCTTCATAGAGGTCTTTCTTCCAACCAGTA
SEQ ID NO: 97: Fragment 2-Construct primer 1 
TAGAGGCAAGGGTTGTTTTTATTGACTAGAAGACGAGACGCAAATGGG
CGGTAGGCGTGTACGGTGGGAGGTCTATATAAGCAGAGCTGGTTTAGTGGATATC
GTATACGCCACCATGGTGAGCAAGGGCGAGGAGCTGTTCACCGGGGTGGTGCCC
ATCCTGGTCGA
SEQ ID NO: 98: Fragment 2-Construct primer 2 
TGGTCACGAGGGTGGGCCAGGGCACGGGCAGCTTGCCGGTGGTGCAG
ATGAACTTCAGGGTCAGCTTGCCGTAGGTGGCATCGCCCTCGCCCTCGCCGGAC
ACGCTGAACTTGTGGCCGTTTACGTCGCCGTCCAGCTCGACCAGGATGGGCACC
ACCCCGGTGA
SEQ ID NO: 99: Fragment 2-Construct primer 3 
CCCGTGCCCTGGCCCACCCTCGTGACCACCCTGACCTACGGCGTGCAG
TGCTTCAGCCGCTACCCCGACCACATGAAGCAGCACGACTTCTTCAAGTCCGCC
ATGCCCGAAGGCTACGTCCAGGAGCGCACCATCTTCTTCAAGGACGACGGCAAC
TACAAGACCCGC
SEQ ID NO: 100: Fragment 2-Construct primer 4 
TTGTCGGCCATGATATAGACGTTGTGGCTGTTGTAGTTGTACTCCAGCTT
GTGCCCCAGGATGTTGCCGTCCTCCTTGAAGTCGATGCCCTTCAGCTCGATGCGG
TTCACCAGGGTGTCGCCCTCGAACTTCACCTCGGCGCGGGTCTTGTAGTTGCCG
TCGTCCTTG
SEQ ID NO: 101: Fragment 2-Construct primer 5 GCCACAACGTCTATATCATGGCCGACAAGCAGAAGAACGGCATCAAGG 
TGAACTTCAAGATCCGCCACAACATCGAGGACGGCAGCGTGCAGCTCGCCGAC

CACTACCAGCAGAACACCCCCATCGGCGACGGCCCCGTGCTGCTGCCCGACAA

CCACTACCTGAGCA
SEQ ID NO: 102: Fragment 2-Construct primer 6 
TACTGGTTGGAAGAAAGACCTCTATGAAGACTCCTTGTACAGCTCGTC
CATGCCGAGAGTGATCCCGGCGGCGGTCACGAACTCCAGCAGGACCATGTGATC
GCGCTTCTCGTTGGGGTCTTTGCTCAGGGCGGACTGGGTGCTCAGGTAGTGGTT
GTCGGGCAGCAG
SEQ ID NO: 103: Fragment 3-Construct primer 1 
TAGAGGCAAGGGTTGTTTTTATTGACTACACCTGCGATCCAAGTAAGTA
TACCGATAATCAACCTCTGGATTACAAAATTTGTGAAAGATTGACTGGTATTCTTA
ACTATGTTGCTCCTTTTACGCTATGTGGATACGCTGCTTTAATGCCTTTGTATCATG
CTATTGCTT
SEQ ID NO: 104: Fragment 3-Construct primer 2 
TGGCAATGCCCCAACCAGTGGGGGTTGCGTCAGCAAACACAGTGCAC
ACCACGCCACGTTGCCTGACAACGGGCCACAACTCCTCATAAAGAGACAGCAA
CCAGGATTTATACAAGGAGGAGAAAATGAAAGCCATACGGGAAGCAATAGCATG
ATACAAAGGCATTAAAG
SEQ ID NO: 105: Fragment 3-Construct primer 3 
ACGCAACCCCCACTGGTTGGGGCATTGCCACCACCTGTCAGCTCCTTT
CCGGGACTTTCGCTTTCCCCCTCCCTATTGCCACGGCGGAACTCATCGCCGCCTG
CCTTGCCCGCTGCTGGACAGGGGCTCGGCTGTTGGGCACTGACAATTCCGTGGT
GTTGTCGGGG
SEQ ID NO: 106: Fragment 3-Construct primer 4 
CGGAAGAGGCCGCAGAGCCGGCAGCAGGCCGCGGGAAGGAAGGTCC
GCTGGATTGAGGGCCGAAGGGACGTAGCAGAAGGACGTCCCGCGCAGAATCCA
GGTGGCAACACAGGCGAGCAGCCATGGAAAGGACGTCAGCTTCCCCGACAACA
 CCACGGAATTGTCAGTGCC
SEQ ID NO: 107: Fragment 3-Construct primer 5 
GGCCTGCTGCCGGCTCTGCGGCCTCTTCCGCGTCTTCGCCTTCGCCCTC
AGACGAGTCGGATCTCCCTTTGGGCCGCCTCCCCGCATCGGTTAATTAAACGGGT
GGCATCCCTGTGACCCCTCCCCAGTGCCTCTCCTGGCCCTGGAAGTTGCCACTC
CAGTGCCCACCAG
SEQ ID NO: 108: Fragment 3-Construct primer 6 
TACTGGTTGGAAGAAAGACCTCTATCACCTGCGATCTTCCCAACTTGCC
CCTTGCTCCATACCACCCCCCTCCACCCCATAATATTATAGAAGGACACCTAGTCA
GACAAAATGATGCAACTTAATTTTATTAGGACAAGGCTGGTGGGCACTGGAGTG
GCAACTTCCAGG
SEQ ID NO: 109: Fragment 4-Construct primer 1 
TAGAGGCAAGGGTTGTTTTTATTGACTACACCTGCGATCGGAAGACAA
CCTGTAGGGCCTGCGGGGTCTATTGGGAACCAAGCTGGAGTGCAGTGGCACAAT
CTTGGCTCACTGCAATCTCCGCCTCCTGGGTTCAAGCGATTCTCCTGCCTCAGCC
TCCCGAGTTGTTGG
SEQ ID NO: 110: Fragment 4-Construct primer 2 
GTAATCCCAGCAATTTGGGAGGCCAAGGTGGGTAGATCACCTGAGATT
AGGAGTTGGAGACCAGCCTGGCCAATATGGTGAAACCCCGTCTCTACCAAAAAA
ACAAAAATTAGCTGAGCCTGGTCATGCATGCCTGGAATCCCAACAACTCGGGAG
GCTGAGGCAGGAGAA
SEQ ID NO: 111: Fragment 4-Construct primer 3 
CACCTTGGCCTCCCAAATTGCTGGGATTACAGGCGTGAACCACTGCTC
CCTTCCCTGTCCTTATCGATAGATCTAGGAACCCCTAGTGATGGAGTTGGCCACT
CCCTCTCTGCGCGCTCGCTCGCTCACTGAGGCCGGGCGACCAAAGGTCGCCCGA
CGCCCGGGCTTT
SEQ ID NO: 112: Fragment 4-Construct primer 4 
CCAGGAAGCTGTAGAGCTGTTCCTGGTTGCGACGCAGGGTGGGCTGTA
CCTGGGGACTGTTAAGCATGGAGTTGGGTACCCCTCGAGGGGATCTGCCTGCAG
GCAGCTGCGCGCTCGCTCGCTCACTGAGGCCGCCCGGGCAAAGCCCGGGCGTC
GGGCGACCTTTGGTCG
SEQ ID NO: 113: Fragment 4-Construct primer 5 
GCAACCAGGAACAGCTCTACAGCTTCCTGGAGCGCCACTCGCCCTACT
TCCGCAGCCACAGTGCGCAGATTAGGAGCGCCACTTCTTTTTGTCACTTGAAAA
ACATGTAAAAATAATGTACTAGGAGACACTTTCAATAAAGGCAAATGTTTTTATTT
GTACACTCTCGGG
SEQ ID NO: 114: Fragment 4-Construct primer 6 
TACTGGTTGGAAGAAAGACCTCTATCACCTGCGATCCACCAGTATCGCA
ACGTGTCCCTGCCAGTGGCGCATAGCGATGCGCGGCAGAACCCCTTTGATTTTTA
AACGGCGCAGACGGCAAGGGTGGGGGGTAAATAATCACCCGAGAGTGTACAAA
TAAAAACATTTGCC
SEQ ID NO: 115: Fragment 22-Construct primer 1 
TAGAGGCAAGGGTTGTTTTTATTGACTAGAAGACGACTGGCATTATGCC
CAGTACATGACCTTATGGGACTTTCCTACTTGGCAGTACATCTACGTATTAGTCAT
CGCTATTACCATGGTGATGCGGTTTTGGCAGTACATCAATGGGCGTGGATAGCGG
TTTGACTCA
SEQ ID NO: 116: Fragment 22-Construct primer 2 
AGACCTCCCACCGTACACGCCTACCGCCCATTTGCGTCAATGGGGCGG
AGTTGTTACGACATTTTGGAAAGTCCCGTTGATTTTGGTGCCAAAACAAACTCC
CATTGACGTCAATGGGGTGGAGACTTGGAAATCCCCGTGAGTCAAACCGCTATC
CACGCCCATTGAT
SEQ ID NO: 117: Fragment 22-Construct primer 3 
TGGGCGGTAGGCGTGTACGGTGGGAGGTCTATATAAGCAGAGCTGGTT
TAGTGAACCGGATCCTGCCATTGCATCCTTCTCTGCAGACTAAGATGGAGTTCCT
 
GAACCAAGACCGCTTGCTGGCCAACCTGTGAAACTGGGCTCAAGGTGAGGGGT
GCTATCTGTGA
SEQ ID NO: 118: Fragment 22-Construct primer 4 
TCGGGGCCACAGCAGCTGAAGGCCCTTCCATAGGCTGTGGCTGGAGA
AGGCGTGTTCCTCTGTTTGGGGTGGTGATAAGTAGGCCAAGGTGACGGGTGCGA
TTTCTGTGTGAGACAATTCCATTAACCATGTCCCTCAATCACAGATAGCACCCCT
CACCTTGAGCCCA
SEQ ID NO: 119: Fragment 22-Construct primer 5 
TGGAAGGGCCTTCAGCTGCTGTGGCCCCGAGGTAAGCTTGGGGGAGG
CTAACTGAAACACGGAAGGAGACAATACCGGAAGGAACCCGCGCTATGACGGC
AATAAAAAGACAGAATAAAACGCACGGTGTTGGGTCGTTGTAGATAAGTAGCAT
GGCGGGTTAATCATT
SEQ ID NO: 120: Fragment 22-Construct primer 6 
TACTGGTTGGAAGAAAGACCTCTATGAAGACTCGCAAAACACTCACGT
GACCTCTAATACAGGACCTCCCTAACCCTATGACGTAATTCACGTCACGACTCCA
CCCCTCCGCCCGCTGTTTAAACCCCGGGCTGTAGTTAATGATTAACCCGCCATGC
TACTTATCTAC
SEQ ID NO: 121: Fragment 23-Construct primer 1 
TAGAGGCAAGGGTTGTTTTTATTGACTACACCTGCGATCTTGCGACATT
TTGCGACACCATGTGGTCTCGCTGGGGGGGGGGGCCCGAGTGAGCACGCAGGG
TCTCCATTTTGAAGCGGGAGGTTTGAACGAGCGCTGGCGCGCTCCGTCGACGAG
CTTTAATGCGGTAGT
SEQ ID NO: 122: Fragment 23-Construct primer 2 
ATCCCGCAAGAGGCCCGGCAGTACCGGCATAACCAAGCCTATGCCTAC
AGCATCCAGGGTGACGGTGCCGAGGATGACGATGAGCGCATTGTTAGATTTCAT
ACACGGTGCCTGACTGCGTTAGCAATTTAACTGTGATAAACTACCGCATTAAAGC
TCGTCGACGGAGCG
SEQ ID NO: 123: Fragment 23-Construct primer 3 
ATGCCGGTACTGCCGGGCCTCTTGCGGGATATCGTCCATTCCGACAGCA
TCGCCAGTCACTATGGCGTGCTGCTAGCGCTATATGCGTTGATGCAATTTCTATGC
GCACCCGTTCTCGGAGCACTGTCCGACCGCTTTGGCCGCCGCCCAGTCCTGCTC
GCTTCGCTACT
SEQ ID NO: 124: Fragment 23-Construct primer 4 
TCTTCCCCATCGGTGATGTCGGCGATATAGGCGCCAGCAACCGCACCTG
TGGCGCCGGTGATGCCGGCCACGATGCGTCCGGCGTAGAGGATCCACAGGACG
GGTGTGGTCGCCATGATCGCGTAGTCGATAGTGGCTCCAAGTAGCGAAGCGAGC
AGGACTGGGCGGCGG
SEQ ID NO: 125: Fragment 23-Construct primer 5 
CTATATCGCCGACATCACCGATGGGGAAGATCGGGCTCGCCACTTCGGG
CTCATGAGCGCTTGTTTCGGCGTGGGTATGGTGGCAGGCCCCGTGGCCGGGGGA
CTGTTGGGCGCCATCTCCTTGCATGCACCATTCCTTGCGGCGGCGGTGCTCAACG
GCCTCAACCTACT
SEQ ID NO: 126: Fragment 23-Construct primer 6 
TACTGGTTGGAAGAAAGACCTCTATCACCTGCGATCGTCATGCCCCGCG
CCCACCGGAAGGAGCTGACTGGGTTGAAGGCTCTCAAGGGCATCGGTCGACGC
TCTCCCTTATGCGACTCCTGCATTAGGAAGCAGCCCAGTAGTAGGTTGAGGCCGT
TGAGCACCGCCGCC
SEQ ID NO: 127: Fragment 28-Construct primer 1 
TAGAGGCAAGGGTTGTTTTTATTGACTAGAAGACGATAGCAGACTATGA
AAAATCTAAAAAAGGTAGATTAAACCCAATACGTGACCGAGCCTTATATATGGTA
AATGGTCTTGTAATGAATAGAGCTTCTTCCCAAAGTGAACATGCTACTTATAAAC
TAAACCAATAT
SEQ ID NO: 128: Fragment 28-Construct primer 2 GAGCTCTTCAAGCTTGCGGCCGCTTAAGTGGTGGGTAGTTGACCTTCG 
GTTTGTTCTTCTCTTTCCTCCAACCAATTATAGAACGGTACTCTTGATCTTTGTTG
CTCCTGTTGTTGTTTTTCCTGTTCCTTCTGTTTTTTATATTGGTTTAGTTTATAAGTA
GCATGTTC
SEQ ID NO: 129: Fragment 28-Construct primer 3 
CACTTAAGCGGCCGCAAGCTTGAAGAGCTCTTCTTTCAGAACGCTCGG
TTGCCGCCGGGCGTTTTTTATGAGACGTCTCGGCCTGTTTGGCCATTATACTGGC
TTAACTATGCGGCATCAGAGCAGATTGTACTGAGAGTGCACCATATGCGGTGTGA
AATACCG
SEQ ID NO: 130: Fragment 28-Construct primer 4 
CCTGATTCTGTGGATAACCGTATTACCGCCTTTGAGTGAGCTGATACCG
CTCGCCGCAGCCGAACGACCGAGCGCAGCGAGTCAGTGAGCGAGGAAGCGGA
AGAGCGCCTGATGCGGTATTTTCTCCTTACGCATCTGTGCGGTATTTCACACCGC
ATATGGTGCACTCT
SEQ ID NO: 131: Fragment 28-Construct primer 5 
GGCGGTAATACGGTTATCCACAGAATCAGGGGATAACGCAGGAAAGAA
CATGTGAGCAAAAGGCCAGCAAAAGGCCAGGAACCGTAAAAAGGCCGCGTTGC
TGGCGTTTTTCCATAGGCTCCGCCCCCCTGACGAGCATCACAAAAATCGACGCT
CAAGTCAGAGGTGGC
SEQ ID NO: 132: Fragment 28-Construct primer 6 
TACTGGTTGGAAGAAAGACCTCTATGAAGACTCGAGAAAGGCGGACA
GGTATCCGGTAAGCGGCAGGGTCGGAACAGGAGAGCGCACGAGGGAGCTTCCA
GGGGGAAACGCCTGGTATCTTTATAGTCCTGTCGGGTTTCGCCACCTCTGACTTG
AGCGTCGATTTTTGT
SEQ ID NO: 133: Fragment 30-Construct primer 1 
TAGAGGCAAGGGTTGTTTTTATTGACTAGAAGACGACGAGCGCAGAAG
TGGTCCTGCAACTTTATCCGCCTCCATCCAGTCTATTAATTGTTGCCGGGAAGCTA
GAGTAAGTAGTTCGCCAGTTAATAGTTTGCGCAACGTTGTTGCCATTGCTGCAGG
 CATCGT
SEQ ID NO: 134: Fragment 30-Construct primer 2 
CGGCCAACTTACTTCTGACAACGATCGGAGGACCGAAGGAGCTAACCG
CTTTTTTGCACAACATGGGGGATCATGTAACTCGCCTTGATCGTTGGGAACCGGA
GCTGAATGAAGCCATACCAAACGACGAGCGTGACACCACGATGCCTGCAGCAAT
GGCAACAACGT
SEQ ID NO: 135: Fragment 30-Construct primer 3 
CCGATCGTTGTCAGAAGTAAGTTGGCCGCAGTGTTATCACTCATGGTTA
TGGCAGCACTGCATAATTCTCTTACTGTCATGCCATCCGTAAGATGCTTTTCTGTG
ACTGGTGAGTACTCAACCAAGTCATTCTGAGAATAGTGTATGCGGCGACCGAGT
TGCTCT
SEQ ID NO: 136: Fragment 30-Construct primer 4 
GCACGAGTGGGTTACATCGAACTGGATCTCAACAGCGGTAAGATCCTT
GAGAGTTTTCGCCCCGAAGAACGTTTTCCAATGATGAGCACTTTTAAAGTTCTG
CTATGTGGCGCGGTATTATCCCGTGTTGACGCCGGGCAAGAGCAACTCGGTCGC
CGCATACACTAT
SEQ ID NO: 137: Fragment 30-Construct primer 5 
GATCCAGTTCGATGTAACCCACTCGTGCACCCAACTGATCTTCAGCATC
TTTTACTTTCACCAGCGTTTCTGGGTGAGCAAAAACAGGAAGGCAAAATGCCGC
AAAAAAGGGAATAAGGGCGACACGGAAATGTTGAATACTCATACTCTTCCTTTT
TCAATATTAT
SEQ ID NO: 138: Fragment 30-Construct primer 6 
TACTGGTTGGAAGAAAGACCTCTATGAAGACTCTTTTCGGGGAAATGT
GGCGATCGCCGCGGAACCCCTATTTGTTTATTTTTCTAAATACATTCAAATATGTAT
CCGCTCATGAGACAATAACCCTGATAAATGCTTCAATAATATTGAAAAAGGAAGA
GTATGAGT
SEQ ID NO: 139: Fragment 1-F
   TAGCACCTGCGATCAAAAGTGCCACCTGACGTCTAA
SEQ ID NO: 140: Fragment 5-F
   TAGGAAGACGAGGTGTTTAGTGCTCCACTTAAACT
SEQ ID NO: 141: Fragment 6-F
   TAGCACCTGCGATCCATCATCGTCACAAAGGTCTTGTT
SEQ ID NO: 142: Fragment 7-F
   TAGCACCTGCGATCCCTAACCGCCCCCTTT
SEQ ID NO: 143: Fragment 8-F
   TAGGAAGACGACCAAGGTTATGAGCACAGTCAT
SEQ ID NO: 144: Fragment 9-F
   TAGCACCTGCGATCCTTCTGCAGCTAGCCAACTA
SEQ ID NO: 145: Fragment 10-F
   TAGCACCTGCGATCGACCCAACCGTAGATGG
SEQ ID NO: 146: Fragment 11-F
   TAGGAAGACGACTACATGAGCGCGGGA
SEQ ID NO: 147: Fragment 12-F
   TAGGAAGACGACCTGATGTGTTTACCGAGTCTTA
SEQ ID NO: 148: Fragment 13-F
   TAGCACCTGCGATCGTCCACTTGGACGTGAG
SEQ ID NO: 149: Fragment 14-F
   TAGGAAGACGATACGAGTCCTGGGCTCT
SEQ ID NO: 150: Fragment 15-F
   TAGGAAGACGAGCAGGTTGAATACTAGGGTTCT
SEQ ID NO: 151: Fragment 16-F
   TAGGAAGACGAGAACCCCGGTCGTC
SEQ ID NO: 152: Fragment 17-F
   TAGCACCTGCGATCGGAGGCGGGAACAAG
SEQ ID NO: 153: Fragment 18-F
   TAGGAAGACGACCTCGGCCCAGATAGA
SEQ ID NO: 154: Fragment 19-F
   TAGCACCTGCGATCCAGCAGGGGTCTTGTG
SEQ ID NO: 155: Fragment 20-F
   TAGCACCTGCGATCCGACCCAAGAGACTCAACTT
SEQ ID NO: 156: Fragment 21-F
   TAGGAAGACGAGTGGCTACGGAGCAGTAT
SEQ ID NO: 157: Fragment 24-F
   TAGGAAGACGATGACTAGATCCACCTGCAC
SEQ ID NO: 158: Fragment 25-F
   TAGCACCTGCGATCGCTCGTTTTATTCAAGGGGCT
SEQ ID NO: 159: Fragment 26-F
   TAGGAAGACGAGCTTGAAACAGCAGGAAGCT
SEQ ID NO: 160: Fragment 27-F
   TAGCACCTGCGATCGGTAGATAGAGAAGATGAACTCC
SEQ ID NO: 161: Fragment 29-F
   TAGCACCTGCGATCTCTCCCTTCGGGAAGC
   SEQ ID NO: 162: Fragment 1-R
   TAGCACCTGCGATCCGTCAATGGGGCGGA
SEQ ID NO: 163: Fragment 5-R
   TAGGAAGACTCGATGGGGCGATTCCTG
SEQ ID NO: 164: Fragment 6-R
   TAGCACCTGCGATCTAGGCTGTCCTCCTTCTC
SEQ ID NO: 165: Fragment 7-R
   TAGCACCTGCGATCTTGGGGGGTAGGTTAAGTG
SEQ ID NO: 166: Fragment 8-R
   TAGGAAGACTCGAAGGTAGCAGTGACCC
SEQ ID NO: 167: Fragment 9-R
   TAGCACCTGCGATCGGTCGGCGAACAGG
SEQ ID NO: 168: Fragment 10-R
   TAGCACCTGCGATCGTAGTTTATTCGGGTTGAGTAGTCTT
SEQ ID NO: 169: Fragment 11-R
   TAGGAAGACTCCAGGTTGGTTAACATCGGTCA
SEQ ID NO: 170: Fragment 12-R
   TAGGAAGACTCGGACGTAAAGGCTAAACCCTT
SEQ ID NO: 171: Fragment 13-R
   TAGCACCTGCGATCCGTAACCATGGATCATCATGCT
SEQ ID NO: 172: Fragment 14-R
   TAGGAAGACTCCTGCCACCTCCCTC
SEQ ID NO: 173: Fragment 15-R
   TAGGAAGACTCGTTCGAACCCCGGATC
SEQ ID NO: 174: Fragment 16-R
   TAGGAAGACTCCTCCGGCGCCATTTCT
SEQ ID NO: 175: Fragment 17-R
   TAGCACCTGCGATCGAGGACTTGCATTTCTGGTC
SEQ ID NO: 176: Fragment 18-R
   TAGGAAGACTCGCTGTCGTCCTTATGCC
SEQ ID NO: 177: Fragment 19-R
   TAGCACCTGCGATCGTCGGAATCCCCAGTTGTT
SEQ ID NO: 178: Fragment 20-R
   TAGCACCTGCGATCCCACGGGATTGGTTGTC
SEQ ID NO: 179: Fragment 21-R
   TAGGAAGACTCCCAGGCGGGCCATT
SEQ ID NO: 180: Fragment 24-R
   TAGGAAGACTCGAGCCATAATAAGTAAGGAAAAGAAAGAATG
SEQ ID NO: 181: Fragment 25-R 
TAGCACCTGCGATCAAGCTACTTGAAACAATTGTTGATATAACTGTTTT
G
SEQ ID NO: 182: Fragment 26-R
   TAGGAAGACTCTACCCAAGTATGGAACCTCAAC
SEQ ID NO: 183: Fragment 27-R
   TAGCACCTGCGATCGCTATTTTTTTTATCATTTTACTGATTTCGTGCGT
SEQ ID NO: 184: Fragment 29-R
   TAGCACCTGCGATCCTCGGCCCTTCCG
SEQ ID NO: 185: Primer 1 for CMV promoter
   CATCAATGGGCGTGGATAGC
SEQ ID NO: 186: Primer 2 for CMV promoter
   GGAGTTGTTACGACATTTTGGAAA
SEQ ID NO: 187: Primer 1 for EGFP-WPRE fragment
   GAGCTGGTTTAGTGGATATC
SEQ ID NO: 188: Primer 2 for EGFP-WPRE fragment
   CAGGGATGCCACCCGTTTAATTA

[Sequence Listing]

## Claims

1. A method for producing a virus-derived construct plasmid, the method comprising:
i) a step of obtaining a virus-derived construct plasmid containing at least one of nucleic acid sequences required to construct a virus-derived construct from a first microbial host cell; and
ii) a step of introducing the virus-derived construct plasmid into a second microbial host cell and placing the second microbial host cell under conditions in which the virus-derived construct plasmid is amplified.

2. A method for producing a virus-derived construct, the method comprising:
i) a step of obtaining a virus-derived construct plasmid containing at least one of nucleic acid sequences required to construct a virus-derived construct from a first microbial host cell;
ii) a step of introducing the virus-derived construct plasmid into a second microbial host cell and placing the second microbial host cell under conditions in which the virus-derived construct plasmid is amplified; and
iii) a step of introducing an introduced plasmid based on the virus-derived construct plasmid into a producer cell to form a virus-derived construct.

3. The method according to claim 2, further comprising, between the step ii) and the step iii), a step of cloning a nucleic acid containing at least one of the nucleic acid sequences required to construct a virus-derived construct in the virus-derived construct plasmid to produce the introduced plasmid.

4. The method according to claim 2, wherein the producer cell is an animal cell.

5. The method according to claim 2, wherein the producer cell is a human embryonic kidney cell and provides a viral vector genome copy number of 4 x 10³ or more per producer cell.

6. The method according to claim 1, wherein the step i) includes obtaining the virus-derived construct plasmid from a plurality of nucleic acid fragments including at least two nucleic acid fragments containing a vector partial nucleic acid sequence other than the nucleic acid sequence required to construct a virus-derived construct.

7. The method according to claim 6, wherein the vector partial nucleic acid sequence includes a nucleic acid sequence or a multiple cloning site having at least one function of nucleic acid replication, drug resistance, copy number adjustment, and stability improvement.

8. The method according to claim 6, further comprising, before the step i), a step of producing at least two of the plurality of nucleic acid fragments by chemical synthesis or an enzymatic reaction.

9. The method according to claim 6, further comprising, before the step i), a step of producing all of the plurality of nucleic acid fragments by chemical synthesis or an enzymatic reaction.

10. The method according to claim 1, wherein the nucleic acid sequence required to construct a virus-derived construct includes:
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

11. The method according to claim 10, wherein the introduced plasmid contains the nucleic acid sequences (A) to (D).

12. The method according to any one of claims 1 to 11, wherein the first microbial host cell is Bacillus subtilis.

13. The method according to any one of claims 1 to 11, wherein the second microbial host cell is Escherichia coli.

14. The method according to claim 1, wherein the step i) includes forming the virus-derived construct plasmid from a nucleic acid fragment.

15. The method according to claim 14, wherein the nucleic acid fragment is an acyclic nucleic acid fragment containing a tandem repeat nucleic acid sequence.

16. The method according to any one of claims 1 to 11, wherein the step i) includes bringing the first microbial host cell that is competent into contact with a nucleic acid containing at least one of the nucleic acid sequences required to construct a virus-derived construct.

17. A virus-derived construct plasmid prepared by the method according to claim 1.

18. A virus-derived construct prepared by the method according to claim 2.

19. A virus-derived construct-containing composition prepared by the method according to claim 2.

20. A plasmid comprising at least some of nucleic acid sequences required to construct a virus-derived construct in an operably linked form,
wherein the nucleic acid sequences required to construct a virus-derived construct include at least two of nucleic acid sequences (A) to (D):
(A) a nucleic acid sequence including a terminal repeat;
(B) a nucleic acid sequence encoding a packaging factor;
(C) a nucleic acid sequence encoding a structural protein; and
(D) a nucleic acid sequence encoding a functional cofactor.

21. The plasmid according to claim 20, wherein the plasmid contains the nucleic acid sequences (A) to (D) in an operably linked form.

22. The plasmid according to claim 20, wherein the nucleic acid sequence including a terminal repeat of (A) contains a desired gene.

23. The plasmid according to claim 20, wherein the virus-derived construct is a virus-derived construct originating from an adeno-associated virus or a retrovirus.

24. The plasmid according to claim 20, wherein the virus-derived construct is a viral vector originating from an adeno-associated virus.

25. The plasmid according to claim 24, wherein the nucleic acid sequence including a terminal repeat of (A) includes two inverted terminal repeats (ITRs),
the nucleic acid sequence encoding a packaging factor of (B) includes rep,
the nucleic acid sequence encoding a structural protein of (C) includes cap, and
the nucleic acid sequence encoding a functional cofactor of (D) includes at least one of E1A, E1B, E2A, E2B, E3, E4, and VA.

26. The plasmid according to claim 24, wherein at least one of the two ITRs is derived from any of adeno-associated virus serotypes 1 to 12 and variants thereof.

27. The plasmid according to claim 24, wherein the nucleic acid sequence (B) includes rep derived from any of adeno-associated virus serotypes 1 to 12 and variants thereof.

28. The plasmid according to claim 24, wherein the nucleic acid sequence (B) includes a promoter different from an adeno-associated virus promoter and at least one of rep78, rep76, rep52, and rep40 located downstream thereof.

29. The plasmid according to claim 28, wherein the promoter is a drug-responsive promoter.

30. The plasmid according to claim 24, wherein the terminal repeat of (A) and the nucleic acid sequence (B) include nucleic acid sequences derived from different adeno-associated virus serotypes.

31. The plasmid according to claim 24, wherein the nucleic acid sequence (C) includes cap derived from any of adeno-associated virus serotypes 1 to 12 and variants thereof.

32. The plasmid according to claim 24, wherein the nucleic acid sequence (C) includes a promoter different from an adeno-associated virus promoter and at least one of VP1, VP2, and VP3 located downstream thereof.

33. The plasmid according to claim 24, wherein the nucleic acid sequence (D) includes a nucleic acid sequence encoding a functional cofactor derived from any of an adenovirus, a herpes virus, a bocavirus, and variants thereof.

34. The plasmid according to claim 24, wherein the nucleic acid sequence (D) includes a nucleic acid sequence derived from any of adeno virus serotypes 1 to 52 and variants thereof.

35. The plasmid according to claim 24, wherein the nucleic acid sequence (D) includes a promoter different from an adeno-associated virus promoter and at least one of E1A, E1B, E2A, E2B, E3, and E4 located downstream thereof.

36. The plasmid according to claim 35, wherein the promoter is a drug-responsive promoter.

37. The plasmid according to claim 24, wherein the nucleic acid sequence including a terminal repeat of (A) includes a 5' ITR, a promoter, a desired gene, a terminator, and a 3' ITR from upstream.

38. The plasmid according to claim 20, wherein the virus-derived construct is a viral vector originating from a retrovirus.

39. The plasmid according to claim 38, wherein the nucleic acid sequence including a terminal repeat of (A) includes two long terminal repeats (LTRs),
the nucleic acid sequence encoding a packaging factor of (B) includes ψ,
the nucleic acid sequence encoding a structural protein of (C) includes at least one of gag, pol, VSV-G, and env, and
the nucleic acid sequence encoding a functional cofactor of (D) includes Rev.

40. The plasmid according to claim 38, wherein the plasmid contains a drug-responsive promoter upstream of at least one of the nucleic acid sequences (C) and (D).

41. The plasmid according to claim 38, wherein the nucleic acid sequence including a terminal repeat of (A) includes a 5' LTR, a promoter, a desired gene, a terminator, and a 3' LTR from upstream.

42. The plasmid according to claim 31, wherein the nucleic acid sequence (B) is present within the nucleic acid sequence (A), and the nucleic acid sequences (C) and (D) are present outside the nucleic acid sequence (A).

43. The plasmid according to claim 20, wherein the plasmid contains 8 or more genes encoded by the nucleic acid sequences (A) to (D) in an operably linked form.

44. The plasmid according to claim 20, wherein a drug-selectable marker nucleic acid sequence is contained between any two genes encoded by the nucleic acid sequences (A) to (D).

45. The plasmid according to any one of claims 20 to 44, wherein the nucleic acid sequence required to construct a virus-derived construct is about 10 kb or more.

46. The plasmid according to any one of claims 20 to 44, wherein the desired gene is 2 to 100 genes.

47. The plasmid according to any one of claims 20 to 44, wherein the plasmid contains, in an operably linked form:
a nucleic acid sequence that promotes plasmid replication in a first microbial host cell; and
a nucleic acid sequence that promotes plasmid replication in a second microbial host cell.

48. The plasmid according to claim 47, wherein the first microbial host cell is Bacillus subtilis.

49. The plasmid according to claim 47, wherein the second microbial host cell is Escherichia coli.

50. The plasmid according to any one of claims 20 to 44, wherein the plasmid provides a viral vector genome copy number of 4 x 10³ or more per producer cell when introduced into a human embryonic kidney cell that is a producer cell.

51. The plasmid according to any one of claims 20 to 44, wherein the plasmid provides a viral vector genome copy number of 1 x 10⁴ or more per producer cell when introduced into a human embryonic kidney cell that is a producer cell.

52. The plasmid according to any one of claims 20 to 44, wherein the plasmid provides a viral vector having a viral vector particle number with respect to a viral vector genome copy number (VP/VG) of 15 or less when introduced into a human embryonic kidney cell that is a producer cell.

53. The plasmid according to any one of claims 20 to 44, wherein the plasmid provides a viral vector having a viral vector particle number with respect to a viral vector genome copy number (VP/VG) of 10 or less when introduced into a human embryonic kidney cell that is a producer cell.

54. The plasmid according to any one of claims 20 to 44, wherein the plasmid and the producer cell together contain the nucleic acid sequence required to construct a virus-derived construct.

55. The plasmid according to any one of claims 20 to 44, wherein a covalently closed circular (CCC) purity is 80% or more.

56. The plasmid according to any one of claims 20 to 44, wherein nucleic acid fragments produced by chemical synthesis or an enzymatic reaction include at least two nucleic acid fragments containing a vector partial nucleic acid sequence.

57. The plasmid according to any one of claims 20 to 44, wherein only a plurality of nucleic acid fragments produced by chemical synthesis or an enzymatic reaction are circularized.

58. The plasmid according to claim 56 or 57, wherein the nucleic acid fragments include three or more nucleic acid fragments.

59. The plasmid according to any one of claims 56 to 58, wherein each of the nucleic acid fragments has a length of 2 kb or less.

60. The plasmid according to any one of claims 56 to 59, wherein the vector partial nucleic acid sequence includes 40% or less of a total sequence of the virus-derived construct plasmid.

61. The plasmid according to any one of claims 56 to 60, wherein the vector partial nucleic acid sequence includes a nucleic acid sequence having at least one function of nucleic acid replication, drug resistance, copy number adjustment, and stability improvement, the virus-derived construct plasmid contains a restriction enzyme recognition sequence upstream and downstream of the vector partial nucleic acid sequence, and the same sequence as the restriction enzyme recognition sequence is not included in the nucleic acid sequence required to construct a virus-derived construct.

62. A microbial host cell comprising the plasmid according to any one of claims 20 to 61.

63. A composition for preparing a virus-derived construct, the composition comprising the plasmid according to any one of claims 20 to 61.

64. A method for producing a viral vector, the method comprising a step of introducing the plasmid according to any one of claims 20 to 61 into a producer cell to form a virus-derived construct.

65. The method according to claim 64, wherein at least some of nucleic acids contained in the plasmid are incorporated into a chromosome of the producer cell.

66. A virus-derived construct produced by the method according to claim 64.

67. A virus-derived construct-containing composition produced by the method according to claim 64.

68. The virus-derived construct-containing composition according to claim 67, wherein a viral vector particle number with respect to a viral vector genome copy number (VP/VG) is 15 or less.

69. A producer cell into which the plasmid according to any one of claims 20 to 61 is introduced, wherein at least some of nucleic acids contained in the plasmid are incorporated into a chromosome of the producer cell.
